# EUROPEAN PATENT APPLICATION

(11) **EP 1 726 584 A1**
(43) Date of publication of application: **29.11.2006**
(21) Application number: 05720820.9
(22) Date of filing: 15.03.2005
(51) Int. Cl.: C07D 239/84, A61K 31/517, A61P 3/10, A61P 9/10, A61P 11/08, A61P 13/04, A61P 19/10, A61P 25/00, A61P 29/00, A61P 43/00, C07D 401/04, C07D 405/04, C07D 409/04

(54) **2-AMINOQUINAZOLINE DERIVATIVE**

(30) Priority: 15.03.2004 JP 2004073322
(71) Applicant: KYOWA HAKKO KOGYO CO., LTD., Chiyoda-ku, Tokyo 100-8185 (JP)
(72) Inventor: NAKASATO, Yoshisuke PHARMACEUTICAL RESEARCH CENTER, Nagaizumi-cho,Sunto-gun,Shizuoka 4118731 (JP); ATSUMI, Eri PHARMACEUTICAL RESEARCH CENTER, Nagaizumi-cho,Sunto-gun,Shizuoka 4118731 (JP); UOCHI, Yumiko BioFrontier Laboraties, Machida-shi, Tokyo 194-8533 (JP); SAKUMA, Takashi PHARMACEUTICAL RESEARCH CENTER, Nagaizumi-cho,Sunto-gun,Shizuoka 4118731 (JP); KURASAWA, Mariko HEAD OFFICE, Chiyoda-ku,Tokyo 100-8185 (JP); FUSE, Eiichi PHARMACEUTICAL RESEARCH CENTER, Nagaizumi-cho,Sunto-gun,Shizuoka 4118731 (JP); NOMOTO, Yuji PHARMACEUTICAL RESEARCH CENTER, Nagaizumi-cho,Sunto-gun,Shizuoka 4118731 (JP)
(74) Representative: Casalonga, Axel
(86) International application number: PCT/JP2005/004565
(87) International publication number: WO 2005/087749

(57) **Abstract**

The present invention provides a compound represented by Formula (I) 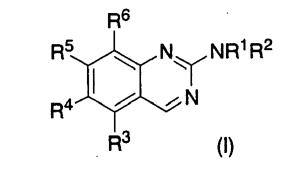 (wherein R¹ and R² are the same or different, and each represents a hydrogen atom, substituted or unsubstituted lower alkyl and the like,
R³ represents substituted or unsubstituted aryl, a substituted or unsubstituted aromatic heterocyclic group and the like,
R⁴ and R⁵ are the same or different, and each represents a hydrogen atom, halogen, substituted or unsubstituted lower alkyl, substituted or unsubstituted aryl and the like, proviso that they are not simultaneously hydrogen atoms, and
R⁶ represents hydroxy or substituted or unsubstituted lower alkoxy), or a pharmaceutically acceptable salt thereof and the like.

## Description

### Technical Field

The present invention relates to 2-amino quinazoline derivatives and the like having phosphodiesterase(PDE)-IV inhibitory activity.

### Background Art

Heretofore, it is known that many hormones and neurotransmitters exhibit their activities by increasing the concentration of an intracellular second messenger, adenosine 3',5'-cyclic monophosphate (cAMP) or guanosine 3',5'-cyclic monophosphate (cGMP). The intracellular concentration of cAMP and cGMP is controlled by their production and decomposition, and the decomposition is carried out by PDE. Accordingly, inhibiting PDE results in increasing the concentration of these intracellular second messengers. Up to the present, it has been made clear that 8 types of isozymes exist in PDE, and an isozyme-selective PDE inhibitor is expected to exhibit a pharmacological effect based on the physiological action and the biological distribution of the isozyme (Trends in Pharmacological Science, 1990, Vol. 11, p. 150; *ibid.,* 1991, Vol. 12, p. 19; Biochemical & Biophysical Research Communications, 1998, Vo. 250, p. 751).

It is known that increasing cAMP concentration in inflammatory leukocytes result in inhibiting the cellular activation. Activation of leukocytes leads to secretion of inflammatory cytokines such as tumor necrosis factor (TNFα), and expression of cell adhesion molecules such as intercellular adhesion molecules (ICAM), and the subsequent cellular infiltration (Journal of Molecular and Cellular Cardiology, 1989, Vol. 12 (Suppl. II), S61).

It is known that increasing cAMP concentration in tracheal smooth muscle cells results in inhibiting the muscle contraction (T. J. Torphy in Directions for New Anti-Asthma Drugs, by S. R. O'Donell, et al., 1988, p. 37, published by Birkhauser-Verlag). Tracheal smooth muscle contraction is an essential symptom of bronchial asthma. In ischemic reperfusion organ injury such as myocardial ischemia, infiltration of inflammatory leukocytes such as neutrophiles is observed in the lesion site. It has been made clear that, in these inflammatory cells and tracheal smooth muscle cells, mainly PDE-IV participates in the decomposition of cAMP. Accordingly, a PDE-IV selective inhibitor may be expected to be effective for prevention and/or treatment of inflammatory diseases as well as airway obstructive diseases, ischemic diseases and the like.

By accompanying cAMP increase, a PDE-IV inhibitor inhibits secretion of inflammatory cytokines such as TNFα, interleukin (IL)-8, and is therefore expected to prevent progress and prolong of inflammatory reaction and the like due to such inflammatory cytokines. For example, it is reported that TNFα downregulated the phosphorylation mechanism of the insulin receptor in muscles and fat cells, therefore being one of the cause of insulin-resistant diabetes (Journal of Clinical Investigation, 1994, Vol. 94, p. 1543). Similarly, it is suggested that since TNFα participates in the pathogenic progress of autoimmune diseases such as rheumatoid arthritis, multiple sclerosis, Crohn's disease and the like, a PDE-IV inhibitor may be effective for these diseases *(*Nature Medicine, 1995, Vol. 1, p. 211; ibid., 1995, Vol. 1, p. 244).

Participation of TFNα in the fatigue feeling after dialysis or in that of cancer patients is reported (International Journal of Artificial Organs, 1998, Vol. 21, p.83; Oncology Nursing Forum, 1992, Vol. 19, p. 419). Accordingly, a PDE-IV inhibitor may be expected to be effective for relieving fatigue and weariness.

A PDE-IV inhibitor is shown therapeutic effect in carcinomatous osteopenia models, sciatic nerve models and ovariectomy models which are animal models of osteoporosis, and therefore it is suggested that the PDE-IV inhibitor may be a therapeutic agent for osteoporosis (Japanese Journal of Pharmacology, 1999, Fol. 79, p.477).

It is known that ureter relaxation promotes excretion of calculi. Since a PDE-IV inhibitor inhibits the vermiculation of ureter, it is suggested that the PDE-IV inhibitor may be effective for prevention and/or treatment of urolithiasis (Journal of Urology, 1998, Vol. 160, p.920).

On the other hand, a antihypertensive agent (JPS39-25050); a PDE inhibitor (WO93/07124); a PDE-IV inhibitor (WO98/22460); a serine/threonine protein-kinase modulator (WO98/50370); an antimicrobial agent (US6156758); a neuropeptide ligand (WO03/26667); and a developer composition (JPH6-324437) which are comprising 2-amino quinazoline derivatives are known.

### Disclosure of the Invention

### Problems to be Solved by the Invention

An object of the present invention is to provide 2-amino quinazoline derivatives and the like having PDE-IV inhibitory activity.

### Means of Solving the Problems

The present invention relates to following (1) to (36).
(1) A 2-amino quinazoline derivative represented by 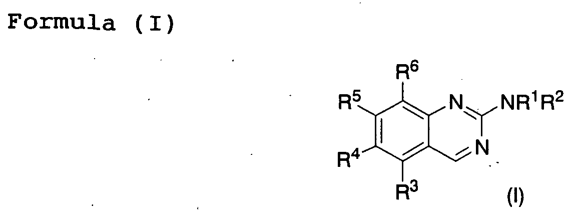 (wherein R¹ and R² are the same or different, and each represents a hydrogen atom, substituted or unsubstituted lower alkyl, substituted or unsubstituted cycloalkyl, substituted or unsubstituted aryl, a substituted or unsubstituted aromatic heterocyclic group, or a substituted or unsubstituted alicyclic heterocyclic group, or R¹ and R² are combined together with the adjacent nitrogen atom to form a substituted or unsubstituted aromatic heterocyclic group, or a substituted or unsubstituted alicyclic heterocyclic group,
   R³ represents substituted or unsubstituted aryl, a substituted or unsubstituted aromatic heterocyclic group, substituted or unsubstituted cycloalkyl, substituted or unsubstituted cycloalkenyl, or a substituted or unsubstituted alicyclic heterocyclic group,
   R⁴ and R⁵ are the same or different, and each represents a hydrogen atom, halogen, hydroxy, carboxy, substituted or unsubstituted lower alkyl, substituted or unsubstituted cycloalkyl, substituted or unsubstituted lower alkenyl, substituted or unsubstituted cycloalkenyl, substituted or unsubstituted lower alkoxy, substituted or unsubstituted cycloalkoxy, substituted or unsubstituted lower alkanoyl, substituted or unsubstituted aryl, a substituted or unsubstituted aromatic heterocyclic group, or a substituted or unsubstituted alicyclic heterocyclic group, proviso that they are not simultaneously hydrogen atoms, and
   R⁶ represents hydroxy or substituted or unsubstituted lower alkoxy), or a pharmaceutically acceptable salt thereof.
(2) The 2-amino quinazoline derivative or a pharmaceutically acceptable salt thereof according to (1), wherein R¹ and R² are the same or different, and each represents a hydrogen atom, substituted or unsubstituted lower alkyl, or substituted or unsubstituted cycloalkyl.
(3) The 2-amino quinazoline derivative or a pharmaceutically acceptable salt thereof according to (1), wherein R¹ and R² are the same or different, and each represents a hydrogen atom, methyl, ethyl or isopropyl.
(4) The 2-amino quinazoline derivative or a pharmaceutically acceptable salt thereof according to (1), wherein R¹ represents a hydrogen atom, and R² represents substituted or unsubstituted lower alkyl, or substituted or unsubstituted cycloalkyl.
(5) The 2-amino quinazoline derivative or a pharmaceutically acceptable salt thereof according to (1), wherein R¹ represents a hydrogen atom, and R² represents methyl, ethyl or isopropyl.
(6) The 2-amino quinazoline derivative or a pharmaceutically acceptable salt thereof according to (1), wherein R¹ represents a hydrogen atom, and R² represents cyclopropyl or cyclopropylmethyl.
(7) The 2-amino quinazoline derivative or a pharmaceutically acceptable salt thereof according to (1), wherein R¹ represents a hydrogen atom, and R² represents aralkyl.
(8) The 2-amino quinazoline derivative or a pharmaceutically acceptable salt thereof according to any one of (1) to (7), wherein R³ represents a substituted or unsubstituted aromatic heterocyclic group.
(9) The 2-amino quinazoline derivative or a pharmaceutically acceptable salt thereof according to any one of (1) to (7), wherein R³ represents substituted or unsubstituted pyridyl.
(10) The 2-amino quinazoline derivative or a pharmaceutically acceptable salt thereof according to any one of (1) to (7), wherein R³ represents substituted or unsubstituted aryl.
(11) The 2-amino quinazoline derivative or a pharmaceutically acceptable salt thereof according to any one of (1) to (7), wherein R³ represents substituted or unsubstituted phenyl.
(12) The 2-amino quinazoline derivative or a pharmaceutically acceptable salt thereof according to any one of (1) to (7), wherein R³ represents substituted or unsubstituted cycloalkyl, substituted or unsubstituted cycloalkenyl, or a substituted or unsubstituted alicyclic heterocyclic group.
(13) The 2-amino quinazoline derivative or a pharmaceutically acceptable salt thereof according to any one of (1) to (12), wherein R⁴ or R⁵ represents formyl or carboxy.
(14) The 2-amino quinazoline derivative or a pharmaceutically acceptable salt thereof according to any one of (1) to (12), wherein R⁴ represents substituted or unsubstituted lower alkyl, or substituted or unsubstituted lower alkenyl.
(15) The 2-amino quinazoline derivative or a pharmaceutically acceptable salt thereof according to any one of (1) to (12), wherein R⁴ represents lower alkyl which substituted with carboxy, or lower alkenyl which substituted with carboxy.
(16) The 2-amino quinazoline derivative or a pharmaceutically acceptable salt thereof according to any one of (1) to (12), wherein R⁴ represents substituted or unsubstituted aryl.
(17) The 2-amino quinazoline derivative or a pharmaceutically acceptable salt thereof according to any one of (1) to (12), wherein R⁴ represents aryl which substituted with carboxy.
(18) The 2-amino quinazoline derivative or a pharmaceutically acceptable salt thereof according to any one of (1) to (17), wherein R⁵ represents substituted or unsubstituted lower alkyl, substituted or unsubstituted cycloalkyl, substituted or unsubstituted lower alkenyl, or substituted or unsubstituted cycloalkenyl.
(19) The 2-amino quinazoline derivative or a pharmaceutically acceptable salt thereof according to any one of (1) to (17), wherein R⁵ represents substituted or unsubstituted aryl, or a substituted or unsubstituted aromatic heterocyclic group.
(20) The 2-amino quinazoline derivative or a pharmaceutically acceptable salt thereof according to any one of (1) to (17), wherein R⁵ represents lower alkyl which substituted with carboxy, lower alkenyl which substituted with carboxy, aryl which substituted with carboxy, an aromatic heterocyclic group which substituted with carboxy, or an alicyclic heterocyclic group which substituted with carboxy.
(21) The 2-amino quinazoline derivative or a pharmaceutically acceptable salt thereof according to any one of (1) to (17), wherein R⁵ represents lower alkyl which substituted with carboxy.
(22) The 2-amino quinazoline derivative or a pharmaceutically acceptable salt thereof according to any one of (1) to (17), wherein R⁵ represents aryl which substituted with carboxy.
(23) The 2-amino quinazoline derivative or a pharmaceutically acceptable salt thereof according to any one of (1) to (17), wherein R⁵ represents phenyl which substituted with carboxy.
(24) The 2-amino quinazoline derivative or a pharmaceutically acceptable salt thereof according to any one of (1) to (17), wherein R⁵ represents an aromatic heterocyclic group which substituted with carboxy.
(25) The 2-amino quinazoline derivative or a pharmaceutically acceptable salt thereof according to any one of (1) to (17); wherein R⁵ represents an alicyclic heterocyclic group which substituted with carboxy.
(26) The 2-amino quinazoline derivative or a pharmaceutically acceptable salt thereof according to any one of (1) to (17), wherein R⁵ represents an alicyclic heterocyclic group which substituted with carboxymethyl.
(27) The 2-amino quinazoline derivative or a pharmaceutically acceptable salt thereof according to any one of (1) to (17), wherein R⁵ represents halogen.
(28) The 2-amino quinazoline derivative or a pharmaceutically acceptable salt thereof according to any one of (1) to (27), wherein R⁶ represents substituted or unsubstituted lower alkoxy.
(29) The 2-amino quinazoline derivative or a pharmaceutically acceptable salt thereof according to any one of (1) to (27), wherein R⁶ represents methoxy.
(30) The 2-amino quinazoline derivative or a pharmaceutically acceptable salt thereof according to any one of (1) to (27), wherein R⁶ represents hydroxy.
(31) A pharmaceutical composition which comprises the 2-amino quinazoline derivative or a pharmaceutically acceptable salt thereof according to any one of (1) to (30) as an active ingredient.
(32) A phosphodiesterase (PDE)-IV inhibitor which comprises the 2-amino quinazoline derivative or a pharmaceutically acceptable salt thereof according to any one of (1) to (30) as an active ingredient.
(33) A preventive and/or therapeutic agent for diseases related to the function of phosphodiesterase (PDE)-IV which comprises the 2-amino quinazoline derivative or a pharmaceutically acceptable salt thereof according to any one of (1) to (30) as an active ingredient.
(34) A method for preventing and/or treating diseases related to the function of phosphodiesterase (PDE)-IV which comprises a step of administering an effective amount of the 2-amino quinazoline derivative or a pharmaceutically acceptable salt thereof according to any one of (1) to (30).
(35) Use of the 2-amino quinazoline derivative or a pharmaceutically acceptable salt thereof according to any one of (1) to (30) for the manufacture of phosphodiesterase (PDE)-IV inhibitor.
(36) Use of the 2-amino quinazoline derivative or a pharmaceutically acceptable salt thereof according to any one of (1) to (30) for the manufacture of a preventive and/or therapeutic agent for diseases related to the function of phosphodiesterase (PDE)-IV.

### Effect of the Invention

The present invention provides 2-amino quinazoline derivatives and the like having PDE-IV inhibitory activity.

### Best Mode for Carrying Out the Invention

Hereinafter, the compounds represented by the general formula (I) are referred to as "Compound (I)". The compounds having the other formula numbers are referred to in the same manner.

In the definitions for each groups in Formula (I):
(i) The halogen includes each atom of fluorine, chlorine, bromine and iodine.
(ii) Examples of the lower alkyl and the lower alkyl moieties of the lower alkoxy include, for example, linear or branched alkyl having 1 to 10 carbon atoms, such as methyl, ethyl, propyl, isopropyl, butyl, *sec*-butyl, tert-butyl, pentyl, neopentyl, hexyl, cyclohexyl, heptyl, octyl, nonyl, decyl, and the like.
(iii) Examples of the cycloalkyl and the cycloalkyl moieties of the cycloalkoxy include, for example, cycloalkyl having 3 to 10 carbon atoms, such as cyclopropyl, cyclobutyl, cyclopentyl, cyclohexyl, cycloheptyl, cyclooctyl, cyclononyl, cyclodecyl, and the like.
(iv) Examples of the lower alkenyl include, for example, linear or branched alkenyl having 2 to 10 carbon atoms, such as vinyl, allyl, 2-butenyl, 3-butenyl, 4-pentenyl, 6-octenyl, 2,6-octadienyl, 9-decenyl, and the like.
(v) Examples of the cycloalkenyl include, for example, cycloalkenyl having 3 to 10 carbon atoms, such as cyclopropenyl, cyclobutenyl, cyclopentenyl, cyclohexenyl, cycloheptenyl, cyclooctenyl, cyclononenyl, cyclodecenyl, and the like.
(vi) Examples of the aryl and the aryl moieties of the aralkyl include, for example, aryl having 6 to 14 carbon atoms, such as phenyl, naphthyl, indenyl, anthryl, and the like.
(vii) The alkylene moieties of the aralkyl have the same meanings as the group formed by removing one hydrogen atom from the lower alkyl (ii) defined above.
(viii) Examples of the aromatic heterocyclic group include, for example, a 5- or 6-membered monocyclic aromatic heterocyclic group containing at least one atom selected from a nitrogen atom, an oxygen atom, and a sulfur atom; and a bicyclic or tricyclic condensed aromatic heterocyclic group in which 3- to 8-membered rings are condensed and containing at least one atom selected from a nitrogen atom, an oxygen atom, and a sulfur atom. More specific examples include pyridyl, pyridonyl, pyrazinyl, pyrimidinyl, pyridazinyl, quinolyl, isoquinolyl, phthalazinyl, quinazolinyl, quinoxalinyl, naphthyridinyl, cinnolinyl, pyrrolyl, pyrazolyl', imidazolyl, triazolyl, tetrazolyl, thienyl, furyl, furazanyl, thiazolyl, oxazolyl, indolyl, indazolyl, benzimidazolyl, isooxazolyl, benzotriazolyl, benzothiazolyl, benzoxazolyl, benzofrazanyl, purinyl, acridinyl, carbazolyl, and the like.
(ix) Examples of the alicyclic heterocyclic group include, for example, a 5- or 6-membered monocyclic alicyclic heterocyclic group containing at least one atom selected from a nitrogen atom, an oxygen atom, and a sulfur atom; and a bicyclic or tricyclic condensed alicyclic heterocyclic group in which 3- to 8-membered rings are condensed and containing at least one atom selected from a nitrogen atom, an oxygen atom, and a sulfur atom. More specific examples include pyrrolidinyl, pyrrolidonyl, piperidino, piperidyl, piperadinyl, morpholino, morpholinyl, thiomorpholino, thiomorpholinyl, homopiperidino, homopiperidyl, homopiperadinyl, tetrahydropyridyl, tetrahydroquinolyl, tetrahydroisoquinolyl, tetrahydrofuranyl, tetrahydropyranyl, dihydrobenzofuranyl, and the like.
(x) Examples of the lower alkanoyl include, for example, linear or branched alkanoyl having 1 to 8 carbon atoms, such as formyl, acetyl, propionyl, butyryl, isobutyryl, valeryl, isovaleryl, pivaloyl, hexanoyl, heptanoyl, octanoyl, and the like.
(xi) Examples of the aromatic heterocyclic group formed together with the adjacent nitrogen atom include 5-or 6-membered monocyclic aromatic heterocyclic group containing at least one nitrogen atom (the monocyclic aromatic heterocyclic group may further contain any other of nitrogen atom(s), oxygen atom(s) or sulfur atom(s)) and bicyclic or tricyclic condensed aromatic heterocyclic group containing at least one nitrogen atom in which 3- to 8-membered rings are condensed (the condensed aromatic heterocyclic group may further contain any other of nitrogen atom(s), oxygen atom(s) or sulfur atom(s)). More specific examples include, for example, pyrrolyl, imidazolyl, indolyl, indazolyl, and the like.
(xii) Examples of the alicyclic heterocyclic group formed together with the adjacent nitrogen atom include 5-or 6-membered monocyclic alicyclic heterocyclic group containing at least one nitrogen atom (the monocyclic alicyclic heterocyclic group may further contain any other of nitrogen atom(s), oxygen atom(s) or sulfur atom(s)) and bicyclic or tricyclic condensed alicyclic heterocyclic group containing at least one nitrogen atom in which 3- to 8-membered rings are condensed (the condensed alicyclic heterocyclic group may further contain any other of nitrogen atom(s), oxygen atom(s) or sulfur atom(s)). More specific examples include, for example, pyrrolidinyl, pyrrolidonyl, piperidino, piperazinyl, morpholino, thiomorpholino, homopiperidino, homopiperazinyl, tetrahydropyridyl, tetrahydroquinolyl, tetrahydroisoquinolyl, and the like.
(xiii) Examples of the substituents in the substituted lower alkyl, the substituted cycloalkyl, the substituted lower alkenyl, the substituted cycloalkenyl, the substituted lower alkoxy, the subsubstituted cycloalkoxy, the substituted lower alkanoyl, the substituted aryl, the substituted aromatic heterocyclic group, the substituted alicyclic heterocyclic group, the substituted aromatic heterocyclic group formed together with the adjacent nitrogen atom, and the substituted aromatic heterocyclic group formed together with the adjacent nitrogen atom may be the same or different, and include, for example, in number of 1 to 3 substituent(s), such as halogen, hydroxy, amino, nitro, mercapto, sulfo, cyano, carboxy, methylenedioxy, carbamoyl, sulfamoyl, lower alkenyl, cycloalkenyl, lower alkynyl, lower alkoxycarbonyl, mono- or di-(lower alkyl)carbamoyl, lower alkylsulfinyl, lower alkylsulfonyl, lower alkylthio, aryloxy, aralkyloxy, aroyl, an aromatic heterocyclic group, an alicyclic heterocyclic group, substituted or unsubstituted cycloalkyl (examples of the substituents in said substituted cycloalkyl may be the same or different, and include, for example, in number of 1 to 3 substituent(s), such as halogen, hydroxy, carboxy, lower alkyl, lower alkoxy, and the like), substituted or unsubstituted lower alkoxy (examples of the substituents in said substituted lower alkoxy may be the same or different, and include, for example, in number of 1 to 3 substituent(s), such as halogen, hydroxy, carboxy, lower alkoxycarbonyl, and the like), substituted or unsubstituted lower alkanoyl (examples of the substituents in said substituted lower alkanoyl may be the same or different, and include, for example, in number of 1 to 3 substituent(s), such as halogen, hydroxy, carboxy, and the like), substituted or unsubstituted aryl (examples of the substituents in said substituted aryl may be the same or different and include, for example, in number of 1 to 3 substituent(s), such as halogen, hydroxy, carboxy, lower alkyl, lower alkoxy, and the like), substituted or unsubstituted aralkyl (examples of the substituents in said substituted aralkyl may be the same or different, and include, for example, in number of 1 to 3 substituent(s), such as halogen, hydroxy, carboxy, lower alkoxy, and the like), substituted or unsubstituted mono- or di-(lower alkyl)amino (examples of the substituents in said mono- or di-(lower alkyl)amino may be the same or different, and include, for example, in number of 1 to 3 substituent(s), such as halogen, hydroxy, carboxy, and the like). The substituent(s) in the substituted cycloalkyl, the substituted cycloalkenyl, the substituted aryl, the substituted aromatic heterocyclic group, the substituted alicyclic heterocyclic group, the substituted aromatic heterocyclic group formed together with the adjacent nitrogen atom, and the substituted aromatic heterocyclic group formed together with the adjacent nitrogen atom may be, in addition to the above substituents, the substituted or unsubstituted lower alkyl (examples of the substituents in said substituted lower alkyl may be the same or different and include, for example, in number of 1 to 3 substituent(s), such as halogen, hydroxy, carboxy, lower alkoxycarbonyl, aromatic heterocyclic group, alicyclic heterocyclic group, and the like). The substituted aryl may also be, dihydrobenzoxazolyl, dihydrobenzothiazolyl, 2-oxodihydrobenzoxazolyl or 2-oxodihydrobenzothiazolyl. Further, the substituents in the substituted cycloalkyl, the substituted cycloalkenyl, the substituted alicyclic heterocyclic group and the substituted alicyclic heterocyclic group formed together with the adjacent nitrogen atom may be oxo.

Herein, the halogen, the lower alkyl and the lower alkyl moieties of the lower alkoxy,' the cycloalkyl, the lower alkenyl, the cycloalkenyl, the aryl and the aryl moieties of the aralkyl, the alkylene moieties of the aralkyl, the aromatic heterocyclic group, the alicyclic heterocyclic group, and the lower alkanoyl have the same meanings as the halogen(i), the lower alkyl(ii), the cycloalkyl(iii), the loweralkenyl(iv), the cycloalkenyl(v), the aryl(vi), the alkylene moieties of the aralkyl(vii), the aromatic heterocyclic group(viii), the alicyclic heterocyclic group(ix), and the lower alkanoyl(x) defined above, respectively.

The lower alkyl moieties of the lower alkoxycarbonyl, the mono- or di-(lower alkyl)amino, the mono- or di-(lower alkyl)carbamoyl, the lower alkylsulfinyl, the lower alkylsulfonyl and the lower alkylthio have the same meanings as the lower alkyl (ii) defined above. The two lower alkyl moieties in the di-(lower alkyl)amino and the di-(lower alkyl)carbamoyl may be the same or different.

Examples of the lower alkynyl include, for example, linear or branched alkynyl having 2 to 6 carbon atoms such as ethynyl, propargyl, 3-butynyl, 3-pentynyl, 3-hexynyl, 4-methyl-2-pentynyl, and the like.

The aryl moieties in the aryloxy, the aralkyloxy, and the aroyl have the same meaning as the aryl (vi) defined above.

The alkylene moieties of the aralkyloxy have the same meaning as the group formed by removing one hydrogen atom from the lower alkyl (ii) defined above.

Examples of the pharmaceutically acceptable salts of Compound (I) include, for example, pharmaceutically acceptable metal salts, ammonium salts, organic amine addition salts, amino acid addition salts, acid addition salts, and the like. The pharmaceutically acceptable metal salts include, for example, alkali metal salts such as sodium salts, potassium salts, and the like; alkaline earth metal salts such as magnesium salts, calcium salts, and the like; aluminum salts; zinc salts, and the like. The pharmaceutically acceptable ammonium salts include, for example, salts of ammonium, tetramethylammonium, or the like. The pharmaceutically acceptable organic amine addition salts include, for example, addition salts of morpholine, piperidine, or the like. The pharmaceutically acceptable amino acid addition salts include, for example, addition salts of lysine, glycine, phenylalanine, or the like. The pharmaceutically acceptable acid addition salts include, for example, inorganic acid salts such as hydrochlorides, sulfates, phosphates, and the like; and organic acid salts such as acetate, maleate, fumarate, tartrates, citrates, and the like.

Among Compounds (I), some stereoisomers, geometrical isomers, tautomers and the like may be existed. All possible isomers and mixtures thereof in any ratio are included in the present invention.

Production Methods of Compound (I) will be described below.

In the Production Methods described below, when a defined group changes under the conditions or is not suitable for carrying out the method, it is possible to obtain the desired compound using a method commonly used in synthetic organic chemistry such as protection/deprotection of functional group [for example, Protective Groups in Organic Synthesis, third edition, T. W. Greene, John Wiley & Sons Inc. (1999)]. If necessary, the order of reaction processes such as introduction of substituents can be changed.

### Production Methods

Compound (I) can be prepared according to the reaction processes in the following Production Methods 1 to 7. The symbols Me, Et and Bu mentioned typically in the following Production Methods and tables represent methyl, ethyl and butyl, respectively.

### Production Method 1

Among Compound (I), Compound (Ib) in which R⁴ represents a hydrogen atom and R⁵ represents R^{5a} (wherein R^{5a} represents substituted or unsubstituted lower alkenyl, substituted or unsubstituted aryl, substituted or unsubstituted aromatic heterocyclic group, or substituted or unsubstituted alicylic heterocyclic group) may be prepared according to the following Production Method 1. 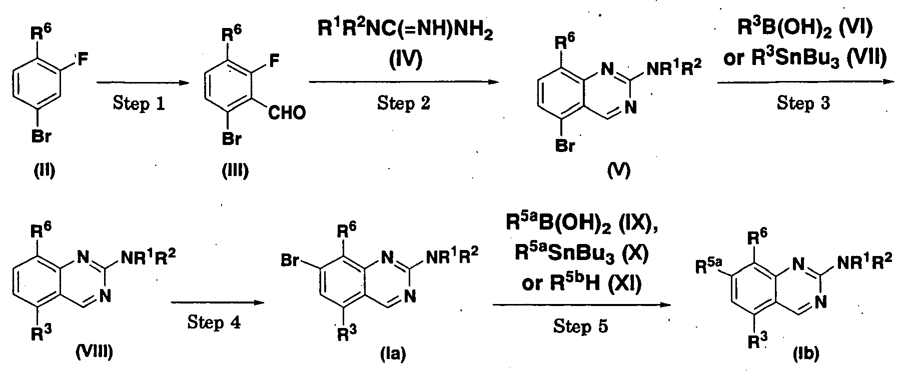 (wherein R¹, R², R³, R^{5a} and R⁶ have the same meanings as defined above, respectively, and R^{5b} represents substituted or unsubstituted alicyclic heterocyclic group)

### Step 1

Compound (III) can be prepared by reacting Compound (II) with 1 to 20 equivalents of N,N-dimethylformamide (DMF) in the presence of 1 to 5 equivalents of a base in a solvent.

The solvent may be any inert solvent to the reaction, and include, but not limit to, for example, tetrahydrofuran (THF), diethyl ether, 1,4-dioxane, dimethoxyethane,' and the like, preferably THF can be used.

As the base, lithium diisopropylamide (LDA), lithium(bistrimethyl silyl)amide and the like, preferably LDA, can be used.

The reaction is completed for about 5 minutes to 48 hours at a temperature between -78 and 0°C, preferably at -78 °C.

Compound (II) is commercially available or can be prepared by the known method of alkylation of hydroxyl group using 4-bromo-2-fluorophenol (commercially available) [for example, Protective Groups in Organic Synthesis, third edition, T. W. Greene, John Wiley & Sons Inc., p.145 (1999), and the like], or the analogous methods thereto.

### Step 2

The present step can be carried out by the known method [for example, Journal of Heterocyclic Chemistry, vol.34, p.385 (1997)] or the analogous methods thereto.

Compound (V) can be prepared by reacting Compound (III) with 1 to 20 equivalents of Compound (IV) in the presence of 1 to 20 equivalents of a base in a solvent.

The solvent may be any inert solvent to the reaction, and include, but not limit to, for example, N,N-dimethylacetamide (DMA), DMF, N-methylpyrrolidone, dimethyl sulfoxide (DMSO), and the like, preferably DMA can be used.

As the base, potassium carbonate, cesium carbonate, sodium methoxide, potassium *tert*-butoxide, and the like, preferably potassium carbonate or cesium carbonate, can be used.

The reaction is completed for about 5 minutes to 48 hours at a temperature between room temperature and 180°C, preferably at 160°C.

Compound (IV) is commercially available or can be prepared by the known method [for example, Journal of Organic Chemistry, vol.57, p.2497 (1992)] or the analogous methods thereto.

### Step 3

Compound (VIII) can be prepared by reacting Compound (V) with 1 to 20 equivalents of Compound (VI) or (VII) in the presence of 0.1 to 10 equivalents of a base and 0.001 to 1 equivalent of a palladium catalyst in a solvent.

The solvent may be any inert solvent to the reaction, and include, but not limit to, for example, acetonitrile, methanol, ethanol, dichloromethane, 1,2-dichloroethane, chloroform, DMA, DMF, dioxane, THF, diethyl ether, diisopropyl ether, benzene, toluene, xylene, N,N-dimethylimidazolidinone, N-methylpyrrolidone, sulfolane, and the mixture of at least one solvent selected from the above with water in an appropriate ratio between 100:1 to 1:100, and the like, preferably a mixture of water and dioxane in a ratio of 1:2 can be used.

As the base, pyridine, triethylamine, N-methylmorpholine, N-methylpiperidine, piperidine, piperazine, potassium acetate, potassium carbonate, cesium carbonate, sodium carbonate, sodium hydrogencarbonate, sodium hydroxide, lithium hydroxide, potassium hydroxide, potassium phosphate, sodium *tert*-butoxide, 1,8-diazabicyclo[5.4.0]-7-undecene (DBU), diisopropyl ethylamine or the like, preferably sodium carbonate can be used. However, when Compound (VII) may be used, the base is not necessary.

As the palladium source of the palladium catalyst, for example, palladium acetate, palladium trifluoroacetate, tris(dibenzylideneacetone)dipalladium, chloroform adducts thereof, or the like can be used; and as the ligand thereof, for example, triphenyl phosphine, 1,1'-bis(diphenylphosphino)ferrocene, o-tolyl phosphine, 1,2-bis(diphenylphosphino)ethane, 1,3-(bisdiphenylphosphino)propane, 1,4-bis(bisdiphenylphosphino)butane, di-tert-butyldiphenyl phosphine, 2-(di-tert-butylphosphino)biphenyl, 2-(dicyclohexylphosphino)biphenyl, or the like, can be used. Preferably 1 to 10 equivalents of the ligand to palladium can be used. Also, for example, commercially-available reagents where a suitable ligand for reaction is previously coordinated with palladium, such as tetrakis(triphenylphosphine)palladium, 1,1'-bis(diphenylphosphino)ferrocene-dichloropalladium/dichloromethane 1/1 adduct, or the like can be used.

The reaction is completed for about 5 minutes to 48 hours at a temperature between room temperature and the boiling point of the solvent used.

Compound (VI) and Compound (VII) are commercially available or can be prepared by the known method [for example, Shin-Jikken-Kagaku-Koza, Vol. 12, the Chemical Society of Japan (1978)] or the analogous methods thereto.

### Step 4

Compound (Ia) can be prepared by reacting Compound (VIII) with 1 to 20 equivalents of bromine in a solvent.

The solvent may be any inert solvent to the reaction, and include, but not limit to, for example, acetic acid, carbon tetrachloride, chloroform, dichloromethane, 1,2-dichloroethane, dioxane, THF, ethyl acetate, and the like, and preferably acetic acid can be used.

The reaction is completed for about 5 minutes to 48 hours at a temperature between 0°C and the boiling point of the solvent used, preferably at 60°C.

Also, this step may be carried out using, for example, N-bromosuccinimide, pyrrolidone tribromide, cuprous bromide, pyridinium tribromide, or the like, in place of bromine, and a solvent suitable to the reagent used. The solvent may be any inert solvent to the reaction, and include, but not limit to, for example, acetonitrile, methanol, ethanol, dichloromethane, 1,2-dichloroethane, chloroform, dimethoxyethane, DMF, dioxane, THF, diethyl ether, diisopropyl ether, N,N-dimethylimidazolidinone, N-methylpyrrolidone, sulforane, and the like, and preferably DMF can be used.

### Step 5

Compound (Ib) can be prepared by reacting Compound (Ia) with Compound (IX) or Compound (X) in a similar manner to the step 3, or by reacting Compound (Ia) with Compound (XI) under the condition similar thereto.

Compound (IX) and Compound (X) are commercially available or can be prepared by the known method [for example, Shin-Jikken-Kagaku-Koza, Vol. 12, the Chemical Society of Japan (1978), and the like] or the analogous methods thereto.

### Production Method 2

Compound (Ib) may also be prepared according to the following Production Method 2. 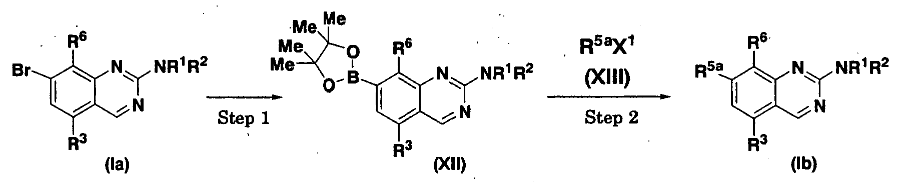 (wherein X¹ represents a leaving group generally used in organic synthetic chemistry, such as chloro, bromo, iodio, methanesulfonyloxy, trifluoromethanesulfonyloxy, or the like, and R¹, R², R³, R^{5a} and R⁶ have the same meanings as defined above, respectively.)

### Step 1

Compound (XII) can be prepared in the same manner as in the step 3 in Production Method 1 using Compound (Ia) and bis(pinacolate)diboron (commercial product).

### Step 2

Compound (Ib) can be prepared in the same manner as in the step 3 in Production Method 1 using Compound (XII) and Compound (XIII).

Compound (XIII) is commercially available or can be prepared by the known method [for example, Shin-Jikken-Kagaku-Koza, Vol. 14, the Chemical Society of Japan (1978), and the like] or the method similar theteto.

### Production Method 3

Among Compound (I); Compound (Id) in which R⁴ is a hydrogen atom and R⁵ is 2-carboxyethenyl, and Compound (If) in which R⁴ is a hydrogen atom and R⁵ is 2-carboxyethyl can be prepared, for example, according to the following Production Method 3. 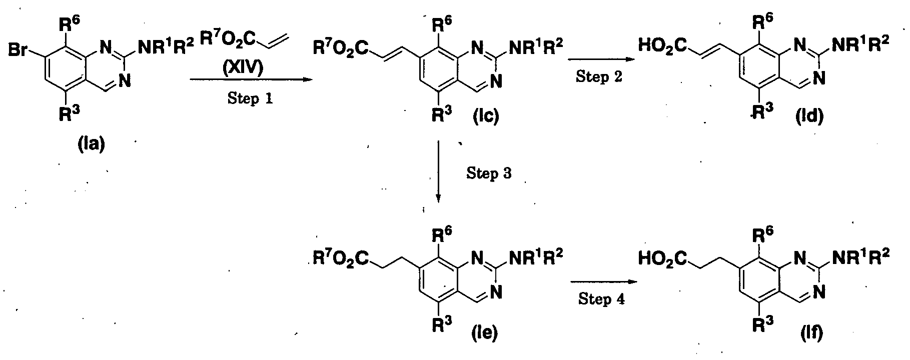 (wherein R¹, R², R³ and R⁶ have the same meanings as defined above, respectively, and R⁷ represents substituted or unsubstituted lower alkyl.)

### Step 1

Compound (Ic) can be prepared by reacting Compound (Ia) with 1 to 20 equivalents of Compound (XIV) in the presence of 0.1 to 10 equivalents of a base and 0.001 to 1 equivalent of a palladium catalyst in a solvent.

The solvent may be any inert solvent to the reaction, and include, but not limit to, for example, acetonitrile, 1,2-dichloroethane, DMA, DMF, DMSO, dioxane, THF, benzene, toluene, xylene, N,N-dimethylimidazolidinone, N-methylpyrrolidone, sulforane, and the like, and preferably DMF can be used.

As the base, triethylamine, pyridine, N-methylmorpholine, N-methylpiperidine, piperidine, piperazine, potassium acetate, potassium carbonate, cesium carbonate, sodium carbonate, sodium hydrogencarbonate, sodium hydroxide, lithium hydroxide, potassium hydroxide, potassium phosphate, sodium tert-butoxide, DBU, diisopropylethylamine, or the like, preferably triethylamine, can be used.

As the palladium source of the palladium catalyst, for example, palladium acetate, palladium trifluoroacetate, tris(dibenzylideneacetone)dipalladium, the chloroform adducts theteof, or the like can be used; and as the ligand thereof, for example, triphenyl phosphine, 1,1'-bis(diphenylphosphino)ferrocene, o-tolyl phosphine, 1,2-bis(diphenylphosphino)ethane, 1,3-(bisdiphenylphosphino)propane, 1,4-bis(bisdiphenylphosphino)butane, di-tert-butyldiphenyl phosphine, 2-(di-tert-butylphosphino)biphenyl, 2-(dicyclohexylphosphino)biphenyl, or the like can be used. Preferably 1 to 10 equivalents of the ligand to palladium can be used. Also, for example, commercially-available reagents in which a suitable ligand for reaction is previously coordinated with palladium, such as tetrakis(triphenylphosphine)palladium. 1,1'-bis(diphenylphosphino)ferrocene-dichloropalladium/dichloromethane 1/1 adduct, can be used.

The reaction is completed for about 5 minutes to 48 hours at a temperature between room temperature and the boiling point of the solvent used, preferably at 100°C.

Compound (XIV) is commercially available or can be prepared by the known method [for example, Shin-Jikken-Kagaku-Koza, Vol. 12, the Chemical Society of Japan (1978), and the like] or the analogous methods thereto.

### Step 2

Compound (Id) can be prepared by reacting Compound (Ic) with 1 to 200 equivalents of a base or with a catalytic amount to 200 equivalents of an acid in the presence or absence of a solvent.

The solvent may be any inert solvent to the reaction, and include, but not limit to, for example, methanol, ethanol, 1-propanol, acetone, methyl ethyl ketone, diethyl ether, THF, dioxane, DMF, dichloromethane, 1,2-dichloroethane, mixed solvent prepared by mixing at least any one solvent selected from the above with water in a suitable ratio between 100:1 to, 1:100, and the like, and preferably 1:4 mixed solvent of water and methanol can be used.

As the base, for example, sodium hydroxide, potassium hydroxide, lithium hydroxide, or the like, preferably lithium hydroxide, can be used.

As the acid, for example, hydrochloric acid, sulfuric acid, acetic acid, formic acid, trifluoroacetic acid, p-toluenesulfonic acid, methanesulfonic acid, titanium tetrachloride, boron trifluoride, or the like, preferably trifluoroacetic acid, can be used.

The reaction is completed for about 5 minutes to 48 hours at a temperature between 0°C and the boiling point of the solvent used, preferably at room temperature.

### Step 3

Compound (Ie) can be prepared by catalytic reduction of Compound (Ic).

The solvent may be any inert solvent to the reaction, and include, but not limit to, for example, methanol, ethanol, acetic acid, ethyl acetate, THF, dioxane, mixed solvent prepared by mixing at least any one solvent selected from the above with water in a suitable ratio between 100:1 to 1:100, and the like, and preferably ethanol can be used.

Example of the condition of catalytic reduction include a condition in which the reaction is carried out in the presence of a catalytic amount to an excessive amount of a catalyst generally used in catalytic reduction, such as palladium-carbon, platinum dioxide, Raney nickel, and the like, under a hydrogen pressure of normal to 50000 kPa or in the presence of a hydrogen donor such as formic acid, ammonium formate, hydrazine, cyclohexene, cyclohexadiene, triethylsilane, and the like, optionally adding 1 to 200 equivalents of ammonia, acetic acid, hydrochloric acid, sulfuric acid, or the like. Preferably, a condition in which the reaction carried out in the presence of 10 wt.% palladium-carbon and under normal hydrogen pressure may be employed.

The reaction is completed for about 5 minutes to 48 hours at a temperature between 0°C and the boiling point of the solvent used, preferably at a room temperature.

Also, Compound (Ie) can be prepared by reacting Compound (Ic) with 1 to 200 equivalents of a reducing agent in a solvent.

The solvent may be any inert solvent to the reaction, and include, but not limit to, for example, methanol, ethanol, acetic acid, ethyl acetate, THF, dioxane, and the like, and preferably methanol can be used.

The examples of the reducing agent include sodium borohydride, sodium hydride/nickel chloride, sodium hydride/cobalt chloride, sodium cyanoborohydride, and the like, and preferably sodium borohydride can be used.

The reaction is completed for about 5 minutes to 48 hours at a temperature between 0°C and the boiling point of the solvent used, preferably at room temperature.

### Step 4

Compound (If) can be prepared in the same manner as in the step 2 in Production Method 3 using Compound (Ie).

### Production Method 4

Compound (VIII) can also be prepared, for example, according to the following Production Method 4. 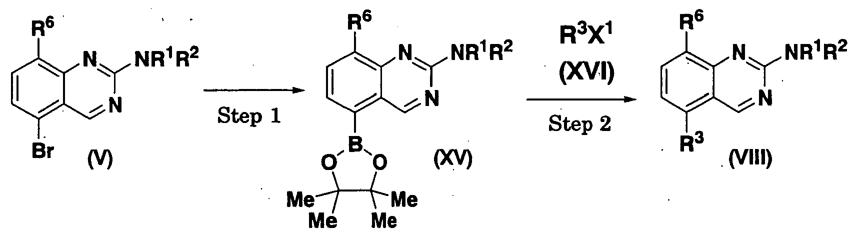 (wherein R¹, R², R³, R⁶ and X¹ have the same meanings as defined above, respectively.)

### Step 1

Compound (XV) can be prepared in the same manner as in the step 3 in Production Method 1 using Compound (V) and bis(pinacolate)diboron (commercial product).

### Step 2

Compound (VIII) can be prepared in the same manner as in the step 3 in Production Method 1 using Compound (XV) and Compound (XVI).

Compound (XVI) is commercially available or can be prepared by the method [for example, Shin-Jikken-Kagaku-Koza, Vol. 14, the Chemical Society of Japan (1978), and the like] or the analogous methods thereto.

### Production Method 5

Among Compound (I), Compound (Ii) in which R⁶ is OR⁸ (where R⁸ represents substituted or unsubstituted lower alkyl) can be prepared, for example, according to the following Production Method 5. 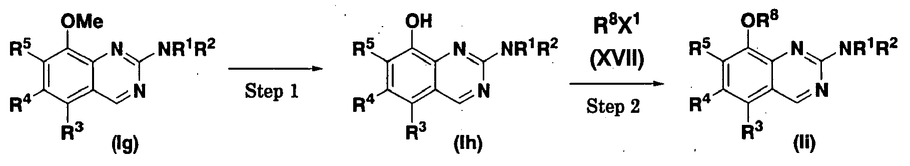 (wherein R¹, R², R³, R⁴, R⁵, R⁸ and X¹ have the same meanings as defined above, respectively.)

### Step 1

Compound (Ih) can be prepared by subjecting Compound (Ig) under the condition of demethylation of aryl methyl ether generally employed in organic synthetic chemistry [by or in a similar manner to a known method, for example, described in Protective Groups in Organic Synthesis 3rd Edition, by T. W. Greene, John Wiley & Sons, Inc., p. 249 (1999)].

Compound (Ig) can be prepared by the method described in Production Methods 1 to 3 or the analogous methods thereto.

### Step 2

Compound (Ii) can be prepared by reacting Compound (Ih) with 1 to 20 equivalents of Compound (XVII) in the presence 1 to 20 equivalents of a base in a solvent.

The solvent may be any inert solvent to the reaction, and include, but not limit to, for example, dimethoxyethane, DMF, dioxane, THF, diethyl ether, diisopropyl ether, benzene, toluene, xylene, pyridine, N,N-dimethylimidazolidinone, N-methylpyrrolidone, sulforane, and the like, and preferably THF or DMF can be used.

As the base, sodium hydride, potassium tert-butoxide, potassium acetate, potassium carbonate, cesium carbonate, sodium carbonate, sodium hydrogencarbonate, sodium hydroxide, lithium hydroxide, potassium hydroxide, potassium phosphate, or the like, preferably sodium hydride can be used.

Compound (XVII) is commercially available or can be prepared by the method [for example, Shin-Jikken-Kagaku-Koza, Vol. 14, the Chemical Society of Japan (1978)] or the analogous methods thereto.

### Production Method 6

Compound (I) can be prepared, for example, according to the following Production Method 6. 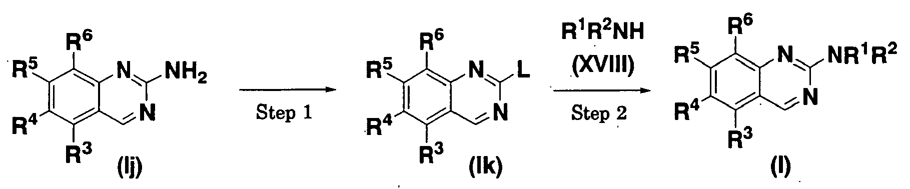 (wherein L represents a leaving group such as chloro, bromo , iodo, and the like, and R¹, R², R³, R⁴, R⁵ and R⁸ have the same meanings as defined above, respectively.)

### Step 1

Compound (Ik) can be prepared by reacting Compound (Ij) with 1 to 100 equivalents of nitrite compound in the presence or absence of a solvent and optionally in the presence of 1 to 1000 equivalents of an acid and 1 to 1000 equivalents of a halogen source.

The solvent may be any inert solvent to the reaction, and include, but not limit to, for example, THF, dioxane, acetone, DMSO, DMF, water, a mixed solvent thereof, and the like, and preferably THF or water can be used.

As the acid, hydroiodic acid, hydrobromic acid, hydrochloric acid, or the like can be used.

As the halogen source, copper(I) chloride, copper(I) bromide, copper(I) iodide, copper(II) chloride, copper(II) bromide, copper(II) iodide, potassium iodide, diiodomethane, or the like, preferably copper(I) iodide or diiodomethane can be used.

As the nitrite compound, nitrous acid, nitrites salt such as sodium nitrite, and the like, nitrosyl halide such as nitrosyl chloride, and the like, alkyl nitrites such as tert-butyl nitrite, isoamyl nitrite, and the like, preferably sodium nitrite or isoamyl nitrite can be used.

The reaction is completed for about 5 minutes to 48 hours at a temperature between -30°C and the boiling point of the solvent used, preferably at 60°C.

Also, Compound (Ij) can be prepared by the method described in Production Methods 1 to 3 or 5, or the analogous methods thereto.

### Step 2

Compound (I) can be prepared by reacting Compound (Ik) with 1 to 1000 equivalents of the amine (XVIII) in the presence or absence of a solvent, optionally in the presence of 1 to 100 equivalents of a base.

The solvent may be any inert solvent to the reaction, and include, but not limit to, for example, acetonitrile, methanol, ethanol, dichloromethane, chloroform, THF, dioxane, acetone, DMSO, DMF, and the like, and preferably DMF or THF can be used.

As the base, pyridine, triethylamine, diisopropylethylamine, N-methylmorpholine, DBU, or the like, preferably triethylamine can be used.

The reaction is completed for about 5 minutes to 48 hours at a temperature between room temperature and the boiling point of the solvent used, preferably at room temperature.

Compound (XVIII) is commercially available.

### Production Method 7

Compound (I) can be prepared, for example, according to the following Production Method 7. 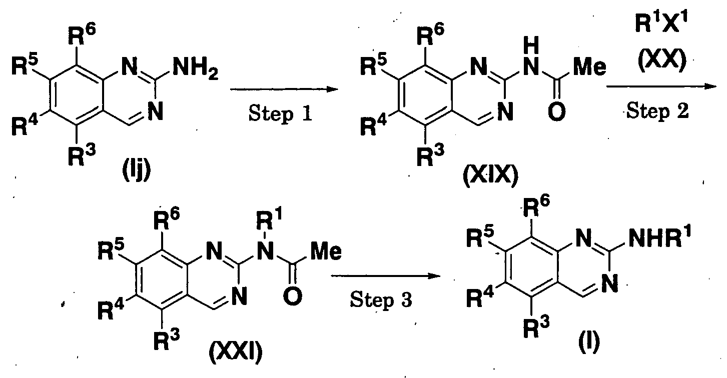 (wherein R¹, R³, R⁴, R⁵, R⁸ and X¹ have the same meanings as defined above, respectively.)

### Step 1

Compound (XIX) can be prepared by reacting Compound (Ij) with 1 to 1000 equivalents of acetylating reagent in the presence or absence of a solvent, optionally in the presence of 1 to 1000 equivalents of a base.

The solvent may be any inert solvent to the reaction, and include, but not limit to, for example, acetonitrile, dichloromethane, chloroform, THF, dioxane, acetone, DMSO, DMF, and the like, and preferably dichloromethane can be used.

As the base, pyridine, triethylamine, diisopropylethylamine, N-methylmorpholine, DBU, or the like, preferably pyridine or triethylamine can be used. In addition, for example, the base such as pyridine may also be used as a solvent in this reaction.

As the acetylating reagent, acetyl chloride, acetic anhydride, or the like can be used.

The reaction is completed for about 5 minutes to 48 hours at a temperature between 0°C and the boiling point of the solvent used, preferably at 80°C.

### Step 2

Compound (XXI) can be prepared by reacting Compound (XIX) with 1 to 20 equivalents of Compound (XX) in the presence of 1 to 20 equivalents of a base in a solvent.

The solvent may be any inert solvent to the reaction, and include, but not limit to, for example, dimethoxyethane, DMF, dioxane, THF, diethyl ether, diisopropyl ether, benzene, toluene, xylene, pyridine, N,N-dimethylimidazolidinone, N-methylpyrrolidone, sulforane, and the like, and preferably THF or DMF can be used.

As the base, sodium hydride, potassium tert-butoxide, potassium acetate, potassium carbonate, cesium carbonate, sodium carbonate, sodium hydrogencarbonate, sodium hydroxide, lithium hydroxide, potassium hydroxide, potassium phosphate, or the like, preferably sodium hydride can be used.

Compound (XX) is commercially available.

### Step 3

Compound (I) can be prepared by reacting Compound (XXI) with 1 to 200 equivalents of a base in a solvent.

The solvent may be any inert solvent to the reaction, and include, but not limit to, for example, methanol, ethanol, 1-propanol, acetone, methyl ethyl ketone, diethyl ether, THF, dioxane, DMF, dichloromethane, 1,2-dichloroethane, mixed solvent prepared by mixing at least any one solvent selected from the above with water in a suitable ratio between 100:1 to 1:100, and the like, and preferably methanol can be used.

As the base, potassium carbonate, cesium carbonate, sodium carbonate, sodium hydroxide, potassium hydroxide, lithium hydroxide, or the like, preferably potassium carbonate can be used.

The reaction is completed for about 5 minutes to 48 hours at a temperature between 0°C and the boiling point of the solvent used, preferably at room temperature.

The intermediates and the desired compounds in the above-mentioned Production Methods may be isolated and purified according to an isolation/purification generally employed in organic synthetic chemistry, for example, filtration, extraction, washing, drying, concentration, recrystallization, various types of chromatography, and the like. Also, the intermediate may be subjected to the next step without further purification.

To obtain a salt of Compound (I), when Compound (I) is obtained as a salt form, the salt of Compound (I) may be purified as it is. When Compound (I) is obtained in a free form, it may be dissolved or suspended in a suitable solvent, followed by addition of an acid or a base, then the resulting salt may be isolated and purified.

Compound (I) and a pharmaceutically acceptable salt thereof may exist in the form of adducts with water or various solvents, and these adducts are also within the scope of the invention.

Examples of Compounds (I) obtained according to the above-mentioned Production Methods are shown in Table 1 to Table 10.

**Table 1-1**

| | | | |
|---|---|---|---|
| 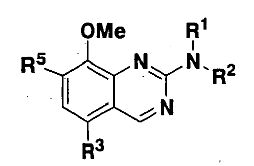 | | | |

| **Compound No.** | **NR**^{**1**}**R**^{**2**} | **R**^{**3**} | **R**^{**5**} |
|---|---|---|---|
| **1** | **NH**_{**2**} | 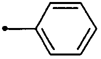 | **Br** |
| **2** | **NH**_{**2**} | 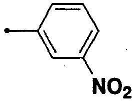 | **Br** |
| **3** | **NH**_{**2**} | 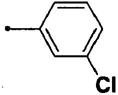 | **Br** |
| **4** | **NMe**_{**2**} | 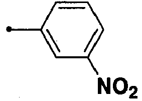 | **Br** |
| **5** | **NMe**_{**2**} | 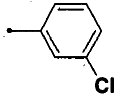 | **Br** |
| **6** | **NMe**_{**2**} | 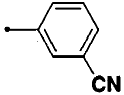 | **Br** |
| **7** | **NEt**_{**2**} | 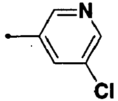 | **Br** |
| **8** | **NHMe** | 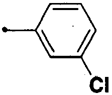 | **Br** |
| **9** | **NHMe** | 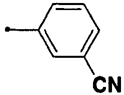 | **Br** |
| **10** | **NHMe** | 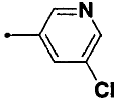 | **Br** |

**Table 1-2**

| | | | |
|---|---|---|---|
| 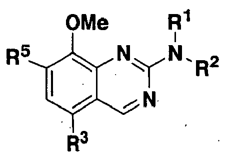 | | | |

| **Compound No.** | **NR**^{**1**}**R**^{**2**} | **R**^{**3**} | **R**^{**5**} |
|---|---|---|---|
| **11** | **NH**_{**2**} | 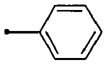 | 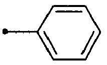 |
| **12** | **NH**_{**2**} | 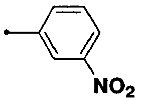 | 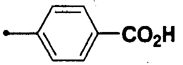 |
| **13** | **NH**_{**2**} | 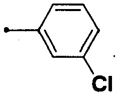 | 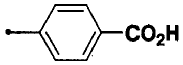 |
| **14** | **NMe**_{**2**} | 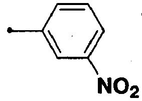 | 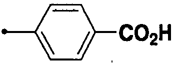 |
| **15** | **NMe**_{**2**} | 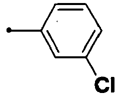 | 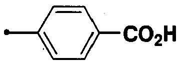 |
| **16** | **NMe**_{**2**} | 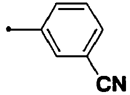 | 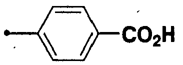 |
| **17** | **NEt**_{**2**} | 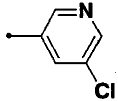 | 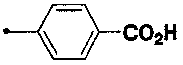 |
| **18** | **NHMe** | 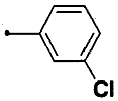 | 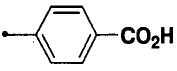 |
| **19** | **NHMe** | 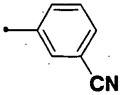 | 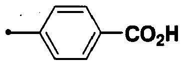 |
| **20** | **NHMe** | 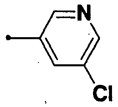 | 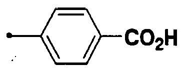 |

**Table 1-3**

| | | | |
|---|---|---|---|
| 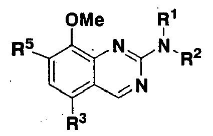 | | | |

| **Compound No.** | **NR**^{**1**}**R**^{**2**} | **R**^{**3**} | **R**^{**5**} |
|---|---|---|---|
| **21** | **NMe**_{**2**} | 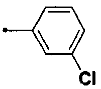 | 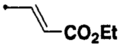 |
| **22** | **NH**_{**2**} | 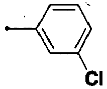 | 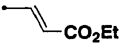 |
| **23** | **NH**_{**2**} | 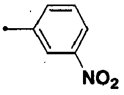 | 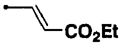 |
| **24** | **NMe**_{**2**} | 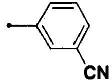 | 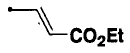 |
| **25** | **NEt**_{**2**} | 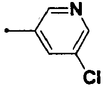 | 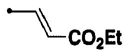 |
| **26** | **NHMe** | 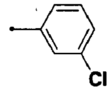 | 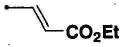 |
| **27** | **NH**_{**2**} | 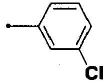 | 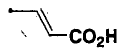 |
| **28** | **NMe**_{**2**} | 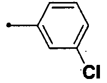 | 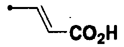 |
| **29** | **NH**_{**2**} | 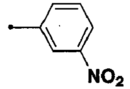 | 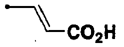 |
| **30** | **NMe**_{**2**} | 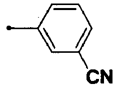 | 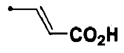 |
| **31** | **NEt**_{**2**} | 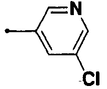 | 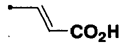 |
| **32** | **NEt**_{**2**} | 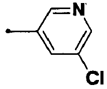 | 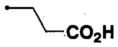 |
| **33** | **NH**_{**2**} | 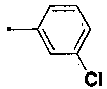 | 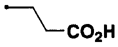 |
| **34** | **NMe**_{**2**} | 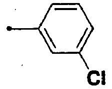 | 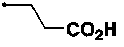 |
| **35** | **NHMe** | 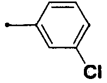 | 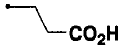 |

**Table 1-4**

| | | | |
|---|---|---|---|
| 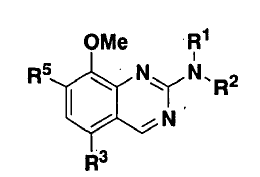 | | | |

| **Compound No.** | **NR**^{**1**}**R**^{**2**} | **R**^{**3**} | **R**^{**5**} |
|---|---|---|---|
| **36** | **NMe**_{**2**} | 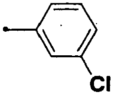 | 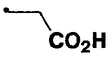 |
| **37** | **NMe**_{**2**} | 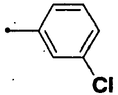 | 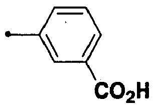 |
| **38** | **NEt**_{**2**} | 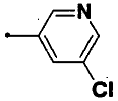 | 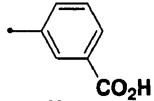 |
| **39** | **NMe**_{**2**} | 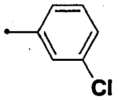 | 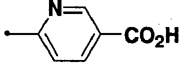 |
| **40** | **NMe**_{**2**} | 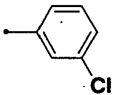 | 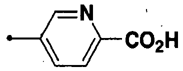 |
| **41** | **NMe**_{**2**} | 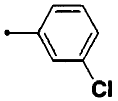 | 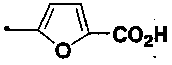 |
| **42** | **NMe**_{**2**} | 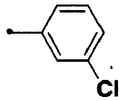 | 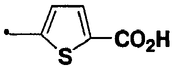 |
| **43** | **NMe**_{**2**} | 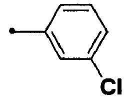 | 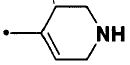 |
| **44** | **NMe**_{**2**} | 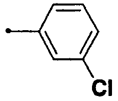 | 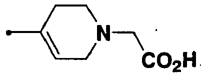 |

**Table 2-1**

| | | | | | |
|---|---|---|---|---|---|
| 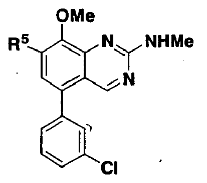 | | | | | |

| **Compound No.** | **R**^{**5**} | **Compound No.** | **R**^{**5**} | **Compound No.** | **R**^{**5**} |
|---|---|---|---|---|---|
| **45** | 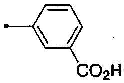 | **58** | 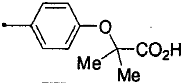 | **71** | 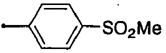 |
| **46** | 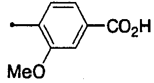 | **59** | 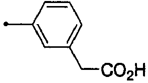 | **72** | 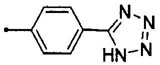 |
| **47** | 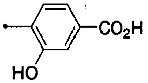 | **60** | 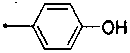 | **73** | 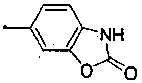 |
| **48** | 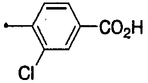 | **61** | 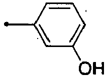 | **74** | 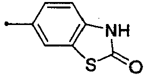 |
| **49** | 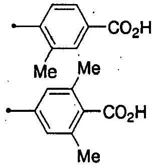 | **62** | 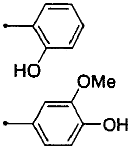 | **75** | 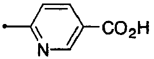 |
| **50** | | **63** | | **76** | 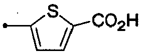 |
| **51** | 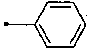 | **64** | 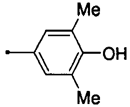 | **77** | 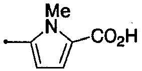 |
| **52** | 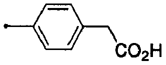 | **65** | 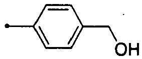 | **78** | 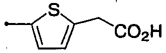 |
| **53** | 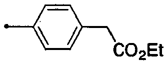 | **66** | 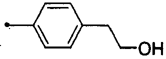 | **79** | 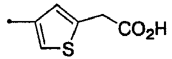 |
| **54** | 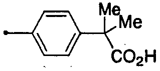 | **67** | 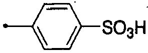 | **80** | 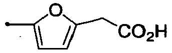 |
| **55** | 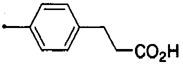 | **68** | 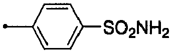 | **81** | 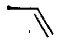 |
| **56** | 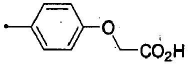 | **69** | 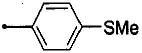 | **82** | 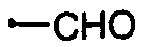 |
| **57** | 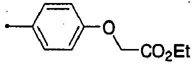 | **70** | 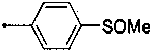 | | |

**Table 2-2**

| | | | |
|---|---|---|---|
| 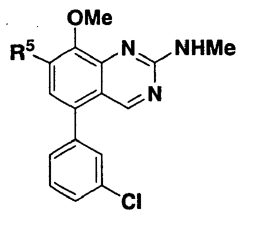 | | | |

| **Compound No.** | **R**^{**5**} | **Compound No.** | **R**^{**5**} |
|---|---|---|---|
| **83** | 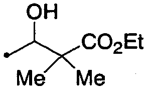 | **90** | 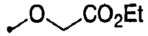 |
| **84** | 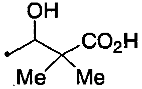 | **91** | 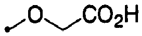 |
| **85** | 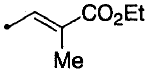 | **92** | 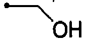 |
| **86** | 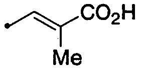 | **93** | 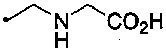 |
| **88** | 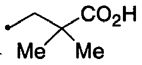 | **94** | 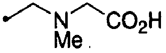 |
| **89** | 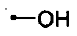 | **95** | 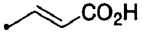 |
| | | **96** | 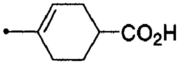 |

**Table 3**

| | | | |
|---|---|---|---|
| 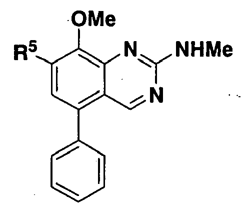 | | | |

| **Compound No.** | **R**^{**5**} | **Compound No.** | **R**^{**5**} |
|---|---|---|---|
| **87** | 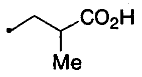 | **99** | 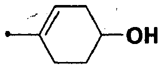 |
| **97** | 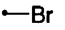 | **100** | 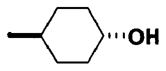 |
| **98** | 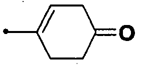 | **101** | 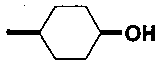 |

**Table 4**

| | | | |
|---|---|---|---|
| 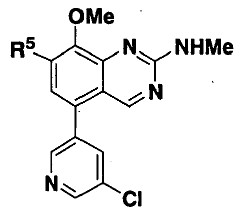 | | | |

| **Compound No.** | **R**^{**5**} | **Compound No.** | **R**^{**5**} |
|---|---|---|---|
| **102** | 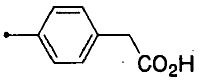 | **105** | 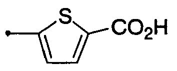 |
| **103** | 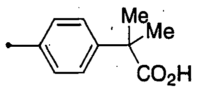 | **106** | 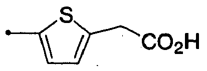 |
| **104** | 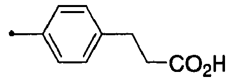 | | |

**Table 5**

| | | |
|---|---|---|
| 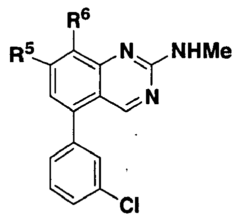 | | |

| **Compound No.** | **R**^{**5**} | |
|---|---|---|
| **107** | 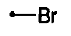 | 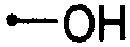 |
| **108** | 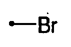 | 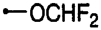 |
| **109** | 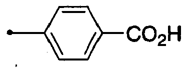 | 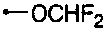 |
| **110** | 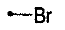 | 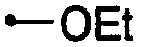 |
| **111** | 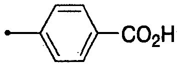 | 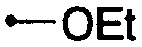 |
| **112** | 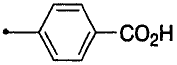 | 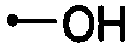 |
| **113** | 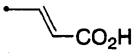 | 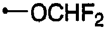 |

**Table 6**

| | | | |
|---|---|---|---|
| 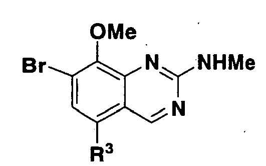 | | | |

| **Compound No.** | **R**^{**3**} | **Compound No.** | **R**^{**3**} |
|---|---|---|---|
| **114** | 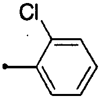 | **123** | 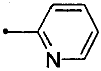 |
| **115** | 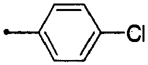 | **124** | 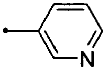 |
| **116** | 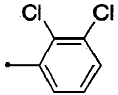 | **125** | 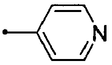 |
| **117** | 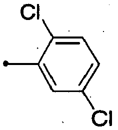 | **126** | 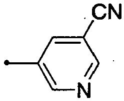 |
| **118** | 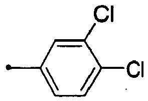 | **127** | 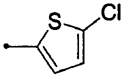 |
| **119** | 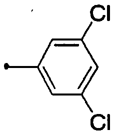 | **128** | 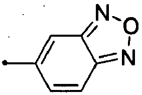 |
| **120** | 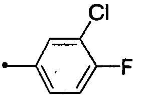 | **129** | 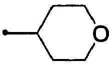 |
| | | **130** | 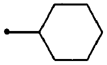 |
| **121** | 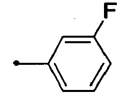 | **131** | 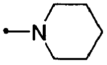 |
| **122** | 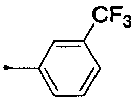 | **132** | 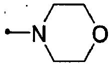 |

**Table 7**

| | | | |
|---|---|---|---|
| 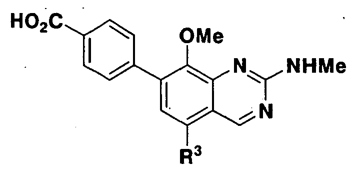 | | | |

| **Compound No.** | **R**^{**3**} | **Compound No.** | **R**^{**3**} |
|---|---|---|---|
| **133** | 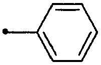 | **143** | 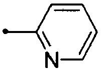 |
| **134** | 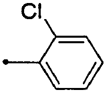 | **144** | 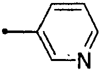 |
| **135** | 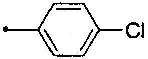 | **145** | 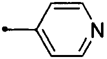 |
| **136** | 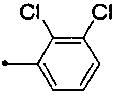 | **146** | 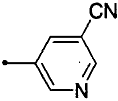 |
| **137** | 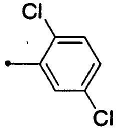 | **147** | 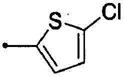 |
| **138** | 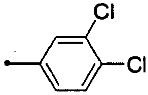 | **148** | 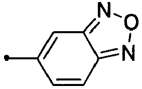 |
| **139** | 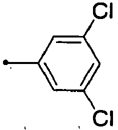 | **149** | 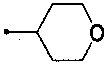 |
| **140** | 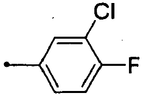 | **150** | 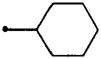 |
| **141** | 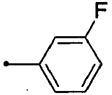 | **151** | 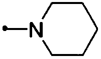 |
| **142** | 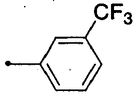 | **152** | 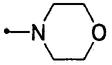 |

**Table 8**

| | | |
|---|---|---|
| 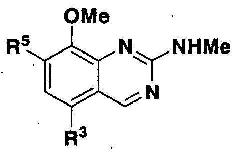 | | |

| **Compound No.** | **R**^{**3**} | **R**^{**5**} |
|---|---|---|
| **153** | 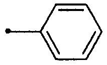 | 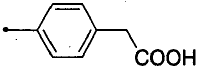 |
| **154** | 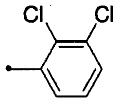 | 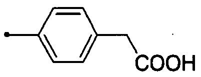 |
| **155** | 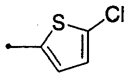 | 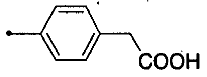 |
| **156** | 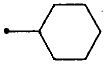 | 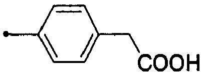 |
| **157** | 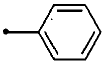 | 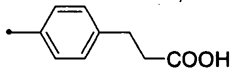 |
| **158** | 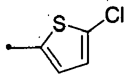 | 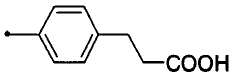 |

**Table 9**

| | | |
|---|---|---|
| 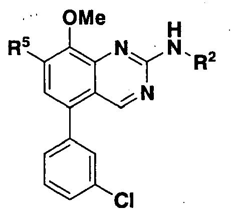 | | |

| **Compound No.** | **R**^{**2**} | **R**^{**5**} |
|---|---|---|
| **159** | 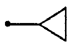 | 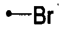 |
| **160** | 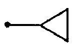 | 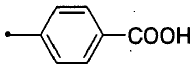 |
| **161** | 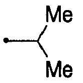 | 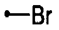 |
| **162** | 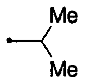 | 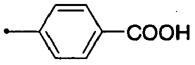 |
| **163** | 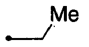 | 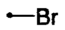 |
| **164** | 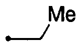 | 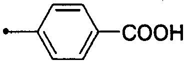 |
| **165** | 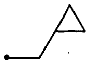 | 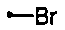 |
| **166** | 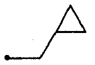 | 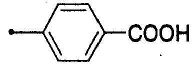 |
| **167** | 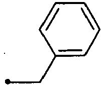 | 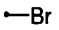 |
| **168** | 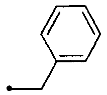 | 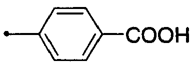 |

**Table 10**

| | | |
|---|---|---|
| 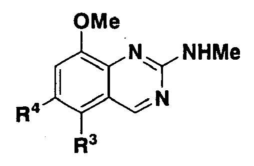 | | |

| **Compound No.** | **R**^{**3**} | **R**^{**4**} |
|---|---|---|
| **169** | 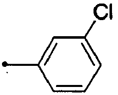 | 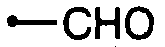 |
| **170** | 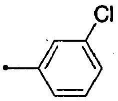 | 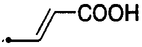 |
| **171** | 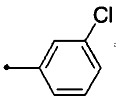 | 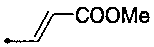 |
| **172** | 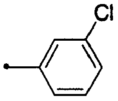 | 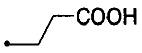 |
| **173** | 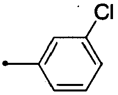 | 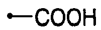 |
| **174** | 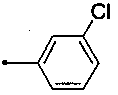 | 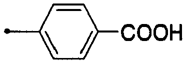 |
| **175** | 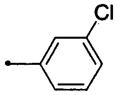 | 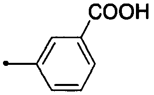 |
| **176** | 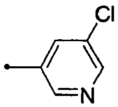 | 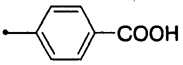 |

Next, pharmacological effects of some typical Compounds (I) are described below with reference to Experimental Example.

### Experimental Example:

### Recombinant Human PDE-IV-Inhibiting Test

A human PDE cDNA(HSPDE4A) was isolated from HL-60 cells. An expected amino acid sequence thereof is the same as the sequence (HSPDE4A4) reported by Bolger G., et al (Mol. Cell. Biol., 1993, Vol. 13, p.6558). The recombinant protein was expressed, using Sf9 insect cells. The PDE activity was measured according to the following two-step process, based on the method by Kincid R. L. and Manganiello V. C. (Method. Enzymol., 1988, Vol. 159, p.457). [³H]cAMP (final concentration :1 µmol/L) was used as the substrate. The reaction was performed in a standard mixture containing N,N-bis(2-hydroxyethyl)-2-aminoethanesulfonic acid (50 mmol/L, pH 7.2), magnesium chloride (1 mmol/L) and soybean trypsin inhibitor (0.1 mg/mL). The reaction was started by the addition of enzyme, and the reaction mixture was incubated at 30°C for 10 to 30 minutes. The reaction was stopped by hydrochloric acid, and the formed 5'-AMP was completely decomposed by 5'-nucleotidase. The resulting mixture was treated with chromatography of DEAE-Sephadex A-25, and the eluted [³H]adenosine was counted with a scintillation counter. The test compounds were dissolved in DMSO, and added to the system (concentration 1.7 %).

In this experiment, Compounds 12, 13, 14, 15, 18, 19, 20, 35, 37 and 43 showed an enzyme-inhibitory activity of 50 % or more at the concentration of 0.1 µmol/L.

Compound (I) or a pharmaceutically-acceptable salt thereof may be sufficiently useful as administered by itself. However, usually, Compound (I) or a pharmaceutically-acceptable salt thereof is preferably provided as various types of pharmaceutical preparations. Such pharmaceutical preparations are used for animals and humans.

The pharmaceutical preparations according to the present invention may comprise Compound (I) or a pharmaceutically acceptable salt thereof alone as an active ingredient. Alternatively, the pharmaceutical preparations may comprise a mixture of Compound (I) or a pharmaceutically acceptable salt thereof with any effective ingredient used for another treatment. Furthermore, these pharmaceutical preparations are prepared by mixing the active ingredient(s) with one or more pharmaceutically acceptable carrier(s) and then employing any method well-known in the technical field of pharmaceutics.

As for administration routes, it is preferred to select the most effective route of administration. Examples of the administration routes include oral administration and parenteral administration such as intravenous administration.

As for the dosage form, for example, tablets, injections and the like are included.

For example, the tablet suitable for oral administration can be prepared with, for example, excipients such as lactose and mannitol; disintegrants such as starch; lubricants such as magnesium stearate; binders such as hydroxypropylcellulose; surfactants such as a fatty acid ester; plasticizers such as glycerin; preseravative such as benzoic acid and p-hydroxybenzoate; and the like.

For example, the injections suitable for parenteral administration preferably comprise a sterilized aqueous preparation containing the active compound and being isotonic to blood of a recipient. Solutions for injections are prepared, using, for example, a carrier of saline solution, glucose solution, or a mixture of saline water and glucose solution.

The dose and the frequency of administration of Compound (I) or a pharmaceutically acceptable salt thereof may vary depending on the administration form, the age and body weight of a patient, nature and severity of the condition to be treated, and the like. In oral administration, the dosage may be 0.01 mg/adult to 1 g/adult, preferably 0.05 to 50 mg/adult, once or a few times a day. In parenteral administration such as intravenous administration, the dosage may be 0.001 to 100 mg/adult, preferably 0.01 to 50 mg/adult, once or a few times a day. However, the dose and the frequency of administration may vary depending upon the above-mentioned various conditions.

The invention is described in detail with reference to the following Reference Examples and Examples. However, the invention is not limited to the following Reference Examples and Examples.

Proton nuclear magnetic resonance (¹H NMR) spectra in Reference Examples and Examples are measured at 270 MHz, unless otherwise specifically indicated. Depending on the compounds and the conditions measured, exchangeable hydrogen may not always be observed clearly in proton nuclear magnetic resonance; in hydrochlorides, the hydrogen on the quaternary nitrogen atom may be observed. In addition, "br" means a broad signal.

### Reference Example 1:

### 6-Bromo-2-fluoro-3-methoxybenzaldehyde (Compound a)

To a solution of diisopropylamine (22.2 mL, 159 mmol) in THF (10 mL) was added a solution of n-butyllithium/n-hexane (1.56 mol/L, 93.8 mL, 146 mmol) at - 78°C under an argon atmosphere, then the mixture was stirred at 0°C for 10 minutes. This LDA-THF solution was cooled to -78°C, and a solution of 4-bromo-2-fluoroanisole (25.0 g, 122 mmol) in THF (10 ml) was dropped slowly thereto. After the reaction mixture was stirred at -78°C for 30 minutes, DMF (14.2 ml, 183 mmol) was dropped thereto at the same temperature, and the mixture was stirred at room temperature for 1 hour. Saturated aqueous ammonium chloride was added to the reaction mixture, then the mixture was extracted with ethyl acetate, and the resulting organic layer was washed with brine, and dried over anhydrous magnesium sulfate. The solvent was evaporated under reduced pressure, the precipitated crystal was collected by filtration, and washed with hexane/ethyl acetate (5/1) to give Compound a (22.8 g, 80 %) as a colorless crystal.
¹H NMR (CDCl₃, δ): 3.91 (s, 3H), 7.04 (t, J = 8.7 Hz, 1H), 7.39 (dd, J = 1.8, 8.7 Hz, 1H), 10.32 (d, J = 1.0 Hz, 1H).

### Reference Example 2:

### 2-Amino-5-bromo-8-methoxyquinazoline (Compound b)

Compound a (5.82 g, 25.0 mmol) and guanidine carbonate (4.95 g, 27.5 mmol) were stirred in DMA (80 mL) at 145°C for 2 hours. After the reaction mixture was cooled to room temperature, water was added thereto, and the precipitated crystal was collected by filtration. The resulting crystal was washed with water and ethanol in that order to give Compound b (4.37 g, 69 %) as a pale brown crystal.
¹H NMR (DMSO-d₆, δ): 3.87 (s, 3H), 7.06 (d, J = 8.4 Hz, 1H), 7.20 (brs, 2H), 7.37 (d, J = 8.4 Hz, 1H), 9.10 (s, 1H).
APCIMS m/z: [M+H]⁺ 256.

### Reference Example 3:

### 5-Bromo-2-dimethylamino-8-methoxyquinazoline (Compound c)

Compound c was prepared in the same manner as in Reference Example 2 using Compound a and 1,1-dimethylguanidine sulfate.
¹H NMR (CDCl₃, δ): 3.35 (s, 6H), 3.99 (s, 3H), 6.85 (d, J = 8.3 Hz, 1H), 7.26 (d, J = 8.3 Hz, 1H), 9.26 (s, 1H).

### Reference Example 4:

### 5-Bromo-2-diethylamino-8-methoxyquinazoline (Compound d)

Compound d was prepared in the same manner as in Reference Example 2 using Compound a and 1,1-diethylguanidine sulfate.
¹H NMR (CDCl₃, δ): 1.24 (t, *J* = 7.1 Hz, 6H), 3.80 (q, *J =* 7.1 Hz, 4H), 3.98 (s, 3H), 6.84 (d, J = 8.3 Hz, 1H), 7.23 (d, J = 8.3 Hz, 1H), 9.25 (s, 1H).

### Reference Example 5:

### 5-Bromo-8-methoxy-2-(methylamino)quinazoline (Compound e)

Compound e was prepared in the same manner as in Reference Example 2 using Compound a and methylguanidine sulfate.
¹H NMR (CDCl₃, δ): 3.15-3.17 (m, 3H), 4.00 (s, 3H), 5.49 (brs, 1H), 6.89 (d, J = 8.2 Hz, 1H), 7.32 (d, J = 8.2 Hz, 1H), 9.26 (s, 1H).

### Reference Example 6:

### 2-Amino-8-methoxy-5-phenylquinazoline (Compound f)

A mixture of Compound b (4.37 g, 17.2 mmol), phenylboronic acid (2.52 g, 20.7 mmol), tetrakis(triphenylphosphine)palladium (994 mg, 0.860 mmol), sodium carbonate (3.65 g, 34.4 mmol), dioxane (40 mL) and water (20 mL) was heated under reflux for 3 hours under an argon atmosphere. After cooling to room temperature, water was added to the mixture, then the precipitated crystal was collected by filtration and washed with water and ethanol in that order to give compound f (3.92 g, 91 %) as a pale yellow crystal.
¹H NMR (DMSO-d₆, δ): 3.91 (s, 3H), 6.95 (brs, 2H), 7.06 (d, *J* = 8.0 Hz, 1H), 7.21 (d, *J* = 8.0 Hz; 1H), 7.41-7.54 (m, 5H), 8.86 (s, 1H).

### Reference Example 7:

### 2-Amino-8-methoxy-5-(3-nitrophenyl)quinazoline (Compound g)

Compound g was prepared in the same manner as in Reference Example 6 using Compound b and 3-nitrophenylboronic acid.
¹H NMR (CDCl₃, δ): 4.09 (s, 3H), 5.40 (brs, 2H), 7.14 (d, *J* = 8.0 Hz, 1H), 7.18 (d, *J* = 8.0 Hz, 1H), 7.67 (t, *J* = 8.0 Hz, 1H), 7.76-7.79 (m, 1H), 8.28-8.33 (m, 2H), 8.96 (s, 1H).

### Reference Example 8:

### 2-Amino-5-(3-chlorophenyl)-8-methoxyquinazoline (Compound h)

Compound h was prepared in the same manner as in Reference Example 6 using Compound b and 3-chlorophenylboronic acid.
¹H NMR (CDCl₃, δ): 4.07 (s, 3H), 5.46 (brs, 2H), 7.12 (s, 2H), 7.29-7.33 (m, 1H), 7.40-7.44 (m, 3H), 9.02 (s, 1H).

### Reference Example 9:

### 2-Dimethylamino-8-methoxy-5-(3-nitrophenyl)quinazoline (Compound i)

Compound i was prepared in the same manner as in Reference Example 6 using Compound c and 3-nitrophenylboronic acid.
¹H NMR (CDCl₃, δ): 3.35 (s, 6H), 4.06 (s, 3H), 7.07 (s, 2H), 7.64 (t, J = 7.9 Hz, 1H), 7.79-7.76 (m, 1H), 8.25-8.33 (m, 2H), 8.94 (s, 1H).

### Reference Example 10:

### 5-(3-Chlorophenyl)-2-dimethylamino-8-methoxyquinazoline (Compound j)

Compound j was prepared in the same manner as in Reference Example 6 using Compound c and 3-chlorophenylboronic acid.
¹H NMR (CDCl₃, δ): 3.34 (s, 6H), 4.05 (s, 3H), 7.01 (d, *J* = 7.9 Hz, 1H), 7.05 (d, J = 7.9 Hz, 1H), 7.31-7.44 (m, 4H), 9.01 (s, 1H).

### Reference Example 11:

### 5-(3-Cyanophenyl)-2-dimethylamino-8-methoxyquinazoline (Compound k)

Compound k was prepared in the same manner as in Reference Example 6 using Compound c and 3-cyanophenylboronic acid.
¹H NMR (CDCl₃, δ): 3.35 (s, 6H), 4.06 (s, 3H), 7.00 (d, *J* = 8.0 Hz, 1H), 7.06 (d, *J* = 8.0 Hz, 1H), 7.57 (t, *J* = 8.0 Hz, 1H), 7.66-7.72 (m, 2H), 7.74-7.75 (m, 1H), 8.92 (s, 1H).

### Reference Example 12:

### 2-Diethylamino-8-methoxy-5-(4,4,5,5-tetramethyl-1,3,2-dioxaborolan-2-yl)quinazoline (Compound 1)

A mixture of Compound d (1.02 g, 3.29 mmol), bis(pinacolate)diboron (919 mg, 3.62 mmol), dichloro(diphenylphosphinoferrocene)palladium (131 mg, 0.16 mmol), potassium acetate (969 mg, 9.87 mg) and dioxane (20 mL) was heated under reflux for 2 hours under an argon atmosphere. After the reaction mixture was cooled to room temperature, saturated aqueous ammonium chloride was added thereto, and the mixture was extracted with ethyl acetate. Then the organic layer was washed with brine, and dried over anhydrous sodium sulfate. The solvent was evaporated under reduced pressure, and the residue was purified by silica gel column chromatography (ethyl acetate/hexane = 1/19) to give Compound 1 (1.05 g, 89 %) as pale yellow oil.
¹H NMR (CDCl₃, δ): 1.24 (t, J = 7.0 Hz, 6H), 1.38 (s, 12H), 3.80 (q, *J* = 7.0 Hz, 4H), 4.01 (s, 3H), 6.98 (d, *J =* 7.8 Hz, 1H), 7.66 (d, *J* = 7.8 Hz, 1H), 9.89 (s, 1H).

### Reference Example 13:

### 5-(5-Chloropyridine-3-yl)-2-diethylamino-8-methoxyquinazoline (Compound m)

Compound m was prepared in the same manner as in Reference Example 6 using Compound 1 and 3-chloro-5-trifluoromethanesulfoxypyridine.
¹H NMR (CDCl₃, δ): 1.25 (t, *J* = 7.0 Hz, 6H), 3.80 (q, *J* = 7.0 Hz, 4H), 4.04 (s, 3H), 6.99 (d, *J* = 7.9 Hz, 1H), 7.06 (d, *J* = 7.9 Hz, 1H), 7.78 (t, *J* = 2.1 Hz, 1H), 8.59 (d, *J* = 2.1 Hz, 1H), 8.62 (d, *J* = 2.1 Hz, 1H), 8.94 (s; 1H).

### Reference Example 14:

### 5-(3-Chlorophenyl)-8-methoxy-2-(methylamino)quinazoline (Compound n)

Compound n was prepared in the same manner as in Reference Example 6 using Compound e, and 3-chlorophenylboronic acid.
¹H NMR (DMSO-d₆, δ): 2.90-2.92 (m, 3H) , 3.94 (s, 3H), 7.08 (d, *J* = 8.0 Hz, 1H), 7.22 (d, J = 8.0 Hz, 1H), 7.42-7.45 (m, 2H), 7.51-7.53 (m, 3H), 8.84 (s, 1H)..

### Reference Example 15:

### 5-(3-Cyanophenyl)-8-methoxy-2-(methylamino)quinazoline (Compound o)

Compound o was prepared in the same manner as in Reference Example 6 using Compound e and 3-cyanophenylboronic acid.
¹H NMR (CDCl₃, δ): 3.15-3.17 (m, 3H), 4.07 (s, 3H), 5.43 (brs, 1H), 7.05 (d, J = 7.9 Hz, 1H), 7.10 (d, J = 7.9 Hz, 1H), 7.56-7.74 (m, 4H), 8.91 (s, 1H).

### Reference Example 16:

### 8-Methoxy-2-methylamino-5-(4,4,5,5-tetramethyl-1,3,2-dioxaborolan-2-yl)quinazoline (Compound p)

Compound p was prepared in the same manner as in Reference Example 12 using Compound e.
¹H NMR (CDCl₃, δ): 1.38 (s, 12H), 3.14-3.16 (m, 3H), 4.03 (s, 3H), 5.33-5.34 (m, 1H), 7.03 (d, *J* = 7.8 Hz, 1H), 7.73 (d, J = 7.8 Hz, 1H), 9.92 (s, 1H).

### Reference Example 17:

### 5-(5-Chloropyridine-3-yl)-8-methoxy-2-(methylamino)quinazoline (Compound q)

Compound q was prepared in the same manner as in Reference Example 6 using Compound p and 3-chloro-5-trifluoromethanesulfoxypyridine.
¹H NMR (CDCl₃, δ): 3.17 (d, J = 5.1 Hz, 3H), 4.07 (s, 3H), 5.46 (brs, 1H), 7.07 (d, J = 8.0 Hz, 1H), 7.11 (d, J = 8.0 Hz, 1H), 7.78 (t, *J* = 2.1 Hz, 1H), 8.59 (d, *J* = 2.1 Hz, 1H), 8.64 (d, J = 2.1 Hz, 1H), 8.95 (brs, 1H).

### Reference Example 18:

### 8-Methoxy-2-methylamino-5-phenylquinazoline (Compound v)

Compound v was prepared in the same manner as in Reference Example 6 using Compound e and phenylboronic acid.
¹H NMR (DMSO-d₆, δ): 2.92 (d, J = 4.8 Hz, 3H), 3.94 (s, 3H), 7.06 (d, J = 8.0 Hz, 1H), 7.23 (d, J = 8.0 Hz, 1H), 7.38 (d, J = 4.8 Hz, 1H), 7.44-7.54 (m, 5H), 8.86 (brs, 1H).

### Reference Example 19:

### 5-(2-Chlorophenyl)-8-methoxy-2-(methylamino)quinazoline (Compound w)

Compound w was prepared in the same manner as in Reference Example 6 using Compound e and 2-chlorophenylbororiic acid.
¹H NMR (DMSO-d₆, δ): 2.90 (d, J = 4.8 Hz, 3H), 3.95 (s, 3H), 6.99 (d, J = 8.0 Hz, 1H), 7.23 (d, J = 8.0 Hz, 1H), 7.41-7.46 (m, 2H), 7.48-7.53 (m, 2H), 7.60-7.65 (m, 1H), 8.42 (brs, 1H).

### Reference Example 20:

### 5-(4-Chlorophenyl)-8-methoxy-2-(methylamino)quinazoline (Compound x)

Compound x was prepared in the same manner as in Reference Example 6 using Compound e and 4-chlorophenylboronic acid.
¹H NMR (DMSO-d₆, δ): 2.90 (d, J = 4.8 Hz, 3H), 3.92 (s, 3H), 7.04 (d, J = 8.0 Hz, 1H), 7.21 (d, J = 8.0 Hz, 1H), 7.39 (d, J = 4.8 Hz, 1H), 7.48 (d, J = 8.4 Hz, 2H), 7.55 (d, J = 8.4 Hz, 2H), 8.83 (brs, 1H).

### Reference Example 21:

### 5-(2,3-Dichlorophenyl)-8-methoxy-2-(methylamino)quinazoline (Compound y)

Compound y was prepared in the same manner as in Reference Example 6 using Compound e and 2,3-dichlorophenylboronic acid.
¹H NMR (DMSO-d₆, δ): 2.89 (d, J = 4.8 Hz, 3H), 3.93 (s, 3H), 6.99 (d, J = 7.9 Hz, 1H.), 7.21 (d, J = 7.9 Hz, 1H), 7.39 (brs, 1H), 7.40 (d, J = 1.1 Hz, 1H), 7.48 (t, J = 7.7 Hz, 1H), 7.74 (dd, J = 1.1, 7.7 Hz, 1H), 8.43 (brs, 1H).

### Reference Example 22:

### 5-(,2,5-Dichlorophenyl)-8-methoxy-2-(methylamino)quinazoline (Compound z)

Compound z was prepared in the same manner as in Reference Example 6 using Compound e and 2,5-dichlorophenylboronic acid.
¹H NMR (DMSO-d₆, δ): 2.90 (d, J = 4.8 Hz, 3H), 3.95 (s, 3H), 7.02 (d, J = 8.0 Hz, 1H), 7.22 (d, J = 8.0 Hz, 1H), 7.43 (brs, 1H), 7.54 (d, J = 2.4 Hz, 1H), 7.57 (dd, J = 2.4, 8.4 Hz, 1H), 7.65 (d, J = 8.4 Hz, 1H), 8.46 (brs, 1H).

### Reference Example 23:

### 5-(3,4-Dichlorophenyl)-8-methoxy-2-(methylamino)quinazoline (Compound aa)

Compound aa was prepared in the same manner as in Reference Example 6 using Compound e and 3,4-dichlorophenylboronic acid.
¹H NMR (DMSO-d₆, δ): 2.90 (d, J = 4.8 Hz, 3H), 3.92 (s, 3H), 7.08 (d, J = 8.0 Hz, 1H), 7.21 (d, J = 8.0 Hz, 1H), 7.41 (brs, 1H), 7.45 (dd, J = 2.2, 8.3 Hz, 1H), 7.73 (d, J = 2.2 Hz, 1H), 7.73 (d, J = 8.3 Hz, 1H), 8.85 (brs, 1H).

### Reference Example 24:

### 5-(3,5-Dichlorophenyl)-8-methoxy-2-(methylamino)quinazoline (Compound ab)

Compound ab was prepared in the same manner as in Reference Example 6 using Compound e and 3,5-dichlorophenylboronic acid.
¹H NMR (DMSO-d₆, δ): 2.90 (d, J = 4.6 Hz, 3H), 3.92 (s, 3H), 7.09 (d, J = 7.9 Hz, 1H), 7.20 (d, J = 7.9 Hz, 1H), 7.42 (brs, 1H), 7.52 (d, J = 1.7 Hz, 2H), 7.67 (t, J = 1.7 Hz, 1H), 8.83 (brs, 1H).

### Reference Example 25:

### 5-(3-Chloro-4-fluorophenyl)-8-methoxy-2-(methylamino)quinazoline (Compound ac)

Compound ac was prepared in the same manner as in Reference Example 6 using Compound e and 3-chloro-4-fluorophenylboronic acid.
¹H NMR (CDCl₃, δ): 3.16 (d, J = 5.1 Hz, 3H), 4.05 (s, 3H), 5.43 (d, J = 5.1 Hz, 1H), 7.03 (d, J = 8.1 Hz, 1H), 7.07 (d, J = 8.1 Hz, 1H), 7.26-7.32 (m, 2H), 7.47 (dd, J = 5.7, 3.8 Hz, 1H), 8.96 (s, 1H).

### Reference Example 26:

### 5-(3-Fluorophenyl)-8-methoxy-2-(methylamino)quinazoline (Compound ad)

Compound ad was prepared in the same manner as in Reference Example 6 using Compound e and 3-fluorophenylboronic acid.
¹H NMR (DMSO-d₆, δ): 2.89 (d, J = 4.3 Hz, 3H), 3.97 (s, 3H), 7.14-7.23 (m, 2H), 7.30-7.37 (m, 2H), 7.54-7.62 (m, 2H), 8.37 (brs, 1H).

### Reference Example 27:

### 8-Methoxy-2-methylamino-5-(3-trifluoromethylphenyl)quinazoline (Compound ae)

Compound ae was prepared in the same manner as in Reference Example 6 using Compound e and 3-trifluoromethylphenylboronic acid.
¹H NMR (CDCl₃, δ): 3.16 (d, J = 5.2 Hz, 3H), 4.07 (s, 3H), 5.44 (d, J = 5.2 Hz, 1H), 7.08 (d, J = 8.0 Hz, 1H), 7.11 (d, J = 8.0 Hz, 1H), 7.59-7.77 (m, 4H), 8.95 (s, 1H).

### Reference Example 28:

### 8-Methoxy-2-methylamino-5-(2-pyridyl)quinazoline (Compound af)

Compound af was prepared in the same manner as in Reference Example 6 using Compound p and 2-bromopyridine.
¹H NMR (CDCl₃, δ): 3.16 (d, J = 5.0 Hz, 3H), 4.07 (s, 3H), 5.37 (d, J = 5.0 Hz, 1H), 7.10 (d, J = 8.0 Hz, 1H), 7.30 (d, J = 8.0 Hz, 1H), 7.31 (ddd, J = 1.0, 4.9, 7.8 Hz, 1H), 7.57 (dt, J = 1.0, 7.8 Hz, 1H), 7.81 (dt, J = 1.8, 7.8 Hz, 1H), 8.74 (ddd, J = 1.0, 1.8, 4.9 Hz, 1H), 9.45 (s, 1H).

### Reference Example 29:

### 8-Methoxy-2-methylamino-5-(3-pyridyl)quinazoline (Compound ag)

Compound ag was prepared in the same manner as in Reference Example 6 using Compound p and 3-bromopyridine.
¹H NMR (CDCl₃, δ): 3.16 (d, J = 5.1 Hz, 3H), 4.07 (s, 3H), 5.41 (brs, 1H), 7.08 (d, J = 7.9 Hz, 1H), 7.12 (d, J = 7.9 Hz, 1H), 7.42 (ddd, J = 0.8, 4.8, 7.8 Hz, 1H), 7.76 (dt, J = 2.0, 7.8 Hz, 1H), 8.68 (dd, J = 2.0, 4.8 Hz, 1H), 8.72 (dd, J = 0.8, 2.0 Hz, 1H), 8.95 (s, 1H).

### Reference Example 30:

### 8-Methoxy-2-methylamino-5-(4-pyridyl)quinazoline (Compound ah)

Compound ah was prepared in the same manner as in Reference Example 6 using Compound p and 4-bromopyridine.
¹H NMR (CDCl₃, δ): 3.16 (d, J = 5.1 Hz, 3H), 4.07 (s, 3H), 5.42 (d, J = 5.1 Hz, 1H), 7.10 (s, 2H), 7.38 (dd, J = 1.7, 4.5 Hz, 2H), 8.71 (dd, J = 1.7, 4.5 Hz, 2H), 9.01 (s, 1H).

### Reference Example 31:

### 5-(5-Cyano-3-pyridyl)-8-methoxy-2-(methylamino)quinazoline (Compound ai)

Compound ai was prepared in the same manner as in Reference Example 6 using Compound p and 3-cyano-5-trifluoromethanesulfoxypyridine.
¹H NMR (CDCl₃, δ): 3.17 (d, J = 5.1 Hz, 3H), 4.08 (s, 3H), 5.49 (brs, 1H), 7.07 (d, J = 7.9 Hz, 1H), 7.13 (d, J = 7.9 Hz, 1H), 8.05 (t, J = 2.0 Hz, 1H), 8.87 (brs, 1H), 8.91 (d, J = 2.0 Hz, 1H), 8.94 (d, J = 2.0 Hz, 1H).

### Reference Example 32:

### 5-(5-Chloro-2-thienyl)-8-methoxy-2-(methylamino)quinazoline (Compound aj)

Compound aj was prepared in the same manner as in Reference Example 6 using Compound p and 2-bromo-5-chlorothiophene.
¹H NMR (CDCl₃, δ): 3.16 (d, J = 5.1 Hz, 3H), 4.05 (s, 3H), 5.39 (brs, 1H), 6.91 (d, J = 3.8 Hz, 1H), 6.97 (d, J = 3.8 Hz, 1H), 7.04 (d, J = 8.1 Hz, 1H), 7.15 (d, J = 8.1 Hz, 1H), 9.27 (s, 1H).

### Reference Example 33:

### 5-(5-Benzofrazanyl)-8-methoxy-2-(methylamino)quinazoline (Compound ak)

Compound ak was prepared in the same manner as in Reference Example 6 using Compound p and 5-bromo benzofurazan.
¹H NMR (CDCl₃, δ): 3.17 (d, J = 5.1 Hz, 3H), 4.08 (s, 3H), 5.45 (brs, 1H), 7.11 (d, J = 8.1 Hz, 1H), 7.17 (d, J = 8.1 Hz, 1H), 7.54 (dd, J = 1.2, 9.2 Hz, 1H), 7.88 (t, J = 1.2 Hz, 1H), 7.93 (dd, J = 1.2, 9.2 Hz, 1H), 9.00 (brs, 1H).

### Reference Example 34:

### 5-(3,6-Dihydro-2H-pyran-4-yl)-8-methoxy-2-(methylamino)quinazoline (Compound al)

Compound al was prepared in the same manner as in Reference Example 6 using Compound p and 4-trifluoromethanesulfonyloxy-3,6-dihydro-2H-pyrane.
¹H NMR (DMSO-d₆, δ): 2.41 (brs, 2H), 2.90 (d, J = 4.8 Hz, 3H), 3.86 (brs, 2H), 3.89 (s, 3H), 4.26 (brs, 2H), 5.79 (brs, 1H), 6.97 (d, J = 7.9 Hz, 1H), 7.12 (d, J = 7.9 Hz, 1H), 7.31 (d, J = 4.8 Hz, 1H), 9.09 (brs, 1H).

### Reference Example 35:

### 8-Methoxy-2-methylamino-5-(4-tetrahydropyranyl)quinazoline (Compound am)

Compound al (330 mg, 1.22 mmol) was dissolved in ethanol (33 mL), and 10 % palladium-carbon (330 mg) was added thereto, and then the mixture was stirred under a hydrogen atmosphere at 40°C for 8 hours. The reaction mixture was filtered through Celite, and the filtrate was concentrated under reduced pressure. The residue was dissolved in dichloromethane (15 mL), and manganese dioxide (530 mg, 6.09 mmol) was added thereto, and then the mixture was stirred for 14 hours. The reaction mixture was filtered through Celite, and the filtrate was concentrated to give Compound am as a yellow solid (333 mg, 1.22 mmol, yield 100 %).
¹H NMR (CDCl₃, δ): 1.81-1.95 (m, 4H), 3.18 (brs, 3H), 3.42 (brs, 1H), 3.60-3.68 (m, 2H), 4.02 (s, 3H), 4.12-4.16 (m, 2H), 7.06 (brs, 2H), 9.35 (brs, 1H).

### Reference Example 36:

### 5-(1-Cyclohexenyl)-8-methoxy-2-(methylamino)quinazoline (Compound an)

Compound an was prepared in the same manner as in Reference Example 6 using Compound p and 1-trifluoromethanesulfonyloxycyclohexene.
¹H NMR (CDCl₃, δ): 1.69-1.86 (m, 4H), 2.21-2.27 (m, 2H), 2.31-2.36 (m, 2H), 3.15 (d, J = 5.1 Hz, 3H), 4.00 (s, 3H), 5.33 (d, J = 5.1 Hz, 1H), 5.72-5.75 (m, 1H), 6.92 (d, J = 8.0 Hz, 1H), 6.99 (d, J = 8.0 Hz, 1H), 9.18 (s, 1H).

### Reference Example 37:

### 5-Cyclohexyl-8-methoxy-2-(methylamino)quinazoline (Compound ao)

Compound ao was prepared in the same manner as in Reference Example 32 using Compound an.
¹H NMR (CDCl₃, δ): 1.26-1.37 (m, 2H), 1.47-1.55 (m, 4H), 1.79-1.85 (m, 1H), 1.89-1.97 (m, 4H), 3.15 (d, J = 5.1 Hz, 3H), 3.99 (s, 3H), 5.34 (brs, 1H), 6.99 (s, 2H), 9.29 (s, 1H).

### Reference Example 38:

### 8-Methoxy-2-methylamino-5-piperidinoquinazoline (Compound ap)

Compound e (1.07 g, 4.00 mmol), sodium t-butoxide (768 mg, 8.00 mmol) and [Pd(PBu₃)(µ-Br)]₂ (155 mg, 0.20 mmol) were dissolved in toluene (200 mL), and piperidine (3.95 mL, 40.0 mmol) was added thereto, then the mixture was stirred at 130°C for 24 hours. To the reaction mixture were added saturated aqueous ammonium chloride and ethyl acetate, and the organic layer was separated. The organic layer was washed with water and brine, dried over anhydrous magnesium sulfate, and concentrated under reduced pressure. The residue was purified by silica gel column chromatography (ethyl acetate/hexane = 1/1) to give compound an as a yellow solid (280 mg, 1.03 mmol, yield 26 %).
¹H NMR (CDCl₃, δ): 1.61 (brs, 2H), 1.75-1.83 (m, 4H), 2.95-2.99 (m, 4H), 3.15 (d, J = 5.0 Hz, 3H), 3.97 (s, 3H), 5.32 (d, J = 5.0 Hz, 1H), 6.65 (d, J = 8.3, Hz, 1H), 6.94 (d, J = 8.3 Hz, 1H), 9.32 (s, 1H).

### Reference Example 39:

### 8-Methoxy-2-methylamino-5-morpholinoquinazoline (Compound aq)

Compound aq was prepared in the same manner as in Reference Example 38 using Compound e and morpholine.
¹H NMR (CDCl₃, δ): 3.03 (t, J = 4.6 Hz, 4H), 6.15 (d, J = 5.1 Hz, 3H), 3.93 (t, J = 4.6 Hz, 4H), 3.98 (s, 3H), 5.38 (brs, 1H), 6.70 (d, J = 8.3 Hz, 1H), 6.96 (d, J = 8.3 Hz, 1H), 9.35 (s, 1H).

### Reference Example 40:

### 2-Bromo-4-fluoro-5-methoxybenzaldehyde (Compound ar)

To a mixture of water (90 mL), potassium bromide (33.9 g, 285 mmol) and bromine (7.33 mL, 142 mmol) was added 4-fluoro-3-methoxybenzaldehyde (8.77 g, 56.9 mmol), and the mixture was stirred at room temperature for 6 hours. The resulting crystal was collected by filtration to give Compound ar (11.9 g, 91 %).
¹H NMR (CDCl₃, δ): 3.93 (s, 3H), 7.37 (d, J = 10.0 Hz, 1H), 7.53 (d, J = 8.9 Hz, 1H), 10.23 (s, 1H).

### Reference Example 41:

### 2-Bromo-4-fluoro-5-methoxybenzaldehyde dimethyl acetal (Compound as)

Compound ar (6.00 g, 25.8 mmol) was dissolved in methanol (150 mL), and trimethyl orthoformate (8.45 mL, 77.3 mmol) and p-toluenesulfonic acid (490 mg, 2.58 mmol) were added thereto, and then the mixture was heated under reflux for 1.5 hours. To the reaction mixture were added saturated aqueous sodium hydrogencarbonate and chloroform, and the organic layer was separated. The organic layer was dried over anhydrous magnesium sulfate, and the solvent was evaporated under reduced pressure to give Compound as (7.19 g, 100 %).
¹H NMR (DMSO-d₆, δ): 3.30 (s, 6H), 3.85 (s, 3H), 5.41 (s, 1H), 7.22 (d, J = 9.2 Hz, 1H), 7.59 (d, J = 10.7 Hz, 1H).

### Reference Example 42:

### 2-Bromo-6-fluoro-3-dimethoxymethyl-5-methoxybenzaldehyde (Compound at)

Compound at was prepared in the same manner as in Reference Example 1 using Compound as.
¹H NMR (DMSO-d₆, δ): 3.35 (s, 6H), 3.91 (s, 3H), 5.56 (s, 1H), 7.45 (d, J = 8.8 Hz, 1H), 10.23 (s, 1H).

### Reference Example 43:

### 2-Amino-5-bromo-6-dimethoxymethyl-8-methoxyquinazoline (Compound au)

Compound au was prepared in the same manner as in Reference Example 2 using Compound at and guanidine carbonate.
¹H NMR (CDCl₃, δ) : 3.01 (s, 6H), 4.05 (s, 3H), 5.59 (brs, 2H), 5.71 (s, 1H), 7.32 (s, 1H), 9.42 (s, 1H).

### Reference Example 44:

### 2-Acetylamino-5-bromo-6-dimethoxymethyl-8-methoxyquinazoline (Compound av)

Compound au (6.00 g, 18.3 mmol) was dissolved in pyridine (60 mL), and acetic anhydride (5.18 mL, 54.9 mmol) was added thereto, and then the mixture was stirred at 90°C for 2 hours. The reaction mixture was concentrated under reduced pressure to about a half, and water was added to the residue. The resulting crystal was collected by filtration, and to the filtrate was added ethyl acetate, then the organic layer was separated. The organic layer was dried over anhydrous magnesium sulfate, and concentrated under reduced pressure. The residue was combined with the previously-obtained crystal, and slurried in methanol to give Compound av (4.60 g, 68 %).
¹H NMR (CDCl₃, δ): 2.59 (s, 3H), 3.47 (s, 6H), 4.07 (s, 3H), 5.75 (s, 1H), 7.44 (s, 1H), 8.64 (s, 1H), 9.70 (s, 1H).

### Reference Example 45:

### 5-Bromo-6-dimethoxymethyl-8-methoxy-2-(methylamino)quinazoline (Compound aw)

Compound av (1.50 g, 4.05 mmol) was dissolved in DMF (15 mL), and iodomethane (1.14 mL, 18.3 mmol) and potassium carbonate (1.12 g, 8.10 mmol) were added thereto, and then the mixture was stirred at 60°C for 6 hours. To the reaction mixture were added ethyl acetate and water, and the organic layer was separated. The organic layer was washed with water, dried over anhydrous magnesium sulfate, and concentrated under reduced pressure. The residue was dissolved in methanol (15 mL), potassium carbonate (1.38 g, 9.96 mmol) was added thereto, and then the mixture was stirred at 60°C for 3 hours. The reaction mixture was concentrated under reduced pressure, and ethyl acetate and water were added to the residue, then the organic layer was separated, and washed with water. The organic layer was dried over anhydrous magnesium sulfate, and concentrated under reduced pressure to give Compound aw (1.14 g, 84 %).
¹H NMR (CDCl₃, δ): 3.16 (d, J = 5.1 Hz, 3H), 3.45 (s, 6H), 4.03 (s, 3H), 5.59 (q, J = 5.1 Hz, 1H), 5.71 (s, 1H), 7.28 (s, 1H), 9.39 (s, 1H).

### Reference Example 46:

### 4-(4-Carboxyphenyl)-1-fluoro-2-methoxybenzene (Compound ax)

Compound ax was prepared in the same manner as in Reference Example 6 using 4-bromo-1-fluoro-2-methoxybenzene and 4-carboxyphenylboronic acid.
¹H NMR (DMSO-d₆, δ): 3.95 (s, 3H), 7.28-7.36 (m, 2H), 7.46-7.49 (m, 1H), 7.82 (d, J = 8.5 Hz, 2H), 8.01 (d, J = 8.5 Hz, 2H), 12.98 (br s, 1H).

### Reference Example 47:

### 5-Bromo-4-(4-carboxyphenyl)-1-fluoro-2-methoxybenzene (Compound ay)

Compound ay was prepared in the same manner as in Example 1 using Compound ax.
¹H NMR (DMSO-d₆, δ): 3.88 (s, 3H), 7.21 (d, J = 8.9 Hz, 1H), 7.54 (d, J = 8.2 Hz, 2H), 7.70 (d, J = 10.9 Hz, 1H), 8.02 (d, J = 8.2 Hz, 2H), 12.86 (brs, 1H).

### Reference Example 48:

### 3-Bromo-4-(4-carboxyphenyl)-1-fluoro-2-formyl-6-methoxybenzene (Compound az)

Compound az was prepared in the same manner as in Reference Example 1 using Compound ay.
¹H NMR (DMSO-d₆, δ): 3.92 (s, 3H), 7.47 (d, J = 8.7 Hz, 1H), 7.54 (d, J = 8.2 Hz, 2H), 8.04 (d, J = 8.2 Hz, 2H), 10.24 (s, 1H).

### Reference Example 49:

### 2-Amino-5-bromo-6-(4-carboxyphenyl)-8-methoxyquinazoline (Compound ba)

Compound ba was prepared in the same manner as in Reference Example 2 using Compound az and guanidine carbonate.
¹H NMR (DMSO-d₆, δ): 3.91 (s, 3H), 7.12 (s, 1H), 7.26 (brs, 2H), 7.60 (d, J = 8.2 Hz, 2H), 8.05 (d, J = 8.2 Hz, 2H), 9.28 (s, 1H).

### Reference Example 50:

### 2-Amino-5-bromo-6-(4-methoxycarbonylphenyl)-8-methoxyquinazoline (Compound bb)

Compound ba was dissolved in DMF (30 mL), and iodomethane (0.600 mL, 9.57 mmol) and potassium carbonate (1.98 g, 14.4 mmol) were added thereto, and then the mixture was stirred at room temperature for 40 minutes. To the reaction mixture was added water, and the resulting crystal was collected by filtration to give Compound bb (1.16 g, 76 %).
¹H NMR (DMSO-d₆, δ): 3.90 (s, 3H), 3.91 (s, 3H), 7.12 (s, 1H), 7.26 (brs, 2H), 7.64 (d, J = 8.3 Hz, 2H), 8.07 (d, J = 8.3 Hz, 2H), 9.28 (s, 1H).

### Reference Example 51:

### 5-Bromo-6-(4-methoxycarbonylphenyl)-8-methoxy-2-(methylamino)quinazoline (Compound bc)

Compound bc was prepared by the method described in Reference Examples 44 and 45 using Compound bb.
¹H NMR (DMSO-d₆, δ): 2.94 (d, J = 4.8 Hz, 3H), 3.90 (s, 3H), 3.93 (s, 3H), 7.14 (s, 1H), 7.64 (d, J = 8.3 Hz, 2H), 7.68 (q, J = 4.8 Hz, 1H), 8.06 (d, J = 8.3 Hz, 2H), 9.28 (s, 1H).

### Reference Example 52:

### 5-Bromo-6-(4-carboxyphenyl)-8-methoxy-2-(methylamino)quinazoline (Compound bd)

Compound bd was prepared in the same manner as in Example 27 using Compound bc.
¹H NMR (DMSO-d₆, δ): 2.95 (d, J = 3.8 Hz, 3H), 3.93 (s, 3H), 7.14 (s, 1H), 7.61 (d, J = 8.1 Hz, 2H), 7.69 (q, J = 3.8 Hz, 1H), 8.05 (d, J = 8.1 Hz, 2H), 9.27 (s, 1H), 13.03 (brs, 1H).

### Reference Example 53:

### 5-Bromo-6-(3-carboxyphenyl)-8-methoxy-2-(methylamino)quinazoline (Compound be)

Compound be was prepared by the method described in Reference Examples 46 to 52 using 4-bromo-1-fluoro-2-methoxybenzene and 3-carboxyphenylboronic acid.
¹H NMR (DMSO-d₆, δ): 2.94 (s, 3H), 3.93 (s, 3H), 7.14 (s, 1H), 7.52-7.72 (m, 3H), 7.95-8.00 (m, 2H), 9.27 (s, 1H).

### Example 1:

### 2-Amino-7-bromo-8-methoxy-5-phenylquinazoline (Compound 1)

Compound f (500 mg, 1.99 mmol) was suspended in acetic acid (10 mL), and bromine (2.00 mL, 3.90 mmol) was added thereto, and then the mixture was stirred at 60°C for 30 minutes. After the reaction mixture was cooled to room temperature, t saturated aqueous sodium hydrogenecarbonate was added thereto. The precipitated crystal was collected by filtration; and the resulting crystal was washed with water and ethanol in that order to give Compound 1 (381 mg, 58 %) as a pale yellow crystal.
¹H NMR (DMSO-*d*₆, δ): 3.94 (s, 3H), 7.09 (brs, 2H), 7.29-7.35 (m, 3H), 7.46-7.56 (m, 3H), 8.34 (s, 1H).
ESIMS *m*/*z*: [M+H]⁺ 330, 332.

### Example 2:

### 2-Amino-7-bromo-8-methoxy-5-('3-nitrophenyl)quinazoline (Compound 2)

Compound 2 was prepared in the same manner as in Example 1 using Compound g.
¹H NMR (DMSO-d₆, δ): 3.95 (s, 3H), 7.12 (brs, 2H), 7.38 (s, 1H), 7.80-7.83 (m, 2H), 8.15 (s, 1H), 8.33-8.36 (m, 1H), 8.39 (s, 1H).
APCIMS *m*/*z:* [M+H]⁺ 375, 377.

### Example 3:

### 2-Amino-7-bromo-5-(3-chlorophenyl)-8-methoxyquinazoline (Compound 3)

Compound 3 was prepared in the same manner as in Example 1 using Compound h.
¹H NMR (DMSO-d₆, δ): 3.95 (s, 3H), 7.10 (brs, 2H), 7.27-7.30 (m, 1H), 7.36 (s, 1H), 7.41-7.48 (m, 2H), 7.55-7.57 (m, 1H), 8.36 (s, 1H).
ESIMS *m*/*z:* [M+H]⁺ 364, 366.

### Example 4:

### 7-Bromo-2-dimethylamino-8-methoxy-5-(3-nitrophenyl)quinazoline (Compound 4)

Compound 4 was prepared in the same manner as in Example 1 using Compound i.
¹H NMR (CDCl₃, δ): 3.31 (s, 6H), 4.06 (s, 3H), 7.21 (s, 1H), 7.67-7.72 (m, 2H), 8.21-8.22 (m, 1H), 8.31-8.36 (m, 1H), 8.49 (s, 1H).
APCIMS m/z: [M+H]⁺ 403, 405.

### Example 5:

### 7-Bromo-5-(3-chlorophenyl)-2-dimethylamino-8-methoxyquinazoline (Compound 5)

Compound 5 was prepared in the same manner as in Example 1 using Compound j.
¹H NMR (CDCl₃, δ): 3.31 (s, 6H), 4.04 (s, 3H), 7.18-7.21 (m, 2H), 7.30-7.32 (m, 1H), 7.42-7.45 (m, 2H), 8.56 (s, 1H). APCIMS *m*/*z:* [M+H]⁺ 392, 394.

### Example 6:

### 7-Bromo-5-(3-cyanophenyl)-2-dimethylamino-8-methoxyquinazoline (Compound 6)

Compound 6 was prepared in the same manner as in Example 1 using Compound k.
¹H NMR (CDCl₃, δ): 3.31 (s, 6H), 4.05 (s, 3H), 7.20 (s, 1H), 7.54-7.64 (m, 3H), 7.74-7.78 (m, 1H), 8.47 (s, 1H).
APCIMS *m*/*z:* [M+H]⁺ 383, 385.

### Example 7:

### 7-Bromo-5-(5-chloropyridine-3-yl)-2-diethylamino-8-methoxyquinazoline (Compound 7)

Compound 7 was prepared in the same manner as in Example 1 using Compound m.
¹H NMR (CDCl₃, δ): 1.23 (t, *J* = 6.9Hz, 6H), 3.76 (q, *J* = 6.8 Hz, 4H), 4.04 (s, 3H), 7.19 (s, 1H), 7.68 (t, J = 2.0 Hz, 1H), 8.46 (d, J = 2.0 Hz, 1H), 8.52 (s, 1H), 8.67 (d, J = 2.0 Hz, 1H).

### Example 8:

### 7-Bromo-5-(3-chlorophenyl)-8-methoxy-2-(methylamino)quinazoline (Compound 8)

Compound 8 was prepared in the same manner as in Example 1 using Compound n.
¹H NMR (DMSO-*d*₆, δ): 2.88-2.89 (m, 3H), 3.96 (s, 3H), 7.27-7.31 (m, 1H), 7.38 (s, 1H), 7.42 (s, 1H), 7.56-7.57 (m, 3H), 8.35 (s, 1H).
APCIMS m/z: [M+H]⁺ 378, 380.

### Example 9:

### 7-Bromo-5-(3-cyanophenyl)-8-methoxy-2-(methylamino)quinazoline (Compound 9)

Compound 9 was prepared in the same manner as in Example 1 using Compound o.
¹H NMR (DMSO-*d*₆, δ): 2.86-2.88 (m, 3H), 3.95 (s, 3H), 7.38 (s, 1H), 7.56 (brs, 1H), 7.65-7.68 (m, 1H), 7.71-7.76 (m, 1H), 7.84-7.85 (m, 1H), 7.94-7.98 (m, 1H), 8.33 (s, 1H).
APCIMS *m*/*z:* [M+H]⁺ 369, 371.

### Example 10:

### 7-Bromo-5-(5-chloropyridine-3-yl)-8-methoxy-2-(methylamino)quinazoline (Compound 10)

Compound 10 was prepared in the same manner as in Example 1 using Compound q.
¹H NMR (CDCl₃, δ): 3.14 (d, *J* = 5.1 Hz, 3H), 4.07 (s, 3H), 5.46 (brs, 1H), 7.25 (s, 1H), 7.68 (t, *J* = 2.1 Hz, 1H), 8.46 (d, *J* = 2.1 Hz, 1H), 8.53 (brs, 1H), 8.69 (d, J = 2.1 Hz, 1H).

### Example 11:

### 2-Amino-8-methoxy-5,7-diphenylquinazoline (Compound 11)

A mixture of Compound 1 (200 mg, 0.60 mmol), phenylboronic acid (88 mg, 0.72 mmol), tetrakis(triphenylphosphine)palladium (35 mg, 0.030 mmol), sodium carbonate (127 mg, 1.20 mmol), dioxane (4 mL) and water (2 mL) was heated under reflux for 2 hours under an argon atmosphere. The reaction mixture was cooled to room temperature, water was added thereto, and the precipitated crystal was collected by filtration. The resulting crystal was washed with water and ethanol in that order to give Compound 11 (137 mg, 70 %) as a pale yellow crystal.
¹H NMR (CDCl₃, δ): 4.08 (s, 3H), 5.38 (s, 2H), 7.11-7.29 (m, 11H), 8.83 (s, 1H).
ESIMS m/z: [M+H]⁺ 328.

### Example 12:

### 2-Amino-7-(4-carboxyphenyl)-8-methoxy-5-(3-nitrophenyl)quinazoline (Compound 12)

Compound 12 was prepared in the same manner as in Example 11 using Compound 2.
¹H NMR (DMSO-d₆, δ): 3.98 (s, 3H), 7.10 (brs, 2H), 7.20 (s, 1H), 7.28 (d, J = 8.4 Hz, 2H), 7.57-7.65 (m, 2H), 7.77 (d, *J =* 8.4 Hz, 2H), 8.01-8.02 (m, 1H), 8.14-8.21 (m, 1H), 8.60 (s, 1H), 12.95 (brs, 1H).
APCIMS *m*/*z:* [M+H]⁺ 417.

### Example 13:

### 2-Amino-7-(4-carboxyphenyl)-5-(3-chlorophenyl)-8-methoxyquinazoline (Compound 13)

Compound 13 was prepared in the same manner as in Example 11 using Compound 3.
¹H NMR (DMSO-*d*₆, δ): 3.95 (s, 3H), 7.05 (brs, 2H), 7.12-7.15 (m, 2H), 7.23-7.24 (m, 1H), 7.26 (d, *J* = 8.2 Hz, 2H), 7.34-7.36 (m, 2H), 7.77 (d, *J* = 8.2 Hz, 2H), 8.54 (s, 1H).
APCIMS *m*/*z*: [M+H]⁺ 406.

### Example 14:

### 7-(4-Carboxyphenyl)-2-dimethylamino-8-methoxy-5-(3-nitrophenyl)quinazoline (Compound 14)

Compound 14 was prepared in the same manner as in Example 11 using Compound 4.
¹H NMR (DMSO-d₆, δ): 3.25 (s, 6H), 4.01 (s, 3H), 7.23 (s, 1H), 7.28 (d, J = 8.3 Hz, 2H), 7.58-7.60 (m, 2H), 7.77 (d, *J* = 8.3 Hz, 2H), 8.02-8.03 (m, 1H), 8.14-8.20 (m, 1H), 8.68 (s, 1H).
APCIMS *m*/*z:* [M-H]⁻ 443.

### Example 15:

### 7-(4-Carboxyphenyl)-5-(3-chlorophenyl)-2-dimethylamino-8-methoxyquinazoline (Compound 15)

Compound 15 was prepared in the same manner as in Example 11 using Compound 5.
¹H NMR (DMSO-*d*_{*6*}, δ): 3.25 (s, 6H), 3.99 (s, 3H), 7.12-7.16 (m, 1H), 7.20 (s, 1H), 7.25-7.26 (m, 1H), 7.28 (d, J = 8.3 Hz, 2H), 7.35-7.41 (m, 2H), 7.79 (d, J = 8.3 Hz, 2H), 8.63 (s, 1H), 12.95 (brs, 1H).
APCIMS m/z: [M-H]⁻ 432.

### Example 16:

### 7-(4-Carboxyphenyl)-5-(3-cyanophenyl)-2-dimethylamino-8-methoxyquinazoline (Compound 16)

Compound 16 was prepared in the same manner as in Example 11 using Compound 6.
¹H NMR (DMSO-d₆, δ): 3.25 (s, 6H), 4.00 (s, 3H), 7.21 (s, 1H), 7.26 (d, *J* = 8.1 Hz, 2H), 7.45-7.54 (m, 2H), 7.70 (s, 1H), 7.76-7.79 (m, 1H), 7.78 (d, *J* = 8.1 Hz, 2H), 8.61 (s, 1H).
APCIMS m/z: [M+H]⁺ 425.

### Example 17:

### 7-(4-Carboxyphenyl)-5-(5-chloropyridine-3-yl)-2-diethylamino-8-methoxyquinazoline (Compound 17)

Compound 17 was prepared in the same manner as in Example 11 using Compound 7.
¹H NMR (DMSO-d₆, δ): 1.18 (t, *J* = 6.9 Hz, 6H), 3.73 (q, *J* = 6.9 Hz, 4H), 4.00 (s, 3H), 7.22 (s, 1H), 7.28 (d, *J* = 8.3 Hz, 2H), 7.81 (d, *J* = 8.3 Hz, 2H), 7.92 (t, *J* = 2.0 Hz, 1H), 8.22 (d, J = 2.0 Hz, 1H), 8.56 (d, *J* = 2.0 Hz, 1H), 8.67 (s, 1H), 12.98 (brs, 1H).
APCIMS m/z: [M+H]⁺ 463.

### Example 18:

### 7-(4-Carboxyphenyl)-5-(3-chlorophenyl)-8-methoxy-2-(methylamino)quinazoline (Compound 18)

Compound 18 was prepared in the same manner as in Example 11 using Compound 8.
¹H NMR (DMSO-*d*₆, δ): 2.92-2.93 (m, 3H), 3.98 (s, 3H), 7.13-7.16 (m, 1H), 7.19 (s, 1H), 7.24-7.25 (m, 1H), 7.26 (d, J = 8.4 Hz, 2H), 7.36-7.38 (m, 2H), 7.52 (brs, 1H), 7.78 (d, J = 8.4 Hz, 2H), 8.55 (s, 1H).
APCIMS *m*/*z*: [M+H]⁺ 420.

### Example 19:

### 7-(4-Carboxyphenyl)-5-(3-cyanophenyl)-8-methoxy-2-(methylamino)quinazoline (Compound 19)

Compound 19 was prepared in the same manner as in Example 11 using Compound 9.
¹H NMR (DMSO-*d*₆, δ): 2.90-2.92 (m, 3H), 3.97 (s, 3H), 7.19 (s, 1H), 7.23 (d, *J* = 8.3 Hz, 2H), 7.44-7.53 (m, 3H), 7.70 (s, 1H), 7.75-7.79 (m, 1H), 7.76 (d, *J* = 8.3 Hz, 2H), 8.52 (s, 1H).
APCIMS m/z: [M+H]⁺ 411.

### Example 20:

### 7-(4-Carboxyphenyl)-5-(5-chloropyridine-3-yl)-8-methoxy-2-(methylamino)quinazoline (Compound 20)

Compound 20 was prepared in the same manner as in Example 11 using Compound 10.
¹H NMR (DMSO-d₆, δ): 2.93 (d, J = 4.6 Hz, 3H), 3.99 (s, 3H), 7.22 (s, 1H), 7.27 (d, *J* = 8.2 Hz, 2H), 7.56 (brs, 1H), 7.81 (d, *J* = 8.2 Hz, 2H), 7.90 (t, *J* = 2.0 Hz, 1H), 8.24 (d, J = 2.0 Hz, 1H), 8.56 (d, *J* = 2.0 Hz, 1H), 8.60 (brs, 1H), 12.96 (brs, 1H).
APCIMS *m*/*z*: [M+H]⁺ 421.

### Example 21:

### 7-{E-[2-(ethoxycarbonyl)ethenyl]}-5-(3-chlorophenyl)-2-dimethylamino-8-methoxyquinazoline (Compound 21)

A mixture of Compound 5 (1.97 g, 5.00 mmol), ethyl acrylate (5.41 mL, 50.0 mmol), palladium acetate (113 mg, 0.500 mmol), 1,1-bis(diphenylphosphino)ferrocene (554 mg, 1.00 mmol), triethylamine (3.46 mL, 25.0 mmol) and DMF (20 mL) was stirred at 100°C for 16 hours under an argon atmosphere. After the reaction mixture was cooled to room temperature, water was added thereto, and the precipitated crystal was collected by filtration and washed with methanol. The resulting crystal was dissolved in ethyl acetate, and the solution was filtrated through Celite, followed by recrystallization to give Compound 21 (1.55 g, 75 %) as a pale yellow crystal.
¹H NMR (CDCl₃, δ): 1.28 (t, J = 7.1 Hz, 3H), 3.30 (s, 6H), 4.08 (s, 3H), 4.19 (q, *J* = 7.1 Hz, 2H), 6.34 (d, *J =* 15.9 Hz, 1H), 7.14-7.18 (m, 1H), 7.25-7.26 (m, 1H), 7.28-7.29 (m, 1H), 7.39-7.47 (m, 2H), 7.51 (d, *J =* 15.9 Hz, 1H), 8.60 (s, 1H).
APCIMS m/z: [M+H]⁺ 412.

### Example 22:

### 2-Amino-7-{E-[2-(ethoxycarbonyl)ethenyl]}-5-(3-chlorophenyl)-8-methoxyquinazoline (Compound 22)

Compound 22 was prepared in the same manner as in Example 21 using Compound 3.
¹H NMR (CDCl₃, δ): 1.29 (t, *J* = 7.1 Hz, 3H), 4.11 (s, 3H), 4.21 (q, *J* = 7.1 Hz, 2H), 5.54 (brs, 2H), 6.38 (d, *J* = 16.0 Hz, 1H), 7.15-7.18 (m, 1H), 7.28-7.31 (m, 2H), 7.42-7.51 (m, 2H), 7.51 (d, *J* = 16.0 Hz, 1H), 8.63 (s, 1H).
APCIMS m/z: [M+H]⁺ 384.

### Example 23:

### 2-Amino-7-{E-[2-(ethoxycarbonyl)ethenyl]}-8-methoxy-5-(3-nitrophenyl)quinazoline (Compound 23)

Compound 23 was prepared in the same manner as in Example 21 using Compound 2.
¹H NMR (DMSO-*d*₆, δ): 1.18 (t, *J* = 7.1 Hz, 3H), 3.97 (s, 3H), 4.10 (q, *J* = 7.1 Hz, 3H), 6.77 (d, *J* = 15.7 Hz, 1H), 7.13 (brs, 2H), 7.25 (d, *J* = 15.8 Hz, 1H), 7.58 (s, 1H), 7.80-7.87 (m, 2H), 8.16-8.17 (m, 1H), 8.38-8.42 (m, 2H).

### Example 24:

### 7-{E-[2-(ethoxycarbonyl)ethenyl]}-5-(3-cyanophenyl)-2-dimethylamino-8-methoxyquinazoline (Compound 24)

Compound 24 was prepared in the same manner as in Example 21 using Compound 6.
¹H NMR (CDCl₃, δ): 1.28 (t, *J* = 7.1 Hz, 3H), 3.33 (s, 6H), 4.09 (s, 3H), 4.19 (q, *J* = 7.1 Hz, 2H), 6.35 (d, *J* = 15.8 Hz, 1H), 7.25-7.26 (m, 1H), 7.39 (d, *J* = 15.8 Hz, 1H), 7.51-7.55 (m, 1H), 7.59-7.66 (m, 2H), 7.76-7.80 (m, 1H), 8.49 (s, 1H).
APCIMS *m*/*z:* [M+H]⁺ 403.

### Example 25:

### 7-{E-[2-(ethoxycarbonyl)ethenyl]}-5-(5-chloropyridine-3-yl)-2-diethylamino-8-methoxyquinazoline (Compound 25)

Compound 25 was prepared in the same manner as in Example 21 using Compound 7.
¹H NMR (CDCl₃, δ): 1.24 (t, J = 7.0 Hz, 3H), 1.28 (t, *J =* 6.9 Hz, 6H), 3.78 (q, J = 6.9 Hz, 4H), 4.08 (s, 3H), 4.20 (q, J = 7.0 Hz, 2H), 6.37 (d, J = 16 Hz, 1H), 7.25 (s, 1H), 7.41 (d, *J* = 16 Hz, 1H), 7.66 (t, *J* = 2.1 Hz, 1H), 8.42 (d, *J* = 2.1 Hz, 1H), 8.53 (s, 1H), 8.70 (d, *J* = 2.1 Hz, 1H).

### Example 26:

### 7-{E-[2-(ethoxycarbonyl)ethenyl]}-5-(3-chlorophenyl)-8-methoxy-2-(methylamino)quinazoline (Compound 26)

Compound 26 was prepared in the same manner as in Example 21 using Compound 8.
¹H NMR (DMSO-d₆, δ): 1.20 (t, *J* = 7.1 Hz, 3H), 2.90-2.92 (m, 3H), 4.02 (s, 3H), 4.11 (q, *J* = 7.1 Hz, 2H), 6.74 (d, *J* = 15.9 Hz, 1H), 7.29-7.31 (m, 1H), 7.30 (d, J = 15.9 Hz, 1H), 7.43 (s, 1H), 7.56-7.64 (m, 3H), 8.39 (brs, 1H).
APCIMS *m*/*z:* [M+H]⁺ 398.

### Example 27:

### 2-Amino-7-[E-(2-carboxyethenyl)]-5-(3-chlorophenyl)-8-methoxyquinazoline (Compound 27)

Compound 22 (200 mg, 0.521 mmol) was dissolved in THF (6 mL) and methanol (6 mL), and 5 % aqueous lithium hydroxide (6 mL) was added thereto, and the mixture was stirred at room temperature for 1 hour. After the reaction mixture was neutralized with 2 mol/L hydrochloric acid, the precipitated crystal was collected by filtration, and the resulting crystal was washed with water to give Compound 27 (166 mg, 90 %) as a pale yellow crystal.
¹H NMR (DMSO-d₆, δ): 4.00 (s, 3H), 6.60 (d, J = 15.9 Hz, 1H), 7.20 (brs, 2H), 7.23 (d, J = 15.9 Hz, 1H), 7.26-7.28 (m, 1H), 7.41 (s, 1H), 7.51 (s, 1H), 7.54-7.62 (m, 2H), 8.37 (s, 1H).
APCIMS m/z: [M+H]⁺ 356.

### Example 28:

### 7-[E-(2-carboxyethenyl)]-5-(3-chlorophenyl)-2-dimethylamino-8-methoxyquinazoline (Compound 28)

Compound 28 was prepared in the same manner as in Example 27 using Compound 21.
¹H NMR (DMSO-*d*₆, δ): 3.23 (s, 6H), 4.02 (s, 3H), 6.62 (d, *J* = 15.8 Hz, 1H), 7.25 (d, *J* = 15.8 Hz, 1H), 7.26-7.30 (m, 1H), 7.42 (s, 1H), 7.53 (s, 1H), 7.58-7.63 (m, 2H), 8.45 (s, 1H):
APCIMS *m*/*z*: [M+H]⁺ 384.

### Example 29:

### 2-Amino-7-[E-(2-carboxyethenyl)]-8-methoxy-5-(3-nitrophenyl)quinazoline (Compound 29).

Compound 29 was prepared in the same manner as in Example 27 using Compound 23.
¹H NMR (DMSO-*d*₆, δ): 4.01 (s, 3H), 6.65 (d, *J* = 15.8 Hz, 1H), 7.19 (d, J = 15.8 Hz, 1H), 7.22 (s, 2H), 7.55 (s, 1H), 7.65-7.73 (m, 1H), 7.77-.7.89 (m, 2H), 8.14-8.15 (m, 1H), 8.37-8.42 (m, 2H).
APCIMS m/z: [M-H]⁻ 365.

### Example 30:

### 7-[E-(2-carboxy)ethenyl]-5-(3-cyanophenyl)-2-dimethylamino-8-methoxyquinazoline (Compound 30)

Compound 30 was prepared in the same manner as in Example 27 using Compound 24.
¹H NMR (DMSO-*d*₆, δ): 3.23 (s, 6H), 4.03 (s, 3H), 6.63 (d, *J* = 15.8 Hz, 1H), 7.19 (d, *J* = 15.8 Hz, 1H), 7.55 (s, 1H), 7.66 (d, *J* = 7.7 Hz, 1H), 7.77 (t, *J* = 7.7 Hz, 1H), 7.86 (s, 1H), 8.01 (d, J = 7.7 Hz, 1H), 8.43 (s, 1H).
APCIMS m/z: [M+H]⁺ 375.

### Example 31:

### 7-[E-(2-carboxy)ethenyl]-5-(5-chloropyridine-3-yl)-2-diethylamino-8-methoxyquinazoline (Compound 31)

Compound 31 was prepared in the same manner as in Example 27 using Compound 25.
¹H NMR (DMSO-d₆, δ): 1.16 (t, J = 6.5 Hz, 6H), 3.71 (q, J = 6.5 Hz, 4H), 4.03 (s, 3H), 6.66 (d, J = 16 Hz, 1H), 7.18 (d, *J =* 16 Hz, 1H), 7.56 (s, 1H), 8.06 (dd, *J =* 1'.8 Hz, 2.4 Hz, 1H), 8.47 (d, J = 1.8 Hz, 1H), 8.51 (s, 1H), 8.80 (d, J = 2.4 Hz, 1H), 12.33 (brs, 1H).
APCIMS m/z: [M+H]⁺ 413.

### Example 32:

### 7-(2-Carboxyethyl)-5-(5-chloropyridine-3-yl)-2-diethylamino-8-methoxyquinazoline (Compound 32)

### Step 1:

Compound 25 (150 mg, 0.34 mmol) was dissolved in ethanol (1.5 mL) and THF (1.5 mL), and sodium borohydride (38 mg, 1.0 mmol) was added thereto, and then the mixture was heated under reflux for 1 hour under an argon atmosphere. After the reaction mixture was cooled to room temperature, water was added thereto, and the mixture was extracted with ethyl acetate. The organic layer was washed with brine, and dried over anhydrous magnesium sulfate. The solvent was evaporated under reduced pressure, and the residue was purified by silica gel thin-layer chromatography (ethyl acetate/hexane = 1/3) to give 7-(2-ethoxycarbonylethyl)-5-(5-chloropyridine-3-yl)-2-diethylamino-8-methoxyquinazoline (39 mg, 26 %) as pale yellow oil.
¹H NMR (CDCl₃, δ): 1.20 (t, *J* = 7.1 Hz, 3H), 1.22 (t, *J* = 7.1 Hz, 6H), 2.46 (t, J = 7.7 Hz, 2H), 2.79 (t, J = 7.7 Hz, 2H), 3.76 (q, J = 7.1 Hz, 4H), 4.04 (s, 3H), 4.07 (q, J = 7.1 Hz, 2H), 6.94 (s, 1H), 7.64 (t, *J* = 2.1 Hz, 1H), 8.42 (d, *J =* 2.1 Hz, 1H), 8.44 (s, 1H), 8.66 (d, *J =* 2.1 Hz, 1H).

### Step 2:

Compound 32 was prepared in the same manner as in Example 27 using the compound obtained in the step 1. ¹H NMR (DMSO-*d*₆, δ): 1.14 (t, *J* = 6.8 Hz, 6H), 2.43 (t, *J* = 7.7 Hz, 2H), 2.61 (t, *J* = 7.7 Hz, 2H), 3.68 (q, *J* = 6.8 Hz, 4H), 3.95 (s, 3H), 7.21 (s, 1H), 7.99 (t, J = 1.8 Hz, 1H), 8.39 (s, 1H), 8.46 (d, *J* = 1.8 Hz, 1H), 8.73 (d, *J* = 1.8 Hz, 1H).
APCIMS m/z: [M+H]⁺. 415.

### Example 33:

### 2-Amino-7-(2-carboxyethyl)-5-(3-chlorophenyl)-8-methoxyquinazoline (Compound 33)

Compound 33 was prepared in the same manner as in Example 32 using Compound 22.
¹H NMR (DMSO-d₆, δ): 2.42 (t, J = 7.5 Hz, 2H), 2.61 (t, J = 7.5 Hz, 2H), 3.90 (s, 3H), 6.85 (s, 2H), 7.15 (s, 1H), 7.24-7.28 (m, 1H), 7.37 (s, 1H), 7.52 (s, 1H), 7.54 (s, 1H), 8.27 (s, 1H).
APCIMS m/z: [M-H]⁻ 356.

### Example 34:

### 7-(2-Carboxyethyl)-5-(3-chlorophenyl)-2-dimethylamino-8-methoxyquinazoline (Compound 34)

Compound 34 was prepared in the same manner as in Example 32 using Compound 21.
¹H NMR (CDCl₃, δ): 2.49 (t, *J* = 7.8 Hz, 2H), 2.81 (t, *J =* 7.8 Hz, 2H), 3.30 (s, 6H), 4.02 (s, 3H), 6.94 (s, 1H), 7.13-7.17 (m, 1H), 7.26-7.27 (m, 1H), 7.40-7.43 (m, 2H), 8.50 (s, 1H).
APCIMS m/z: [M+H]⁺ 386.

### Example 35:

### 7-(2-Carboxyethyl)-5-(3-chlorophenyl)-8-methoxy-2-(methylamino)quinazoline (Compound 35).

Compound 35 was prepared in the same manner as in Example 32 using Compound 26.
¹H NMR (DMSO-d₆, δ): 2.41-2.47 (m, 2H), 2.60-2.66 (m, 2H), 2.87-2.89 (m, 3H), 3.94 (s, 3H), 7.18 (s, 1H), 7.24-7.31 (m, 2H), 7.38-7.39 (m, 1H), 7.53-7.55 (m, 2H), 8.29 (s, 1H).
APCIMS *m*/*z:* [M+H]⁺ 372.

### Example 36:

### 7-Carboxymethyl-5-(3-chlorophenyl)-2-dimethylamino-8-methoxyquinazoline (Compound 36)

### Step 1:

A mixture of Compound 5 (393 mg, 1.00 mmol), allyltributyltin (0.372 mL, 1.20 mmol), tetrakis(triphenylphosphine)palladium (58 mg, 0.050 mmol) and dioxane (5 mL) was heated under reflux for 3 hours under an argon atmosphere. After the reaction mixture was cooled to room temperature, water was added thereto, and the mixture was extracted with ethyl acetate. The organic layer was washed with brine, and dried over anhydrous magnesium sulfate. The solvent was evaporated under reduced pressure, and the residue was triturated with hexane. The resulting precipitate was collected by filtration to give 7-allyl-5-(3-chlorophenyl)-2-dimethylamino-8-methoxyquinazoline (287 mg, 81 %) as a pale yellow crystal.
¹H NMR (CDCl₃, δ): 3.20-3.23 (m, 2H), 3.31 (s, 6H), 4.04 (s, 3H), 4.88-4.95 (m, 1H), 5.01-5.06 (m, 1H), 5.80-5.93 (m, 1H), 6.92 (s, 1H), 7.18-7.21 (m, 1H), 7.26-7.27 (m, 1H), 7.38-7.40 (m, 2H), 8.56 (s, 1H).
APCIMS m/z: [M+H]⁺ 354.

### Step 2:

The compound (280 mg, 0.791 mmol) obtained in the step 1 was dissolved in THF (15 mL) and water (3 mL), and 2.5 % aqueous osmium tetroxide (0.16 mL, 0.016 mmol) and 50 % aqueous methylmorpholine-N-oxide (0.185 mL, 0.791 mmol) were added thereto, and then the mixture was stirred at room temperature for 24 hours. To the reaction mixture were added water and chloroform, and the mixture was extracted. The organic layer was washed with brine, and dried over anhydrous magnesium sulfate. The solvent was evaporated under reduced pressure, and the residue was purified by silica gel column chromatography (chloroform/methanol = 30/1) to give 5-(3-chlorophenyl)-7-(2,3-dihydroxypropyl)-2-dimethylamino-8-methoxyquinazoline (140 mg, 46 %) as a pale yellow crystal.
¹H NMR (DMSO-d₆, δ): 2.37-2.46 (m, 1H), 2.58-2.65 (m, 1H), 3.20 (s, 6H), 3.61-3.68 (1H, m), 3.95 (s, 3H), 4.48-4.52 (m, 1H), 4.56-4.60 (m, 1H), 7.20-7.24 (m, 2H), 7.28-7.33 (m, 2H), 7.42-7.43 (m, 1H), 7.51-7.53 (m, 2H), 8.36 (s, 1H).
APCIMS m/z: [M+H]⁺ 388.

### Step 3:

The compound (128 mg, 0.330 mmol) obtained in the step 2 was dissolved in acetic acid (3 mL), methanol (3 mL) and water (1.5 mL), and sodium periodate (85 mg, 0.396 mmol) was added thereto under ice-cooling. After being stirred for 30 minutes at room temperature, amidosulfonic acid (58 mg, 0.594 mmol) and chlorous acid (42 mg, 0.462 mmol) were added to the mixture, and the mixture stirred at room temperature for 4 hours. Aqueous sodium thiosulfate was added to the reaction mixture, and the mixture was extracted with chloroform. The organic layer was washed with brine, and dried over anhydrous magnesium sulfate. The solvent was evaporated under reduced pressure, and the residue was purified by silica gel column chromatography (chloroform/methanol = 10/1) to give Compound 36 (65 mg, 53 %) as a pale yellow crystal.
¹H NMR (CDCl₃, δ): 3.30 (s, 6H), 3.51 (s, 2H), 4.03 (s, 3H), 6.97 (s, 1H), 7.16-7.19 (m, 1H), 7.28-7.29 (m, 1H), 7.38-7.42 (m, 2H), 8.54 (s, 1H).
APGIMS m/z: [M+H]⁺ 372.

### Example 37:

### 7-(3-Carboxyphenyl)-5-(3-chlorophenyl)-2-dimethylamino-8-methoxyquinazoline (Compound 37)

Compound 37 was prepared in the same manner as in Example 11 using Compound 5 and 3-carboxyphenylboronic acid.
¹H NMR (DMSO-d₆, δ): 3.25 (s, 6H), 3.99 (s, 3H), 7.13-7.16 (m, 1H), 7.20 (s, 1H), 7.25 (s, 1H), 7.33-7.39 (m, 4H), 7.73-7.78 (m, 2H), 8.62 (s, 1H).
APCIMS m/z: [M-H]⁻ 432.

### Example 38:

### 7-(3-Carboxyphenyl)-5-(5-chloropyridine-3-yl)-2-diethylamino-8-methoxyquinazoline (Compound 38)

Compound 38 was prepared in the same manner as in Example 11 using Compound 7 and 3-carboxyphenylboronic acid.
¹H NMR (DMSO-*d*₆, δ): 1.18 (t, J = 6.9 Hz, 6H), 3.73 (q, *J* = 6.9 Hz, 4H), 4.00 (s, 3H), 7.22 (s, 1H), 7.39 (m, 2H), 7.71 (s, 1H), 7.78 (m, 1H), 7.92 (t, *J* = 2.1 Hz, 1H), 8.23 (d, *J =* 2.1 Hz, 1H), 8.54 (d, *J* = 2.1 Hz, 1H), 8.66 (s, 1H).
APCIMS m/z: [M+H]⁺ 463.

### Example 39:

### 7-(5-Carboxypyridine-2-yl)-5-(3-chlorophenyl)-2-dimethylamino-8-methoxyquinazoline (Compound 39)

### Step 1:

Using Compound 5, 5-(3-Chlorophenyl)-2-dimethylamino-8-methoxy-7-(4,4,5,5-tetramethyl-1,3,2-dioxaborolan-2-yl)quinazoline (compound r) was prepared in the same manner as in Reference Example 12.

### Step 2:

Compound 39 was prepared in the same manner as in Example 11 using Compound r obtained in the step 1 and 6-chloronicotinic acid.
¹H NMR (DMSO-d₆, δ): 3.26 (s, 6H), 3.99 (s, 3H), 7.06 (d, J = 8.3 Hz, 1H), 7.13-7.15 (m, 1H), 7.27-7.28 (m, 1H), 7.36-7.46 (m, 3H), 7.98 (dd, *J* = 2.1, 8.3 Hz, 1H), 8.67 (s, 1H), 9.04 (d, *J* = 2.1 Hz, 1H).
APCIMS *m*/*z:* [M+H]⁺ 435.

### Example 40:

### 7-(6-Carboxypyridine-3-yl)-5-(3-chlorophenyl)-2-dimethylamino-8-methoxyquinazoline (Compound 40)

Compound 40 was prepared in the same manner as in Example 11 using Compound r and 5-bromo-2-carboxypyridine.
¹H NMR (DMSO-*d*₆, δ): 3.25 (s, 6H), 4.01 (s, 3H), 7.15-7.18 (m, 1H), 7.28-7.44 (m, 4H), 7.80 (dd, J = 2.3, 8.3 Hz, 1H), 7.94 (d, J = 8.3 Hz, 1H), 8.41 (d, J = 2.3 Hz, 1H), 8.64 (s, 1H).
APCIMS m/z: [M+H]⁺ 435.

### Example 41:

### 7-(5-Carboxyfuran-2-yl)-5-(3-chlorophenyl)-2-dimethylamino-8-methoxyquinazoline (Compound 41)

Compound 41 was prepared in the same manner as in Example 11 using Compound r and 5-bromo-2-furoic acid.
¹H NMR (DMSO-d₆, δ): 3.21 (s, 6H), 4.01 (s, 3H), 5.55 (d, *J* = 3.6 Hz, 1H), 7.06 (d, *J* = 3.6 Hz, 1H), 7.25-7.29 (m, 1H), 7.42 (s, 1H), 7.49 (s, 1H), 7.52-7.61 (m, 2H), 8.44 (s, 1H).
APCIMS m/z: [M+H]⁺ 424.

### Example 42:

### 7-(5-Carboxythiophene-2-yl)-5-(3-chlorophenyl)-2-dimethylamino-8-methoxyquinazoline (Compound 42)

Compound 43 was prepared in the same manner as in Example 11 using Compound r and 5-bromo-2-thiophenecarboxylic acid.
¹H NMR (DMSO-*d*₆, δ): 3.22 (s, 6H), 4.01 (s, 3H), 7.10 (d, J = 3.9 Hz, 1H), 7.23-7.26 (m, 1H), 7.35 (s, 1H), 7.36-7.38 (m, 1H), 7.46-7.55 (m, 2H), 7.54 (d, J = 3.9 Hz, 1H), 8.47 (s, 1H).
APCIMS *m*/*z*: [M+H]⁺ 440.

### Example 43:

### 5-(3-Chlorophenyl)-2-dimethylamino-8-methoxy-7-(1,2,5,6-tetrahydropyridine-4-yl)quinazoline (Compound 43)

### Step 1:

Using Compound r and 4-trifluoromethanesulfonyloxy-3,6-dihydro-2H-pyridine-1-carboxylic acid tert-butyl ester, 7-(1-tert-butoxycarbonyl-1,2,5,6-tetrahydropyridine-4-yl)-5-(3-chlorophenyl)-2-dimethylamino-8-methoxyquinazoline (compound s) was prepared in the same manner as in Example 11.
¹H NMR (CDCl₃, δ): 1.45 (s, 9H), 1.96 (brs, 2H), 3.32 (brs; 8H), 3.91 (brs, 2H), 4.04 (s, 3H), 5.58 (brs, 1H), 6.89 (s, 1H), 7.15-7.18 (m, 1H), 7.28-7.29 (m, 1H), 7.33-7.36 (m, 2H), 8.73 (s, 1H).
APCIMS *m*/*z:* [M+H]⁺ 495.

### Step 2:

Compound s (900 mg, 1.82 mmol) obtained in the step 1 was dissolved in dichloromethane (12 mL), and trifluoroacetic acid (6 mL) was added thereto under ice-cooling. After being stirred at room temperature for 10 minutes, the solvent was evaporated under reduced pressure, and saturated aqueous sodium hydrogencarbonate was added to the residue, and then the mixture was extracted with chloroform. The organic layer was washed with brine, and dried over anhydrous magnesium sulfate. The solvent was evaporated under reduced pressure, and the residue was recrystallized from hexane/ethyl acetate to give Compound 43 (603 mg, 84 %) as a pale yellow crystal.
¹H NMR (CDCl₃, δ): 1.92-1.94 (m, 2H), 2.79-2.83 (m, 2H), 3.32 (s, 6H), 3.36-3.39 (m, 2H), 4.04 (s, 3H), 5.62-5.63 (m, 1H), 6.93 (s, 1H), 7.17-7.21 (m, 1H), 7.31-7.32 (m, 1H), 7.35-7.47 (m, 2H), 8.74 (s, 1H).
APCIMS m/z: [M+H]⁺ 395.

### Example 44:

### 7-(1-Carboxymethyl-1,2,5,6-tetrahydropyridine-4-yl)-5-(3-chlorophenyl)-2-dimethylamino-8-methoxyquinazoline (Compound 44)

### Step 1:

Compound 43 (385 mg, 1.00 mmol) was dissolved in DMF (8 mL), and potassium carbonate (276 mg, 2.00 mmol) and ethyl bromoacetate (0.166 mL, 1.50 mmol) were added thereto at room temperature. After being stirred at room temperature for 2 hours, saturated aqueous ammonium chloride was added to the reaction mixture, and the mixture was extracted with ethyl acetate. The organic layer was washed with brine, and dried over anhydrous magnesium sulfate. The solvent was evaporated under reduced pressure, and the residue was purified by silica gel column chromatography (hexane/ethyl acetate = 1/3) to give 5-(3-chlorophenyl)-2-dimethylamino-7-(1-ethoxycarbonylmethyl-1,2,5,6-tetrahydropyridine-4-yl)-8-methoxyquinazoline (compound u) (328 mg, 68 %) as a pale yellow crystal.
¹H NMR (CDCl₃, δ): 1.27 (t, J = 7.1 Hz, 3H), 2.04 (brs, 2H), 2.52-2.56 (m, 2H), 3.16-3.19 (m, 1H), 3.25 (s, 2H), 3.32 (s, 6H), 4.02 (s, 3H), 4.18 (q, J = 7.1 Hz, 2H), 5.56-5.58 (m, 1H), 6.96 (s, 1H), 7.17-7.21 (m, 1H), 7.30-7.31 (m, 1H), 7.34-7.36 (m, 2H), 8.74 (s, 1H).
APCIMS *m*/*z*: [M+H]⁺ 481.

### Step 2:

Compound 44 was prepared in the same manner as in Example 27 using Compound u obtained in the step 1.
¹H NMR (DMSO-*d*₆, δ): 2.32 (brs, 4H), 3.22 (s, 6H), 3.72 (brs, 2H), 3.96 (brs, 2H), 3.97 (s, 3H), 5.55 (brs, 1H), 7.08 (s, 1H), 7.28-7.31 (m, 1H), 7.41 (s, 1H), 7.50-7.52 (m, 2H), 8.54 (s, 1H).
ESIMS m/z: [M+H]⁺ 453.

### Example 45:

### 7-(3-Carboxyphenyl)-5-(3-chlorophenyl)-8-methoxy-2-(methylamino)quinazoline (Compound 45)

Compound 45 was prepared in the same manner as in Example 11 using Compound 8 and 3-carboxyphenylboronic acid, followed by trituration with ethanol as a yellow solid.
¹H NMR (DMSO-d₆, δ): 2.92 (d, J = 4.3 Hz, 3H), 3.97 (s, 3H), 6.95-6.98 (m, 1H), 7.05-7.19 (m, 4H), 7.32-7.44 (m, 3H), 7.69 (d, J = 7.4 Hz, 1H), 7.78 (s, 1H), 8.53 (brs, 1H).
APCIMS m/z: [M+H]⁺ 420.
m.p.: 270°C

### Example 46:

### 7-(4-Carboxy-2-methoxyphenyl)-5-(3-chlorophenyl)-8-rriethoxy-2-(methylamino)quinazoline (Compound 46)

### Step 1:

Using Compound 8, 5-(3-chlorophenyl)-8-methoxy-2-methylamino-7-(4,4,5,5-tetramethyl-1,3,2-dioxaborolan-2-yl)quinazoline (compound bf) was prepared in the same manner as in Reference Example 12.
¹H NMR (CDCl₃, δ): 1.17 (s, 12H), 3.15 (brs, 3H), 4.08 (s, 3H), 5.53 (brs, 1H); 7.18-7.45 (m, 5H), 8.74 (brs, 1H).

### Step 2:

Compound 46 was prepared in the same manner as in Example 11 using Compound bf and methyl 3-methoxy-4-trifluoromethanesulfonyloxybenzoate (for example, it is obtained according to the method described in WO2003/048137).
¹H NMR (DMSO-d₆, δ): 2.92 (s, 3H), 3.57 (s, 3H), 3.92 (s, 3H), 7.05 (brs, 2H), 7.45-7.07 (m, 7H), 8.55 (s, 1H).
APCIMS m/z: [M+H]⁺ 450.

### Example 47:

### 7-(4-Carboxy-2-hydroxyphenyl)-5-(3-chlorophenyl)-8-methoxy-2-(methylamino)quinazoline (Compound 47)

Compound 47 was prepared in the same manner as in Example 11 using Compound bf and 4-bromo-3-hydroxybenzoic acid (for example, it is obtained according to the method described in WO99/16747).
¹H NMR (DMSO-d₆, δ): 2.91 (s, 3H), 3.92 (s, 3H), 6.94-7.07 (m, 2H), 7.09-7.16 (m, 1H), 7.18-7.24 (m, 2H), 7.26-7.38 (m, 3H), 7.39 (brs, 1H), 8.53 (s, 1H), 9.65 (s, 1H).
APCIMS m/z: [M+H]⁺ 436.

### Example 48:

### 7-(4-Carboxy-2-chlorophenyl)-5-(3-chlorophenyl)-8-methoxy-2-(methylamino)quinazoline (Compound 48)

Compound 48 was prepared in the same manner as in Example 11 using Compound bf and methyl 3-chloro-4-trifluoromethanesulfonyloxybenzoate (for example, it is obtained according to the method described in W02002/022113).
¹H NMR (DMSO-d₆, δ): 2.92 (s, 3H), 3.93 (s, 3H), 7.30-7.04 (m, 6H), 7.61 (m, 1H), 7.77 (s, 1H), 8.54 (s, 1H).
APCIMS m/z: [M-H]⁻ 453.

### Example 49:

### 7-(4-Carboxy-2-methylphenyl)-5-(3-chlorophenyl)-8-methoxy-2-(methylamino)quinazoline (Compound 49)

Compound 49 was prepared in the same manner as in Example 11 using Compound 8 and 4-carboxy-2-methylphenylboronic acid (for example, it is obtained according to the method described in WO97/34901).
¹H NMR (DMSO-d₆, δ): 2.08 (d, J = 9.2 Hz, 3H), 2.93 (s, 3H), 3.93 (s, 3H), 6.97-7.35 (m, 6H), 7.45 (brs, 1H), 7.55-7.65 (m, 1H), 7.69-7.75 (m, 1H), 8.53 (s, 1H), 12.84 (brs, 1H).
APCIMS m/z: [M+H]⁺ 434.

### Example 50:

### 7-(4-Carboxy-3,5-dimethylphenyl)-5-(3-chlorophenyl)-8-methoxy-2-(methylamino)quinazoline (Compound 50)

Compound 50 was prepared in the same manner as in Example 11 using Compound bf and 4-bromo-2,6-dimethylbenzoic acid (for example, it is obtained according to the method described in Journal of American Chemical Society, 1941, Vol. 63, p. 1679).
¹H NMR (DMSO-d₆, δ): 2.15 (s, 6H), 2.91 (d, J = 4.3.Hz, 3H), 3.97 (s, 3H), 6.88 (s, 2H), 7.42-7.13 (m, 5H), 8.49 (s, 1H).
APCIMS m/z: [M+H]⁺ 448.

### Example 51:

### 5-(3-Chlorophenyl)-8-methoxy-2-methylamino-7-phenylquinazoline (Compound 51)

Compound 51 was prepared in the same manner as in Example 11 using Compound 8 and phenylboronic acid.
¹H NMR (CDCl₃, δ): 3.17 (brs, 3H), 4.07 (s, 3H), 5.38 (br s, 1H), 6.99-7.28 (m, 10H), 8.79 (s, 1H).
APCIMS m/z: [M+H]⁺ 376.

### Example 52:

### 7-[(4-Carboxymethyl)phenyl)-5-(3-chlorophenyl)-8-methoxy-2-(methylamino)quinazoline (Compound 52)

### Step 1:

Compound 52 was prepared in the same manner as in Example 11 using Compound bf and 4-bromophenylacetic acid, followed by trituration with diisopropyl ether as a yellow solid.
¹H NMR (DMSO-d₆, δ): 2.92 (brs, 3H), 3.50 (s, 2H), 3.97 (s, 3H), 7.08-7.42 (m, 10H), 8.51 (s, 1H).
APCIMS m/z: [M+H]⁺ 434.
m.p.: 296-298°C

### Example 53:

### 5-(3-Chlorophenyl)-7-[4-(ethoxycarbonylmethyl)phenyl]-8-methoxy-2-(methylamino)quinazoline (Compound 53)

Compound 52 (183.9 mg, 0.426 mmol) was dissolved in anhydrous DMF (5 mL), and potassium carbonate (88.1 mg, 0.638 mmol) and iodoethane (0.051 mL, 0.638 mmol) were added thereto, and then the mixture was stirred overnight at room temperature. To the reaction mixture was added water, and the mixture was extracted with ethyl acetate. The organic layer was washed with water, and dried over anhydrous magnesium sulfate. The solvent was evaporated under reduced pressure, and the resulting solid was triturated with chloroform to give Compound 53 (122.2 mg, 62 %) as a yellow solid.
¹H NMR (CDCl₃, δ): 1.24 (t, J = 7.3 Hz, 3H), 3.16 (brs, 3H), 3.56 (s, 2H), 4.06 (s, 3H), 4.15 (q, J = 7.3 Hz, 2H), 5.38 (brs, 1H), 7.00-7.33 (m, 9H), 8.77 (s, 1H).
APCIMS m/z: [M+H]⁺ 462.

### Example 54:

### 7-[4(1-Carboxy-1-methylethyl)phenyl]-5-(3-chlorophenyl)-8-methoxy-2-(methylamino)quinazoline (Compound 54)

Compound 54 was prepared in the same manner as in Example 11 using Compound bf and 2-(4-bromophenyl)-2-methylpropionic acid (for example, it is obtained according to the method described in Journal of Organic Chemistry, 1994, Vol. 59, p. 2620).
¹H NMR (DMSO-d₆, δ): 1.39 (s, 6H), 2.91 (brs, 3H), 3.97 (s, 3H), 7.09-7.23 (m, 7H), 7.35-7.39 (m, 3H), 8.50 (s, 1H).
APCIMS m/z: [M+H]⁺ 462.

### Example 55:

### 7-[4(2-Carboxyethyl)phenyl]-5-(3-chlorophenyl)-8-methoxy-2-(methylamino)quinazoline (Compound 55)

Compound bf and 3-(4-trifluoromethanesulfonyloxyphenyl)propionic acid [for example, it is obtained according to the method described in Protective Groups in Organic Synthesis, third edition, T. W. Greene, John Wiley & Sons Inc. (1999)] was reacted in the same manner as in Example 11, and the resulting solid was triturated with diisopropyl ether to give Compound 55 as a yellow solid.
¹H NMR (DMSO-d₆, δ): 2.46 (t, J =. 7.7 Hz, 2H), 2.75 (t, J = 7.7 Hz, 2H), 2.90 (brs, 3H), 3.96 (s, 3H), 7.04-7.13 (m, 6H), 7.22 (s, 1H), 7.34-7.38 (m, 3H), 8.50 (s, 1H).
APCIMS m/z: [M+H]⁺ 448.
m.p.: 208-213°C

### Example 56:

### 7-[4-(Carboxymethoxy)phenyl]-5-(3-chlorophenyl)-8-methoxy-2-(methylamino)quinazoline (Compound 56)

Compound 56 was prepared in the same manner as in Example 27 using Compound 57.
¹H NMR (DMSO-d₆, δ): 2.90 (d, J = 4.6 Hz, 3H), 3.96 (s, 3H), 4.60 (s, 2H), 6.76 (d, J = 8.6 Hz, 2H), 7.06 (d, J = 8.6 Hz, 2H), 7.12-7.14 (m, 2H), 7.23-7.39 (m, 4H), 8.49 (brs, 1H).
APCIMS m/z: [M+H]⁺ 450.

### Example 57:

### 5-(3-Chlorophenyl)-7-[4-(ethoxycarbonylmethoxy)phenyl]-8-methoxy-2-(methylamino)quinazoline (Compound 57)

Compound 60 (160 mg, 0.410 mmol) was dissolved in DMF (10 mL), and ethyl bromoacetate (54 mL, 0.49 mmol) and potassium carbonate (85 mg, 0.61 mmol) were added thereto, and then the mixture was stirred at room temperature for 5 hours. After water was added to the reaction mixture, the mixture was extracted with ethyl acetate, and the organic layer was washed with water and brine, then dried over anhydrous sodium sulfate. The solvent was evaporated under reduced pressure, and the resulting residue was triturated with ethanol to give Compound 57 (150 mg, 77 %) as a pale yellow crystal.
¹H NMR (CDCl₃, δ): 1.28 (t, J = 7.1 Hz, 3H), 3.15 (d, J = 5.0 Hz, 3H), 4.06 (s, 3H), 4.26 (q, J = 7.1 Hz, 2H), 4.58 (s, 2H), 5.36 (brs, 1H), 6.75-6.78 (m, 2H), 6.98-7.08 (m, 4H), 7.17-7.27 (m, 3H), 8.76 (s, 1H).
APCIMS m/z: [M+H]⁺ 478.

### Example 58:

### 7-[4-(1-Carboxy-1-methylethoxy)phenyl]-5-(3-chlorophenyl)-8-methoxy-2-(methylamino)quinazoline (Compound 58)

Compound 58 was prepared in the same manner as in Example 11 using Compound bf and 2-(4-bromophenoxy)-2-methyl propanoic acid (for example, it is obtained according to the method described in Canadian Journal of Chemistry, 1989, Vol. 67, p. 1472).
¹H NMR (DMSO-d₆, δ): 1.46 (s, 6H), 2.91 (d, J = 4.8 Hz, 3H), 3.97 (s, 3H), 6.68 (d, J = 8.8 Hz, 2H), 7.03 (d, J = 8.6 Hz, 2H), 7.13-7.18 (m, 3H), 7.33-7.38 (m, 3H), 8.53 (brs, 1H).
APCIMS m/z: [M+H]⁺ 478.

### Example 59:

### 7-[3-(Carboxymethyl)phenyl]-5-(3-chlorophenyl)-8-methoxy-2-(methylamino)quinazoline (Compound 59)

Compound bf and 3-bromophenylacetic acid was reacted in the same manner as in Example 11, and the resulting solid was triturated with diisopropyl ether to give Compound 59 as a yellow solid.
¹H NMR (DMSO-d₆, δ): 2.91 (brs, 3H), 3.43 (s, 2H), 3.97 (s, 3H), 6.98-7.40 (m, 10H), 8.51 (s, 1H).
APCIMS m/z: [M+H]⁺ 434.
m.p. : 258-261°C

### Example 60:

### 5-(3-Chlorophenyl)-7-(4-hydroxyphenyl)-8-methoxy-2-(methylamino)quinazoline (Compound 60)

Compound 60 was prepared in the same manner as in Example 11 using Compound 8 and 4-(4,4,5,5-tetramethyl-1,3,2-dioxaborolan-2-yl)phenol.
¹H NMR (DMSO-d₆, δ): 2.91 (d, J = 4.6 Hz, 3H), 3.96 (s, 3H), 6.61 (d, J = 8.4 Hz, 2H), 6.94 (d, J = 8.4 Hz, 2H), 7.11-7.19 (m, 3H), 7.35-7.37 (m, 3H), 8.51 (brs, 1H), 9.36 (brs, 1H).
APCIMS m/z: [M+H]⁺ 422.

### Example 61:

### 5-(3-Chlorophenyl)-7-(3-hydroxyphenyl)-8-methoxy-2-(methylamino)quinazoline (Compound 61)

Compound 61 was prepared in the same manner as in Example 11 using Compound 8 and 3-(4,4,5,5-tetramethyl-1,3,2-dioxaborolan-2-yl)phenol.
¹H NMR (DMSO-d₆, δ): 2.91 (d, J = 4.8 Hz, 3H), 3.96 (s, 3H), 6.52-6.60 (m, 3H), 6.97-7.03 (m, 1H), 7.11-7.22 (m, 3H), 7.33-7.42 (m, 3H), 8.52 (brs, 1H), 9.33 (brs, 1H).
APCIMS m/z: [M+H]⁺ 392.

### Example 62:

### 5-(3-Chlorophenyl)-7-(2-hydroxyphenyl)-8-methoxy-2-(methylamino)quinazoline (Compound 62)

Compound 62 was prepared in the same manner as in Example 11 using Compound 8 and 2-(4,4,5,5-tetramethyl-1,3,2-dioxaborolan-2-yl)phenol.
¹H NMR (DMSO-d₆, δ): 2.92 (d, J = 4.0 Hz, 3H), 3.91 (s, 3H), 6.62 (t, J = 7.3 Hz, 1H), 6.74-6.87 (m, 2H), 6.99-7.39 (m, 7H), 8.52 (brs, 1H), 9.25 (brs, 1H).
APCIMS m/z: [M+H]⁺ 392.

### Example 63:

### 5-(3-Chlorophenyl)-7-(4-hydroxy-3-methoxyphenyl)-8-methoxy-2-(methylamino)quinazoline (Compound 63)

Compound 63 was prepared in the same manner as in Example 11 using Compound 8 and 2-methoxy-4-(4,4,5,5-tetramethyl-1,3,2-dioxaborolan-2-yl)phenol.
¹H NMR (DMSO-d₆, δ): 2.91 (d, J = 4.6 Hz, 3H), 3.50 (s, 3H), 3.98 (s, 3H), 6.57 (brs, 1H), 6.66 (brs, 2H), 7.13-7.17 (m, 2H), 7.26 (m, 1H), 7.34-7.39 (m, 3H), 8.53 (brs, 1H), 8.94 (brs, 1H).
APCIMS m/z: [M+H]⁺ 422.

### Example 64:

### 5-(3-Chlorophenyl)-7-(4-hydroxy-3,5-dimethylphenyl)-8-methoxy-2-(methylamino)quinazoline (Compound 64)

Compound 64 was prepared in the same manner as in Example 11 using Compound 8 and 2,6-dimethyl-4-(4,4,5,5-tetramethyl-1,3,2-dioxaborolan-2-yl)phenol.
¹H NMR (DMSO-d₆, δ):2.03 (s, 6H), 2.90 (d, J = 4.7 Hz, 3H), 3.96 (s, 3H), 6.70 (s, 2H), 7.10-7.14 (m, 2H), 7.22 (s, 1H), 7.33-7.38 (m, 3H), 8.17 (brs, 1H), 8.49 (brs, 1H).
APCIMS m/z: [M+H]⁺ 420.

### Example 65:

### 5-(3-Chlorophenyl)-7-[4-(hydroxymethyl)phenyl]-8-methoxy-2-(methylamino)quinazoline (Compound 65)

Compound 65 was prepared in the same manner as in Example 11 using Compound bf and 4-bromophenyl methanol.
¹H NMR (DMSO-d₆, δ): 2.92 (d, J = 4.3 Hz, 3H), 3.97 (s, 3H), 4.43 (s, 2H), 7.43-7.10 (m, 10H), 8.51 (s, 1H).
APCIMS m/z: [M+H]⁺ 406.

### Example 66:

### 5-(3-Chlorophenyl)-7-[4-(2-hydroxyethyl)phenyl]-8-methoxy-2-(methylamino)quinazoline (Compound 66)

Compound 66 was prepared in the same manner as in Example 11 using Compound bf and 4-bromophenyl ethanol.
¹H NMR (DMSO-d₆, δ): 2.67 (t, J = 7.0 Hz, 2H), 2.91 (d, J = 4.6 Hz, 3H), 3.55 (t, J = 7.0 Hz, 2H), 3.97 (s, 3H), 7.04-7.43 (m, 11H), 8.50 (s, 1H).
APCIMS m/z: [M+H]⁺ 420.

### Example 67:

### 5-(3-Chlorophenyl)-8-methoxy-2-methylamino-7-(4-sulfophenyl)quinazoline (Compound 67)

Compound 67 was prepared in the same manner as in Example 11 using Compound bf and 4-bromobenzenesulfonic acid monohydrate.
¹H NMR (DMSO-d₆, δ): 3.06 (brs, 3H), 4.08 (s, 3H), 7.06-7.18 (m, 3H), 7.26-7.48 (m, 7H), 8.88 (brs, 1H).
APCIMS m/z: [M+H]⁺ 456.

### Example 68:

### 5-(3-Chlorophenyl)-8-methoxy-2-methylamino-7-(4-sulfamoylphenyl)quinazoline (Compound 68)

Compound 68 was prepared in the same manner as in Example 11 using Compound bf and 4-bromobenzene sulfonamide.
¹H NMR (DMSO-d₆, δ): 2.91 (d, J = 4.6 Hz, 3H), 3.97 (s, 3H), 7.13-7.16 (m, 2H), 7.29-7.48 (m, 8H), 7.66 (d, J = 8.2 Hz, 2H), 8.51 (brs, 1H).
APCIMS m/z: [M+H]⁺ 455.

### Example 69:

### 5-(3-Chlorophenyl)-8-methoxy-2-methylamino-7-(4-methylthiopheneyl)quinazoline (Compound 69)

Compound 69 was prepared in the same manner as in Example 11 using Compound 8 and 4-(methylthio)phenylboronic acid.
¹H NMR (CDCl₃, δ): 2.46 (s, 3H), 3.16 (d, J = 5.1 Hz, 3H), 4.06 (s, 3H), 5.38 (brs, 1H), 6.99-7.13 (m, 6H), 7.18-7.29 (m, 3H), 8.76 (s, 1H).
APCIMS m/z: [M+H]⁺ 422.

### Example 70:

### 5-(3-Chlorophenyl)-7-(4-methanesulfinylphenyl)-8-methoxy-2-(methylamino)quinazoline (Compound 70)

Compound 69 (200 mg, 0.470 mmol) was dissolved in methylene chloride (15 mL), and cooled to -10°C under a nitrogen atmosphere. To the solution was added m-chloroperbenzoic acid (130 mg, 0.520 mmol), and the mixture was stirred at -10°C for 30 minutes. To the reaction mixture was added aqueous sodium sulfite, and the mixture was warmed to room temperature. The reaction mixture was extracted with chloroform, and then the organic layer was washed with saturated aqueous sodium hydrogencarbonate and brine, and dried over anhydrous magnesium sulfate. The solvent was evaporated under reduced pressure, and the residue was purified by silica gel column chromatography (n-hexane/ethyl acetate = 3/1 to 0/1), and then triturated with ethanol to give Compound 70 (110 mg, 53 %) as a pale yellow crystal.
¹H NMR (CDCl₃, δ): 2.71 (s, 3H), 3.17 (d, J = 5.0 Hz, 3H), 4.08 (s, 3H), 5.46 (brs, 1H), 7.01 (d, J = 7.3 Hz, 1H), 7.08 (s, 1H), 7.13-7.15 (m, 1H), 7.19-7.31 (m, 4H), 7.51 (d, J = 8.1 Hz, 2H), 8.79 (brs, 1H).
APCIMS m/z: [M+H]⁺ 438.
m.p.: 197°C

### Example 71:

### 5-(3-Chlorophenyl)-7-(4-methanesulfonylphenyl)-8-methoxy-2-(methylamino)quinazoline (Compound 71)

Compound 69 (100 mg, 0.240 mmol) was dissolved in methylene chloride (8 mL), and cooled to -10°C under a nitrogen atmosphere. To the solution was added m-chloroperbenzoic acid (150 mg, 0.607 mmol), and the mixture was stirred at -10°C for 1.5 hours. To the reaction mixture was added aqueous sodium sulfite, and the mixture was warmed to room temperature. The reaction mixture was extracted with chloroform, and then the organic layer was washed with saturated aqueous sodium hydrogencarbonate and brine, and dried over anhydrous magnesium sulfate. The solvent was evaporated under reduced pressure, and the residue was purified by silica gel column chromatography (n-hexane/ethyl acetate = 3/1 to 0/1), and then triturated with ethanol to give Compound 71 (14 mg, 13 %) as a pale yellow crystal.
¹H NMR (CDCl₃, δ): 3.04 (s, 3H), 3.17 (d, J = 4.8 Hz, 3H), 4.08 (s, 3H), 5.61 (brs, 1H), 6.99-7.28 (m, 5H), 7.34 (d, J = 8.4 Hz, 2H), 7.80 (d, J = 8.4 Hz, 2H), 8.78 (brs, 1H).
APCIMS m/z: [M+H]⁺ 454.

### Example 72:

### 5-(3-Chlorophenyl)-8-methoxy-2-methylamino-7-[4-(1H-tetrazole-5-yl)phenyl]quinazoline (Compound 72)

Compound 72 was prepared in the same manner as in Example 11 using Compound bf and 5-(4-bromophenyl)-1H-tetrazole.
¹H NMR (DMSO-d₆, δ): 2.92 (s, 3H), 4.00 (s, 3H), 7.39 (d, J = 8.2 Hz, 2H), 7.50-7.17 (m, 5H), 7.89 (d, J = 8.2 Hz, 2H), 8.56 (s, 1H).
APCIMS m/z: [M+H]⁺ 444.

### Example 73:

### 5-(3-Chlorophenyl)-8-methoxy-2-methylamino-7-(2-oxo-2,3-dihydrobenzoxazole-6-yl)quinazoline (Compound 73)

Compound 73 was prepared in the same manner as in Example 11 using Compound bf and 6-bromo-3H-benzoxazole-2-one (for example, it is obtained according to the method described in WO2002/050070).
¹H NMR (DMSO-d₆, δ): 2.91 (d, J = 4.2 Hz, 3H), 3.97 (s, 3H), 6.91-6.95 (m, 2H), 7.12-7.16 (m, 3H), 7.27 (s, 1H), 7.36-7.38 (m, 3H), 8.52 (brs, 1H).
APCIMS m/z: [M+H]⁺ 433.

### Example 74:

### 5-(3-Chlorophenyl)-8-methoxy-2-methylamino-7-(2-oxo-2,3-dihydrobenzothiazole-6-yl)quinazoline (Compound 74)

Compound 74 was prepared in the same manner as in Example 11 using Compound bf and 6-bromo-3H-benzothiazole-2-one.
¹H NMR (DMSO-d₆, δ): 2.91 (d, J = 4.4 Hz, 3H), 3.97 (s, 3H), 6.90-6.98 (m, 2H), 7.14-7.17 (m, 2H), 7.26 (s, 1H), 7.33-8.32 (m, 4H), 8.51 (brs, 1H).
APCIMS m/z: [M+H]⁺ 449.

### Example 75:

### 7-(5-Carboxypyridine-2-yl)-5-(3-chlorophenyl)-8-methoxy-2-(methylamino)quinazoline (Compound 75)

Compound 75 was prepared in the same manner as in Example 11 using Compound bf and 6-chloronico.tinic acid.
¹H NMR (DMSO-d₆, δ): 2.93 (brs, 3H), 3.99 (s, 3H), 7.06 (d, J = 9.0 Hz, 1H), 7.15 (d, J = 7.8 Hz, 1H), 7.28-7.44 (m, 4H), 7.59 (brs, 1H), 7.98 (dd, J = 2.4, 9.0 Hz, 1H), 8.59 (s, 1H), 9.03 (d, J = 2.4 Hz, 1H).
APCIMS m/z: [M+H]⁺ 421.

### Example 76:

### 7-(5-Carboxythiophene-2-yl)-5-(3-chlorophenyl)-8-methoxy-2-(methylamino)quinazoline (Compound 76)

Compound 76 was prepared in the same manner as in Example 11 using Compound bf and 2-bromothiophene-5-carboxylic acid.
¹H NMR (DMSO-d₆, δ): 2.91 (s, 3H), 4.01 (s, 3H)., 7.09 (d, J = 3.5 Hz, 1H), 7.26 (d, J = 7.1 Hz, 1H), 7.60-7.35 (m, 5H), 8.42 (s, 1H).
APCIMS m/z: [M+H]⁺ 436.

### Example 77:

### 7-(5-Carboxy-1-methylpyrrole-2-yl)-5-(3-chlorophenyl)-2-methylamino-8-methoxyquinazoline (Compound 77)

### Step 1:

5-Bromo-1-methylpyrrole-2-carboxylic acid was prepared in the same manner as in Example 27 using methyl 5-bromo-1-methylpyrrole-2-carboxylate (for example, it is prepared according to the method described in WO2003/040147 and the like).
¹H NMR (CDCl₃, δ): 3.93 (s, 3H), 6.25 (d, J = 4.1 Hz, 1H), 7.08 (d, J = 4.1 Hz, 1H).

### Step 2:

Compound 77 was prepared in the same manner as in Example 11 using compound bf and 5-bromo-1-methylpyrrole-2-carboxylic acid obtained in the step 1.
¹H NMR (DMSO-d₆, δ): 2.92 (d, J = 4.6 Hz, 3H), 3.52 (s, 3H), 3.95 (s, 3H), 5.73-5.76 (m, 1H), 6.45-6.53 (m, 1H), 7.07 (s, 1H), 7.11-7.17 (m, 1H), 7.21-7.26 (m, 1H), 7.33-7.39 (m, 2H), 8.56 (s, 1H).
APCIMS m/z: [M+H]⁺ 423.

### Example 78:

### 7-(5-Carboxymethylthiophene-2-yl)-5-(3-chlorophenyl)-8-methoxy-2-(methylamino)quinazoline (Compound 78)

Compound 78 was prepared in the same manner as in Example 11 using Compound bf and (5-bromothiophene-2-yl)acetate (for example, it is prepared according to the method described in Journal of Chemical Society Perkin Transaction, 1990, Vol. 1, p. 2911).
¹H NMR (DMSO-d₆, δ): 2.90 (s, 3H), 3.69 (s, 2H), 4.00 (s, 3H), 6.76 (d, J = 3.7 Hz, 1H) , 6.80 (d, J = 3.7 Hz, 1H) , 7.54-7.19 (m, 5H), 8.38 (s, 1H).
APCIMS m/z: [M+H]⁺ 440.

### Example 79:

### 7-[5-(Carboxymethyl)thiophene-3-yl]-5-(3-chlorophenyl)-8-methoxy-2-(methylamino)quinazoline (Compound 79)

Compound 79 was prepared in the same manner as in Example 11 using Compound bf and (4-bromothiophene-2-yl)acetic acid (for example, it is prepared according to the method described in WO99/16763).
¹H NMR (DMSO-d₆, δ): 2.89 (s, 3H), 3.66 (s, 2H), 3.97 (s, 3H), 6.68 (s, 1H), 6.98 (s, 1H), 7.44-7.16 (m, 5H), 8.45 (s 1H).
APCIMS m/z: [M+H]⁺ 440.

### Example 80:

### 7-[5-(Carboxymethyl)furan-2-yl]-5-(3-chlorophenyl)-8-methoxy-2-(methylamino)quinazoline (Compound 80)

### Step 1:

Ethyl furan-2-ylacetate (530 mg, 3.42 mmol) was dissolved in DMF (20 mL), and cooled to 0°C. To the solution was added bromine (260 mL, 5.14 mmol), and the mixture was stirred at room temperature for 17 hours. To the reaction mixture were added water and ethyl acetate, and the mixture was extracted. The organic layer was washed with water and brine, and dried over anhydrous magnesium sulfate. The solvent was evaporated under reduced pressure, and the residue was purified by silica gel column chromatography (n-hexane/ethyl acetate = 1/0 to 9/1) to give ethyl 5-bromofuran-2-ylacetate (90 mg, 11 %) as yellow oil.
¹H NMR (CDCl₃, δ): 1.28 (t, J = 7.2 Hz, 3H), 3.65 (s, 2H), 4.18 (q, J = 7.2 Hz, 2H), 6.21-6.25 (m, 2H).

### Step 2:

Using ethyl 5-bromofuran-2-ylacetate obtained in the step 1, 5-bromofuran-2-ylacetic acid was prepared in the same manner as in Example 27.
¹H NMR (CDCl₃, δ): 3.33 (s, 2H), 5.92 (d, J = 3.5 Hz, 1H), 6.16 (d, J = 3.5 Hz, 1H).

### Step 3:

Compound 80 was prepared in the same manner as in Example 11 using Compound bf and 5-bromofuran-2-ylacetic acid obtained in the step 2.
¹H NMR (DMSO-d₆, δ): 2.90 (d, J = 4.2 Hz, 3H), 3.61 (s, 2H), 4.00 (s, 3H), 5.42 (d, J = 3.3 Hz, 1H), 6.15 (d, J = 3.3 Hz, 1H), 7.24-7.26 (m, 1H), 7.36 (s, 1H), 7.48-7.61 (m, 4H), 8.36 (brs, 1H).
APCIMS m/z: [M+H]⁺ 424.

### Example 81:

### 5-(3-Chlorophenyl)-8-methoxy-2-methylamino-7-vinylquinazoline (Compound 81)

Compound 8 (600 mg, 1.58 mmol) was suspended in anhydrous THF (10 mL), and tributylvinyltin (0.555 mL, 1.90 mL), and tetrakis(triphenylphosphine)palladium (92.1 mg, 0.0797 mmol) were added thereto, then the mixture was stirred at 100°C for 5.5 hours under an argon atmosphere. The reaction mixture was cooled to room temperature, and saturated aqueous ammonium fluoride was added thereto, then the mixture was filtered through Celite. The filtrate was extracted with ethyl acetate. The organic layer was washed with brine, and dried over anhydrous magnesium sulfate. The solvent was evaporated under reduced pressure, and the residue was triturated with ethanol to give Compound 81 (361 mg, 70 %) as a yellow solid.
¹H NMR (CDCl₃, δ): 3.14 (brs, 3H) , 4.10 (s, 3H) , 5.21 (dd, J = 0.8, 11 Hz, 1H), 5.37 (brs, 1H), 5.69 (dd, J = 0.8, 17 Hz, 1H), 6.53 (dd, J = 11, 17 Hz, 1H), 7.14-7.18 (m, 1H), 7.25-7.31 (m, 2H), 7.38-7.45 (m, 2H), 8.58 (s, 1H).

### Example 82:

### 5-(3-Chlorophenyl)-7-formyl-8-methoxy-2-(methylamino)quinazoline (Compound 82)

Compound 81 (296 mg, 0.923 mmol) was dissolved in THF (30 mL) and water (15 mL), and a 2.5 wt.% solution of osmium tetroxide in 2-propanol (0.93 mL, 0.0742 mmol) was added thereto, and then sodium periodate (396 mg, 1.85 mmol) was added thereto slowly. The mixture was stirred at room temperature for 4 hours. To the reaction mixture was added water, and the mixture was extracted with chloroform. The organic layer was washed with brine, and dried over anhydrous magnesium sulfate. The solvent was evaporated under reduced pressure, and triturated with ethanol to give Compound 82 (211 mg, 70 %) as a yellow solid.
¹H NMR (CDCl₃, δ): 3.17 (brs, 3H), 4.12 (s, 3H), 5.67 (brs, 1H), 7.26-7.61 (m, 5H), 8.74 (brs, 1H), 9.76 (s, 1H).

### Example 83:

### 7-(2-Ethoxycarbonyl-1-hydroxy-2-methylpropyl)-5-(3-chlorophenyl)-8-methoxy-2-(methylamino)quinazoline

### (Compound 83)

To a solution of diisopropylamine (0.103 mL, 0.735 mmol) in THF (5 mL) was added a 1.58 mol/L solution of n-butyllithium in n-hexane (0.464 mL, 0.735 mmol) at -78°C under a nitrogen atmosphere, and the mixture was stirred at the same temperature for 10 minutes. Next, a solution of ethyl isobutyrate (0.098 mL, 0.735 mmol) in THF (1 mL) was dropped slowly thereto, and the mixture was further stirred at the same temperature for 30 minutes. Next, a suspension of Compound 82 (20 mg, 0.061 mmol) in THF (1 mL) was dropped slowly thereto, and the mixture was stirred at the same temperature for 1 hour. Then to the mixture was added saturated aqueous ammonium chloride, and the mixture was extracted with ethyl acetate. The organic layer was washed with brine, and dried over anhydrous magnesium sulfate. The solvent was evaporated under reduced pressure, and the residue was purified by preparative thin-layer chromatography (hexane/ethyl acetate = 1/1) to give Compound 83 (21.5 mg, 80 %) as pale yellow oil.
¹H NMR (CDCl₃, δ): 0.97 (d, J = 6.4 Hz, 3H), 1.12 (s, 3H), 1.18-1.26 (m, 3H), 3.09-3.16 (m, 3H), 3.93-4.06 (m, 4H), 4.08-4.20 (m, 2H), 4.86-4.95 (m, 1H), 5.37 (brs, 1H), 7.10-7.15 (m, 0.5H), 7.20-7.30, (m, 2H), 7.32-7.36 (m, 0.5H), 7.36-7.45 (m, 2H), 8.48 (s, 0.5H), 8.52 (s, 0.5H).
APCIMS m/z: [M+H]⁺ 444.

### Example 84:

### 7-(2-Carboxy-1-hydroxy-2-methylpropyl)-5-(3-chlorophenyl)-8-methoxy-2-(methylamino)quinazoline (Compound 84)

Compound 84 was prepared in the same manner as in Example 27 using Compound 83.
¹H NMR (CDCl₃, δ): 0.78 (brs, 3H), 1.30 (brs, 3H), 3.07-3.14 (3H, m), 4.40-4.55 (brs, 1H), 7.12-7.19 (m, 0.5H), 7.25-7.52 (m, 4.5H), 8.63-8.75 (m, 1H).
APCIMS m/z: [M+H]⁺ 415.

### Example 85:

### 7-[E-(2-ethoxycarbonyl-1-propenyl)]-5-(3-chlorophenyl)-8-methoxy-2-(methylamino)quinazoline (Compound 85)

Sodium hydride (0.146 g, 3.66 mmol) was suspended in THF (6 mL), and triethyl 2-phosphonopropionate (0.785 mL, 3.66 mmol) was dropped thereto under ice cooling, and then the mixture was stirred at room temperature for 1 hour. Next, to the mixture was added a suspension of Compound 82 (0.500 g, 1.53 mmol) in THF (4 ml) slowly under ice-cooling, and the mixture was stirred at room temperature for 2 hours. Next, saturated aqueous ammonium chloride was added to the reaction mixture, and the mixture was extracted with ethyl acetate. The organic layer was washed with brine, and dried over anhydrous magnesium sulfate. The solvent was evaporated under reduced pressure, and the residue was purified by silica gel column chromatography (ethyl acetate/hexane = 63/35) to give Compound 85 (0.503 g, 80 %).
¹H NMR (CDCl₃, δ):1.24 (t, J = 7.2 Hz, 3H), 2.05 (d, J = 1.7 Hz, 3H), 3.15 (d, J = 5.1 Hz, 3H), 4.06 (s, 3H), 4.13-4.22 (m, 2H), 5.42 (brs, 1H), 7.07 (s, 1H), 7.11-7.17 (m, 1H), 7.35-7.44 (m, 4H), 8.73 (brs, 1H).

### Example 86:

### 7-[E-(2-carboxy-1-propenyl)]-5-(3-chlorophenyl)-8-methoxy-2-(methylamino)quinazoline (Compound 86)

Compound 86 was prepared in the same manner as in Example 27 using Compound 85.
¹H NMR (DMSO-d₆, δ): 1.89 (d, J = 0.9 Hz, 3H), 2.89 (d, J = 4.6 Hz, 3H), 3.94 (s, 3H), 6.91-6.96 (m, 1H), 7.10-7.52 (m, 6H), 8.38-8.45 (m, 1H).
APCIMS m/z: [M+H]⁺ 384.

### Example 87:

### 7-(2-Carboxypropyl)-8-methoxy-2-methylamino-5-phenylquinazoline (Compound 87)

### Step 1:

Using Compound 85, 7-(2-Ethoxycarbonylpropyl)-8-methoxy-2-methylamino-5-phenylquinazoline (Compound bg) was prepared by reduction in the same manner as in Reference Example 35.
APCIMS m/z: [M+H]⁺ 380.

### Step 2:

Compound 87 was prepared by hydrolysis in the same manner as in Example 27 using the compound bg obtained in the step 1.
¹H NMR (CDCl₃, δ): 1.01 (d, J = 6.8 Hz, 3H), 2.55-2.70 (m, 2H), 2.85-2.97 (m, 1H), 2.97-3.15 (m, 3H), 3.94 (s, 3H), 5.88 (brs, 1H), 6.96 (s, 1H), 7.20-7.29 (m, 2H), 7.39-7.50 (m, 3H), 8.49 (s, 1H).
APCIMS m/z: [M+H]⁴ 352.

### Example 88:

### 7-(2-Carboxy-2-methylpropyl)-5-(3-chlorophenyl)-8-methoxy-2-(methylamino)quinazoline (Compound 88)

### Step 1:

Compound 83 (0.312 g, 0.700 mmol) was dissolved in trifluoroacetic acid (3 mL), and triethylsilane (1.12 mL, 7.03 mmol) was added thereto under ice-cooling, and then the mixture was stirred at room temperature for 60 hours. After the mixture was neutralized by addition of saturated aqueous sodium hydrogencarbonate, the mixture was extracted with ethyl acetate, and then the resulting organic layer was washed with brine, and dried over anhydrous magnesium sulfate. The solvent was evaporated under reduced pressure, and the resulting residue was purified by silica gel column chromatography (chloroform/methanol = 90/10 to 75/25) to give 3,4-dihydro-5-(3-chlorophenyl)-7-(2-ethoxycarbonyl-2-methylpropyl)-8-methoxy-2-(methylamino)quinazoline (Compound bh) (0.21 g, 70 %).
¹H NMR (CDCl₃, δ): 0.95 (s, 3H), 0.97 (s, 3H), 1.24 (t, J = 7.1 Hz, 3H), 2.68 (d, J = 14.1 Hz, 1H), 2.75 (d, J = 14.1 Hz, 1H), 2.93 (brs, 3H), 4.05-4.17 (m, 4H), 6.69 (s, 1H), 6.90-6.98 (m, 1H), 7.02-7.08 (m, 1H), 7.29-7.38 (m, 2H).

### Step 2:

By treating with manganese dioxide in the same manner as in the step 2 in Example 100, 5-(3-chlorophenyl)-7-(2-ethoxycarbonyl-2-methylpropyl)-8-methoxy-2-(methylamino)quinazoline (Compound bi) was prepared using the compound bh obtained in the step 1.
¹H NMR (CDCl₃, δ): 0.98 (s, 3H), 1.00 (s, 3H), 1.24 (t, J = 7.1 Hz, 3H), 2.86 (d, J = 13.9 Hz, 1H), 2.94 (d, J = 13.9 Hz, 1H), 3.10-3.20 (m, 3H), 4.02 (s, 3H), 4.13 (q, J = 7.1 Hz, 2H), 7.06-7.19 (m, 2H), 7.24-7.28 (m, 1H), 7.40-7.50 (m, 2H), 8.66 (brs, 1H).
APCIMS m/z: [M+H]⁺ 428.

### Step 3:

Compound 88 was prepared in the same manner as in Example 27 using Compound bi obtained in the step 2.
¹H NMR (DMSO-d₆, δ): 0.74 (s, 6H), 2.66 (s, 2H), 2.85 (d, J = 4.8 Hz, 3H), 3.85 (s, 3H), 7.13-7.29 (m, 3H), 7.45-7.56 (m, 3H), 8.23 (s, 1H).
APCIMS m/z: [M+H]⁺ 400.

### Example 89:

### 5-(3-Chlorophenyl)-7-hydroxy-8,-methoxy-2-(methylamino)quinazoline (Compound 89)

Compound bf (0.692 mg, 1.63 mmol), 0.5 mol/L aqueous sodium hydroxide (9.6 mL) and sodium hydrogencarbonate (1.11 g, 13.2 mmol) were added to acetone (34 mL), and the mixture was stirred at room temperature for 1.5 hours. After ice-cooling, to the mixture was added Oxone (1.81 g, 2.93 mmol), and the mixture was stirred for 15 minutes under ice-cooling. After saturated aqueous sodium hydrogensulfite was added to the reaction mixture to quench the reaction, sodium hydrogencarbonate was added thereto to adjust pH of the solution to 7.6. Then, water was added thereto, and the precipitated solid was collected by filtration. To the resulting solid was added 1 mol/L aqueous sodium hydroxide, and the mixture was stirred, and then filtered. To the resulting filtrate was added aqueous hydrochloric acid to adjust the pH to 7, and the precipitated solid was collected by filtration. The solid was further triturated (with diisopropyl ether) to give Compound 89 (0.298 g, 58 %) as a yellow solid.
¹H NMR (DMSO-d₆, δ): 3.11 (d, J = 5.1Hz, 3H), 4.04 (s, 3H), 5.22 (brs, 1H), 6.84 (s, 1H), 7.29-7.53 (m, 4H), 8.62 (s, 1H).
APCIMS m/z: [M+H]⁺ 316.
Example 90:

### 5-(3-Chlorophenyl)-7-ethoxycarbonylmethoxy-8-methoxy-2-(methylamino)quinazoline (Compound 90)

To Compound 89 (0.25 g, 0.79 mmol) were added DMF (3 mL), potassium carbonate (0.12 g, 87 mmol) and ethyl bromoacetate (0.15 g, 0.87 mmol), and the mixture was stirred at room temperature for 8 hours under an argon atmosphere. Water was added to the reaction mixture, and the precipitated solid was collected by filtration to give Compound 90 (0.29 g, 90 %) as a yellow solid.
¹H NMR (CDCl₃, δ): 1.25 (t, J = 7.2 Hz, 3H), 3.12 (d, J = 5.0 Hz, 3H), 4.17 (s, 3H), 4.20 (q, J = 7.2 Hz, 2H), 5.33 (s, 2H), 5.34 (q, J = 5.0 Hz, 1H), 6.91 (s, 1H), 7.43-7.27 (m, 4H), 8.75 (s, 1H).
APCIMS m/z: [M+H]⁺ 402.

### Example 91:

### 7-Carboxymethoxy-5-(3-chlorophenyl)-8-methoxy-2-(methylamino)quinazoline (Compound 91)

Compound 91 was prepared in the same manner as in Example 27 using Compound 90.
¹H NMR (DMSO-d₆, δ): 2.88 (s, 3H), 3.95 (s, 3H), 4.76 (s, 2H), 7.10 (s, 1H), 7.53-7.16 (m, 4H), 8.52 (s, 1H).
APCIMS m/z: [M-H]⁻ 372.

### Example 92:

### 5-(3-Chlorophenyl)-7-hydroxymethyl-8-methoxy-2-(methylamino)quinazoline (Compound 92)

Compound 82 (50.1 mg, 0.153 mmol) was suspended in ethanol (5 mL) and THF (5 mL), and sodium borohydride (6.7 mg, 0.177 mmol) was added thereto with the aid of a sodium chloride-ice bath, and then the mixture was stirred at the same temperature for 4 hours. After acetone was added to the reaction mixture, the mixture was warmed to room temperature, and the solvent was evaporated under reduced pressure. To the residue was added water, and the precipitated solid was collected by filtration. The resulting solid was washed with water, and purified by preparative thin-layer chromatography (ethyl acetate/hexane = 3/1) to give Compound 92 (10.2 mg, 20 %) as a yellow solid.
¹H NMR (CDCl₃, δ): 3.12 (brs, 3H), 4.09, (s, 3H), 4.52 (brs, 2H), 5.38 (brs, 1H), 7.20-7.43 (m, 5H), 8.59 (brs, 1H).
APCIMS m/z: [M+H]⁺ 330.

### Example 93:

### 7-{N-(carboxymethyl)aminomethyl}-5-(3-chlorophenyl)-8-methoxy-2-(methylamino)quinazoline mono hydrochloride (Compound 93)

Compound 82 (350 mg, 1.01 mmol) was dissolved in 1,2-dichloroethane (10 mL), and glycine hydrochloride (161 mg, 1.28 mmol) and sodium triacetoxyborohydride (714.8 mg, 3.37 mol) was added thereto, and then the mixture was stirred at room temperature for 8 hours. To the reaction mixture was added water, and the pH of the mixture was adjusted to 2 with 2 mol/L hydrochloric acid. Then, to the mixture was added ethyl acetate and the mixture was separate into an organic layer and an aqueous layer. The pH of the aqueous layer adjusted to 10 with 1 mol/L aqueous sodium hydroxide, and then extracted with ethyl acetate. The organic layer was washed with brine, dried over anhydrous magnesium sulfate, and the solvent was evaporated under reduced pressure. The residue was dissolved in ethanol (3.5 mL), and 1 mol/L aqueous sodium hydroxide (1.9 mL) was added thereto, and then the mixture was stirred overnight. After the pH of the reaction mixture was adjusted to 5 with 1 mol/L hydrochloric acid, the precipitated crystal was collected by filtration, and washed with water. The resulting solid was suspended in' ethanol, and 4 mol/L hydrogen chloride - ethyl acetate was added thereto, and the mixture was stirred at room temperature for 1 hour. Then, the solvent was evaporated under reduced pressure. The resulting crystal was triturated with 2-propanol to give Compound 93 (65.9 mg, 15 %) as a yellow solid.
¹H NMR (DMSO-d₆, δ): 2.51-2.73 (m, 2H), 2.91 (s, 3H), 3.75 (s, 2H), 4.00 (s, 3H), 7.31-7.61 (m, 5H), 8.35 (brs, 1H).
APCIMS m/z: [M+H]⁺ 387.

### Example 94:

### 7-[N-(carboxymethyl)-N-methylaminomethyl]-5-(3-chlorophenyl)-8-methoxy-2-(methylamino)quinazoline mono hydrochloride (Compound 94)

Compound 94 was prepared in the same manner as in Example 93 using Compound 82 and sarcosine.
¹H NMR (DMSO-d₆, δ): 2.50-2.51 (m, 2H), 2.62 (s, 3H), 2.91 (s, 3H), 3.96-4.01 (m, 5H), 7.29=7.62 (m, 5H), 8.33 (brs, 1H).
APCIMS m/z: [M+H]⁺ 401.

### Example 95:

### 7-[E-(2-carboxyethenyl)]-5-(3-chlorophenyl)-8-methoxy-2-(methylamino)quinazoline (Compound 95)

Compound 26 (124 mg, 0.311 mmol) was dissolved in ethanol (3 mL), and 1 mol/L aqueous sodium hydroxide (1 mL) was added thereto, and then the mixture was stirred at room temperature for 8 hours. Ethanol was evaporated under reduced pressure from the mixture, and the aqueous layer was washed with ethyl acetate. Then, the pH of the aqueous layer was adjusted to 4 with 1 mol/L hydrochloric acid, and the precipitated crystal was collected by filtration. The crystal was washed with water, and triturated with chloroform to give Compound 95 (29.9 mg 26 %) as a yellow solid.
¹H NMR (CDCl₃, δ): 3.15 (brs, 3H), 4.10 (s, 3H), 5.74 (brs, 1H), 7.15-7.18 (m, 1H), 7.26-7.29 (m, 1H), 7.42-7.48 (m, 3H), 7.56 (d, J = 15.7 Hz, 1H), 8.60 (brs, 1H).
APCIMS m/z: [M+H]⁺ 370.
m.p.: 291-295°C.

### Example 96:

### 7-(4-Carboxy-1-cyclohexenyl)-5-(3-chlorophenyl)-8-methoxy-2-(methylamino)quinazoline (Compound 96)

### Step 1:

To Compound bf (0.615 g, 1.45 mmol) were added dimethoxyethane (9.6 mL), water (6.4 mL), sodium carbonate (0.307 g, 2.90 mmol), lithium bromide (0.378 g, 4.35 mmol), triphenyl phosphine (0.0304 g, 0.116 mmol), ethyl 4-trifluoromethanesulfonyloxy-3-cyclohexene-1-carboxylate (for example, it is prepared according to the method described in WO2002/59105) (0.525 g, 1.73 mmol) and 10 % palladium-carbon (50 % water-containing reagent, 0.148 g), and the mixture was heated under reflux for 3 hours under an argon atmosphere. After the reaction mixture was cooled to room temperature, water was added thereto, then the mixture was extracted with chloroform, and the organic layer was dried over anhydrous sodium sulfate. The solvent was evaporated, and the residue was roughly purified by silica gel column chromatography (ethyl acetate/n-hexane) to give 5-(3-chlorophenyl)-7-(4-ethoxycarbonyl-1-cyclohexenyl)-8-methoxy-2-(methylamino)quinazoline (Compound bj) (0.62 g, crude product).
¹H NMR (CDCl₃, δ): 1.27 (t, J = 7.3 Hz, 3H), 1.50-2.50 (m, 7H), 3.16 (m, 3H), 4.07 (s, 3H), 4.16 (q, J = 7.3 Hz, 2H), 5.77 (s, 1H), 6.93 (s, 1H), 7.09-7.44 (m, 5H), 8.71 (s, 1H).

### Step 2:

To Compound bj (0.62 g, crude product) obtained in the step 1 were added ethanol (18 mL) and aqueous potassium hydroxide (6 mol/L, 3.6 mL). After the mixture was stirred at room temperature for 5 hours, methanol (10 mL) and water (4 mL) were added thereto, and the mixture was stirred at 40°C for 24 hours. After the solvent was evaporated, hydrochloric acid was added to the residue to adjust the pH of the mixture to 1, and then the mixture was extracted with chloroform. The organic layer was dried over anhydrous sodium sulfate, and the solvent was evaporated. The residue was purified by silica gel column chromatography (methanol/chloroform), followed by trituration (with toluene) to give Compound 96 (0.356 g, 58 % in 2 steps) as a yellow solid.
¹H NMR (DMSO-d₆, δ): 1.55 (m, 1H), 2.30-1..87 (m, 6H), 2.89 (d, J = 4.6 Hz, 3H), 3.94 (s, 3H), 5.48 (s, 1H), 6.99 (s, 1H), 7.51-7.25 (m, 5H), 8.53 (s, 1H).
APCIMS m/z: [M+H]⁺ 424.

### Example 97:

### 7-Bromo-8-methoxy-2-methylamino-5-phenylquinazoline

### (Compound 97)

Compound 97 was prepared in the same manner as in Example 1 using Compound v.
¹H NMR (DMSO-d₆, δ): 2.88 (d, J = 4.8 Hz, 3H), 3.96 (s, 3H), 7.28-7.32 (m, 2H), 7.37 (s, 1H), 7.46-7.57 (m, 4H), 8.34 (brs, 1H).
APCIMS m/z: [M+H]⁺ 344, 346.

### Example 98:

### 8-Methoxy-2-methylamino-7-(4-oxo-1-cycrohexenyl)-5-phenylquinazoline (Compound 98)

### Step 1:

Using Compound 97, 8-Methoxy-2-methylamino-5-phenyl-7-(4,4,5,5-tetramethyl-1,3,2-dioxaborolan-2-yl)quinazoline (Compound bk) was prepared in the same manner as in Reference Example 12.
¹H NMR (CDCl₃, δ): 1.14 (s, 12H), 3.16 (brs, 3H), 4.08 (s, 3H), 5.37 (br s, 1H), 7.09-7.45 (m, 6H), 9.04 (brs, 1H).

### Step 2:

Using Compound bk obtained in the step 1 and 1,4-dioxaspiro[4,5]-7-decen-8-yl trifluoromethanesulfonate (for example, it is prepared according to the method described in US2002/23552), 7-(1,4-Dioxaspiro[4,5]-7-decen-8-yl)-8-methoxy-2-methylamino-5-phenylquinazoline (compound bl) was prepared in the same manner as in Example 11.
¹H NMR (CDCl₃, δ): 1.51-1.56 (m, 2H), 2.00-2.10 (m, 2H), 2.24-2.29 (m, 2H), 3.13 (d, J = 5.1 Hz, 3H), 3.89-3.97 (m, 4H), 4.05 (s, 3H), 5.27 (brs, 1H), 5.49 (m, 1H), 6.98 (s, 1H), 7.27-7.68 (m, 5H), 8.79 (s, 1H).
APCIMS m/z: [M+H]⁺ 404.

### Step 3:

Compound bl (140 mg, 0.350 mmol) obtained in the step 2 was dissolved in THF (12 mL), and 5 mol/L hydrochloric acid (2.5 mL) was added thereto, and then the mixture was stirred at room temperature for 1 hour. The reaction mixture was poured into a mixture of ice-water and saturated aqueous sodium hydrogencarbonate, and extracted with ethyl acetate. The organic layer was washed with brine, and dried over anhydrous magnesium sulfate. The solvent was evaporated under reduced pressure, and the residue was triturated with ethanol to give Compound 98 (130 mg, quantitative) as a pale yellow crystal.
¹H NMR (CDCl₃, δ): 2.17-2.25 (m, 4H), 2.92 (d, J = 3.9 Hz, 2H), 3.14 (d, J = 5.0 Hz, 3H), 4.07 (s, 3H), 5.33 (brs, 1H), 5.77 (t, J = 3.6 Hz, 1H), 6.97 (s, 1H), 7.26-7.68 (m, 5H), 8.76 (s, 1H).
APCIMS m/z: [M+H]⁺ 360.

### Example 99:

### 7-(4-Hydroxy-1-cyclohexenyl)-8-methoxy-2-methylamino-5-phenylquinazoline (Compound 99)

Compound 98 (120 mg, 0.340 mmol) was dissolved in methanol (15 mL), and cooled to -10°C under a nitrogen atmosphere. To the solution was added sodium borohydride (25 mg, 0.67 mmol), and the mixture was stirred at -10°C for 1.5 hours. To the reaction mixture was added acetone (1 mL), and the mixture was warmed to room temperature, then water was added thereto, and the mixture was extracted with ethyl acetate. The organic layer was washed with water and brine, and dried over anhydrous magnesium sulfate. The solvent was evaporated under reduced pressure, and the residue was purified by silica gel column chromatography (n-hexane/ethyl acetate = 2/1 to 1/3) and then triturated with ethanol to give Compound 99 (32 mg, 26 %) as a pale yellow crystal.
¹H NMR (CDCl₃, δ): 1.50-1.73 (m, 2H), 1.93-2.08 (m, 2H), 2.34-2.40 (m, 2H), 3.13 (d, J = 5.1 Hz, 3H), 3.78-3.86 (m, 1H), 4.06 (s, 3H), 5.29 (brs, 1H), 5.46-5.52 (m, 1H), 6.93 (s, 1H), 7.24-7.36 (m, 2H), 7.37-7.44 (m, 4H), 8.75 (s, 1H).
APCIMS m/z: [M+H]⁺ 362.

### Example 100:

### 7-(trans-4-Hydroxycyclohexyl)-8-methoxy-2-methylamino-5-phenylquinazoline (Compound 100)

### Step 1:

Compound 9.8 (40 mg, 0.11 mmol) was dissolved in acetic acid (4 mL), and platinum oxide (20 mg) was added thereto, and then the mixture was stirred at room temperature for 5 hours under a hydrogen atmosphere. The reaction mixture was filtered through Celite, and washed with ethanol. The solvent was evaporated under reduced pressure to give 3,4-dihydro-7-(4-hydroxycyclohexyl)-8-methoxy-2-methylamino-5-phenylquinazoline (Compound bm) (40 mg, quantitative) as pale yellow oil.
¹H NMR (CDCl₃, δ): 1.07-1.94 (m, 9H), 2.94 (s, 3H), 3.96 (s, 3H), 4.02 (s, 2H), 6.73-7.38 (m, 6H).
ESIMS m/z: [M+H]⁺ 366.

### Step 2:

Compound bm (74 mg, 0.20 mmol) obtained in the step 1 was dissolved in chloroform (20 mL), and manganese dioxide (350 mg, 4.00 mmol) was added thereto, and then the mixture was stirred at room temperature for 40 hours followed by stirred at 50°C for 3 hours. The reaction mixture was filtered through Celite, and washed with chloroform. The solvent was evaporated under reduced pressure, and the residue was purified by silica gel column chromatography (n-hexane/ethyl acetate = 2/1 to 0/1) and then triturated with isopropyl ether to give Compound 100 (15 mg, 21 %) as a pale yellow crystal.
¹H NMR (CDCl₃, δ): 1.12-2.02 (m, 8H), 2.45-2.53 (m, 1H), 3.11 (d, J = 5.1 Hz, 3H), 3.62-3.67 (m, 1H), 4.06 (s, 3H), 5.27 (brs, 1H), 6.98 (s, 1H), 7.21-7.24 (m, 3H), 7.43-7.47 (m, 3H), 8.49 (s, 1H).
APCIMS m/z: [M+H]⁺ 364.

### Example 101:

### 7-(cis-4-Hydroxycyclohexyl)-8-methoxy-2-(methylamino)-5-phenylquinazoline (Compound 101)

As a side product in the step 2 of Example 100, Compound 101 (16 mg, 22 %) was obtained as a pale yellow crystal.
¹H NMR (CDCl₃, δ): 1.26-2.05 (m, 8H), 2.48-2.56 (m, 1H), 3.11 (d, J = 5.1 Hz, 3H), 4.00-4.05 (m, 2H), 4.07 (s, 3H), 5.27 (brs, 1H), 7.10 (s, 1H), 7.22-7.26 (m, 2H), 7.42-7.48 (m, 3H), 8.49 (s, 1H).

APCIMS m/z: [M+H]⁺ 364.

### Example 102:

### 7-[4-(Carboxymethyl)phenyl]-5-(5-chloropyridine-3-yl)-8-methoxy-2-(methylamino)quinazoline (Compound 102)

Compound 102 was obtained as a pale yellow crystal by reacting Compound 10 with 4-carboxymethylphenylboronic acid in the same manner as in Example 11, followed by recrystallizing from DMSO-water.
¹H NMR (DMSO-d₆, δ): 2.92 (d, J = 3.7 Hz, 3H), 3.42 (s, 2H), 3.97 (s, 3H), 7.05-7.17 (m, 5H), 7.49 (brs, 1H), 7.90 (s, 1H), 8.23 (s, 1H), 8.53 (s, 1H), 8.54 (s, 1H).
APCIMS m/z: [M+H]⁺ 435.
m. p.: 260°C.

### Example 103:

### 7-[4-(1-Carboxy-1-methylethyl)phenyl]-5-(5-chloropyridine-3-yl)-8-methoxy-2-(methylamino)quinazoline (Compound 103)

### Step 1:

Using Compound 10, 5-(5-Chloropyridine-3-yl)-8-methoxy-2-methylamino-7-(4,4,5,5-tetramethyl-1,3,2-dioxaborolan-2-yl)quinazoline (Compound bn) was prepared in the same manner as in Reference Example 12.
¹H NMR (CDCl₃, δ): 1.17 (s, 12H), 3.16 (s, 3H), 4.10 (s, 3H), 5.45 (brs, 1H), 7.41 (s, 1H), 7.64 (dd, J = 1.6, 2.2 . Hz, 1H), 8.41 (d, J = 1.6 Hz, 1H), 8.63 (d, J = 2.2 Hz, 1H), 8.67 (s, 1H).

### Step 2:

Compound 103 was prepared in the same manner as in Example 11 using Compound bn obtained in the step 1 and 2-(4-bromophenyl)-2-methylpropionic acid.
¹H NMR (DMSO-d₆, δ): 1.45 (s, 6H), 2.92 (s, 3H), 3.98 (s, 3H), 7.12 (d, J = 8.3 Hz, 2H), 7.19 (s, 1H), 7.24 (d, J = 8.3 Hz, 2H), 7.50 (brs, 1H), 7.87 (dd, J = 1.6, 2.2 Hz, 1H), 8.25 (d, J = 1.6 Hz, 1H), 8.56 (d, J = 2.2 Hz, 1H), 8.56 (s, 1H).
APCIMS m/z: [M+H]⁺ 463.

### Example 104:

### 7-[4-(2-Carboxyethyl)phenyl]-5-(5-chloropyridine-3-yl)-8-methoxy-2-(methylamino)quinazoline (Compound 104)

Compound 104 was obtained as a pale yellow crystal by reacting Compound bn with 3-(4-trifluoromethanesulfonyloxyphenyl)propionic acid in the same manner as in Example 11, followed by triturating with ethanol.
¹H NMR (DMSO-d₆, δ) : 2.32-2.37 (m, 2H), 2.71-2.77 (m, 2H), 2.91 (d, J = 4.1 Hz, 3H), 3.96 (s, 3H), 7.04-7.15 (m, 5H), 7.46 (brs, 1H), 7.84 (s, 1H), 8.21 (s, 1H), 8.54 (s, 2H).
APCIMS m/z: [M+H]⁺ 449.
m.p.: 214°C.

### Example 105:

### 7-(5-Carboxythiophene-2-yl)-5-(5-chloropyridine-3-yl)-8-methoxy-2-(methylamino)quinazoline (Compound 105)

Compound 105 was prepared in the same manner as in Example 11 using Compound bn and 2-bromothiophene-5-carboxylic acid.
¹H NMR (DMSO-d₆, δ): 2.91(s, 3H), 4.00 (s, 3H), 7.09 (d, J = 3.0 Hz, 1H), 7.36 (s, 1H), 7.55 (d, J = 3.0 Hz, 1H), 8.00 (s, 1H), 8.39 (s, 1H), 8.48 (s, 1H), 8.71 (s, 1H).
APCIMS m/z: [M+H]⁺ 427.

### Example 106:

### 7-[5-(Carboxymethyl)thiophene-2-yl]-5-(5-chloropyridine-3-yl)-8-methoxy-2-(methylamino)quinazoline (Compound 106)

Compound 106 was prepared in the same manner as in Example 11 using Compound bn and ethyl 2-bromothiophene-5-acetate.
¹H NMR (DMSO-d₆, δ): 2.91 (s, 3H), 3.70 (s, 2H), 4.00 (s, 3H), 6.79 (d, J = 3.6 Hz, 1H), 6.85 (d, J = 3.6 Hz, 1H), 7.30 (s, 1H), 7.56 (brs, 1H), 7.97 (t, J = 2.1 Hz, 1H), 8.37 (d, J = 2.1 Hz, 1H), 8.46 (s, 1H), 8.69 (d, J = 2.1 Hz, 1H).
APCIMS m/z: [M+H]⁺ 441.

### Example 107:

### 7-Bromo-5-(3-chlorophenyl)-8-hydroxy-2-(methylamino)quinazoline (Compound 107)

Compound 8 (1.00 g, 2.64 mmol) was suspended in DMF (40 mL), and sodium thiomethoxide (740 mg, 10.6 mmol) was added thereto, and then the mixture was stirred under a nitrogen atmosphere at 90°C for 1 hour. After the reaction mixture was cooled to room temperature and saturated aqueous ammonium chloride was added thereto, then the mixture was extracted with ethyl acetate. The organic layer was washed with water and brine, and dried over anhydrous magnesium sulfate. The solvent was evaporated under reduced pressure to give Compound 107 (960 mg, quantitative) as pale yellow oil.
¹H NMR (CDCl₃, δ): 3.10 (d, J = 5.1 Hz, 3H), 5.41 (brs, 1H), 7.17-7.23 (m, 1H), 7.28-7.31 (m, 1H), 7.41-7.45 (m, 3H), 7.61 (brs, 1H), 8.52 (brs, 1H).
APCIMS m/z: [M-H]⁻ 364.

### Example 108:

### 7-Bromo-5-(3-chlorophenyl)-8-difluoromethoxy-2-(methylamino)quinazoline (Compound 108)

Compound 107 (960 mg, 2.64 mmol) was dissolved in DMF (40 mL), and sodium chlorodifluoroacetic acid (1.21 g, 7.92 mmol) and cesium carbonate (2.58 g, 7.92 mmol) were added thereto, and then the mixture was stirred at 60°C for 3 hours. To the reaction mixture were added saturated aqueous ammonia and water, and the mixture was extracted with ethyl acetate. The organic layer was washed with water and brine, and dried over anhydrous magnesium sulfate. The solvent was evaporated under reduced pressure, and the residue was purified by silica gel column chromatography (n-hexane/ethyl acetate = 9/1 to 4/1) followed by triturated with ethanol to give Compound 108 (660 mg, 60 %) as a pale yellow crystal.
¹H NMR (CDCl₃, δ): 3.11 (d, J = 5.1 Hz, 3H), 5.43 (brs, 1H), 7.16-7.20 (m, 1H), 7.29-7.30 (m, 1H), 7.42-7.51 (m, 3H), 7.73 (s, 1H), 8.55 (brs, 1H).
APCIMS m/z: [M+H]⁺ 416.

### Example 109:

### 7-(4-Carboxyphenyl)-5-(3-chlorophenyl)-8-difluoromethoxy-2-(methylamino)quinazoline (Compound 109)

Compound 109 was obtained as a pale yellow crystal by reacting Compound 108 with 4-carboxyphenylboronic acid in the same manner as in Example 11, followed by triturating with ethanol.
¹H NMR (DMSO-d₆, δ): 2.94 (d, J = 3.8 Hz, 3H), 7.10-7.19 (m, 3H), 7.28-7.40 (m, 3H), 7.56 (s, 1H) , 7.60 (t, J = 75 Hz, 1H), 7.73-7.76 (m, 3H), 8.60 (brs, 1H).
APCIMS m/z: [M+H]⁺ 456.
m.p.: 230°C.

### Example 110:

### 7-Bromo-5-(3-chlorophenyl)-8-ethoxy-2-(methylamino)quinazoline (Compound 110)

Compound 107 (500 mg, 1.37 mmol) was dissolved in anhydrous DMF (10 mL), and anhydrous potassium carbonate (284 mg, 2.06 mmol) and iodoethane (0.165 mL, 2.06 mmol) were added thereto, and then the mixture was stirred at room temperature for 2 hours. To the reaction mixture was added water, and the precipitated solid was collected by filtration, followed by washed with water. The resulting solid was triturated with ethanol to give Compound 110 (464 mg, 86 %) as a yellow solid.
¹H NMR (DMSO-d₆, δ): 1.45 (t, J = 7.0 Hz, 3H), 2.89 (brs, 3H), 4.24 (q, J = 7.0 Hz, 3H), 7.23-7.30 (m, 1H), 7.36 (s, 1H), 7.41 (s, 1H), 7.52-7.57 (m, 3H), 8.36 (brs, 1H).

### Example 111:

### 7-(4-Carboxyphenyl)-5-(3-chlorophenyl)-8-ethoxy-2-(methylamino)quinazoline (Compound 111)

Compound 111 was prepared in the same manner as in Example 11 using Compound 110 and 4-carboxyphenylboronic acid.
¹H NMR (CDCl₃, δ): 1.61 (t, J= 6.9 Hz, 3H), 3.17 (brs, 3H), 4.32 (q, J = 6.9 Hz, 2H), 7.01 (d, J = 6.6 Hz, 1H), 7.11 (s, 1H), 7.19-7.29 (m, 5H), 7.95 (d, J = 8.1 Hz, 2H), 8.77 (s, 1H).
APCIMS m/z: [M+H]⁺ 334.

### Example 112:

### 7-(4-Carboxyphenyl)-5-(3-chlorophenyl)-8-hydroxy-2-(methylamino)quinazoline (Compound 112)

Compound 18 (879 mg, 2.09 mmol) was dissolved in anhydrous DMF, and sodium thiomethoxide (596 mg, 8.50 mmol) was added thereto, and then the mixture was stirred at 90°C for 2 hours under a nitrogen atmosphere. The reaction mixture was cooled to room temperature, and saturated aqueous ammonium chloride was added thereto, then the precipitated solid was collected by filtration. The resulting solid was washed with water, followed by triturated with hexane and diisopropyl ether to give Compound 112 (490 mg, 58 %) as a yellow solid.
¹H NMR (DMSO-d₆, δ) : 2.98 . (brs, 3H), 7.12-7.21 (m, 5H), 7.30-7.35 (m, 2H), 7.45 (brs, 1H), 7.75 (d, J = 8.4 Hz, 2H), 8.55 (s, 1H), 9.38 (brs, 1H).
APCIMS m/z: [M+H]⁺ 406.
m.p.: 291-296°C

### Example 113:

### 7-[E-(2-carboxyethenyl)]-5-(3-chlorophenyl)-8-difluoromethoxy-2-(methylamino)quinazoline (Compound 113)

### Step 1:

Using Compound 108, 7-{[E-(2-ethoxycarbonyl)ethenyl]}-5-(3-chlorophenyl)-8-difluoromethoxy-2-(methylamino)quinazoline (Compound bo) was prepared in the same manner as in Example 21.
¹H NMR (CDCl₃, δ): 1.28 (t, J = 7.2 Hz, 3H), 3.13 (brs, 3H), 4.19 (q, J = 7.2 Hz, 2H), 5.68 (brs, 1H), 6.37 (d, J = 15.9 Hz, 1H), 7.16 (dt, J = 1.8, 6.6 Hz, 1H), 7.27 (s, 1H), 7.40-7.55 (m, 4H), 7.81 (t, J = 72.3 Hz, 1H), 8.58 (brs, 1H).

### Step 2:

Compound 113 was prepared in the same manner as in Example 27 using bo obtained in the step 1.
¹H NMR (CDCl₃, δ): 3.12 (brs, 3H), 5.65 (brs, 1H), 6.32 (d, J = 15.7 Hz, 1H), 7.12-7.77 (m, 7H), 8.56 (brs, 1H).
APCIMS m/z: [M+H]⁺ 406.

### Example 114:

### 7-Bromo-5-(2-chlorophenyl)-8-methoxy-2-(methylamiho)quinazoline (Compound 114)

Compound 114 was prepared in the same manner as in Example 1 using Compound w.
¹H NMR (DMSO-d₆, δ): 2.89 (d, J = 4.8 Hz, 3H), 3.98 (s, 3H), 7.36-7.40 (m, 2H), 7.48-7.57 (m, 3H), 7.65-7.68 (m, 1H), 8.20 (brs, 1H).
APCIMS m/z: [M+H]⁺ 378, 380.

### Example 115:

### 7-Bromo-5-(4-chlorophenyl)-8-methoxy-2-(methylamino)quinazoline (Compound 115)

Compound 115 was prepared in the same manner as in Example 1 using Compound x.
¹H NMR (DMSO-d₆, δ): 2.88 (d, J = 4.6 Hz, 3H), 3.96 (s, 3H), 7.35 (d, J = 8.3 Hz, 2H), 7.37 (s, 1H), 7.55 (brs, 1H), 7.59 (d, J = 8.3 Hz, 2H), 8.37 (brs, 1H).

APCIMS m/z: [M+H]⁺ 378, 380.

### Example 116:

### 7-Bromo-5-(2,3-dichlorophenyl)-8-methoxy-2-(methylamino)quinazoline (Compound 116)

Compound 116 was prepared in the same manner as in Example 1 using Compound y.
¹H NMR (DMSO-d₆, δ): 2.87 (d, J = 4.8 Hz, 3H), 3.98 (s, 3H), 7.37 (dd, J = 1.5, 7.8 Hz, 1H), 7.40 (s, 1H), 7.53 (t, J = 7.8 Hz, 1H), 7.58 (brs, 1H), 7.80 (dd, J = 1.5, 7.8 Hz, 1H), 8.26 (brs, 1H).
APCIMS m/z: [M+H]⁺ 412, 414.

### Example 117:

### 7-Bromo-5-(2,5-dichlorophenyl)-8-methoxy-2-(methylamino)quinazoline (Compound 117)

Compound 117 was prepared in the same manner as in Example 1 using Compound z.
¹H NMR (DMSO-d₆, δ): 2.89 (d, J = 4.8 Hz, 3H), 3.98 (s, 3H), 7.40 (s, 1H), 7.53 (d, J = 2.4 Hz, 1H), 7.59 (brs, 1H), 7.61 (dd, J = 2.4, 8.6 Hz, 1H), 7.70 (d, J = 8.6 Hz, 1H), 8.27 (brs, 1H).
APCIMS m/z: [M+H]⁺ 412, 414.

### Example 118:

### 7-Bromo-5-(3,4-dichlorophenyl)-8-methoxy-2-(methylamino)quinazoline (Compound 118)

Compound 118 was prepared in the same manner as in Example 1 using Compound aa.
¹H NMR (DMSO-d₆, δ): 2.87 (d, J = 4.6 Hz, 3H), 3.95 (s, 3H), 7.32 (dd, J = 1.7, 8.3 Hz, 1H), 7.36 (s, 1H), 7.56 (brs, 1H), 7.64 (d, J = 1.7 Hz, 1H), 7.77 (d, J = 8.3 Hz, 1H), 8.40 (brs, 1H).
APCIMS m/z: [M+H]⁺ 412, 414.

### Example 119:

### 7-Bromo-5-(3,5-dichlorophenyl)-8-methoxy-2-(methylamino)quinazoline (Compound 119)

Compound 119 was prepared in the same manner as in Example 1 using Compound ab.
¹H NMR (DMSO-d₆, δ): 2.87 (d, J = 4.6 Hz, 3H), 3.95 (s, 3H), 7.36 (s, 1H), 7.42 (d, J = 1.8 Hz, 2H), 7.56 (brs, 1H), 7.74 (t, J = 1.8 Hz, 1H), 8.39 (brs, 1H).
APCIMS m/z: [M+H]⁺ 412, 414.

### Example 120:

### 7-Bromo-5-(3-chloro-4-fluorophenyl)-8-methoxy-2-(methylamino)quinazoline (Compound 120)

Compound 120 was prepared in the same manner as in Example 1 using Compound ac.
¹H NMR (DMSO-d₆, δ): 2.88 (d, J = 4.5 Hz, 3H), 3.96 (s, 3H), 7.31-7.39 (m, 2H), 7.54-7.61 (m, 3H), 8.41 (s, 1H).

### Example 121:

### 7-Bromo-5-(3-fluorophenyl)-8-methoxy-2-(methylamino)quinazoline (Compound 121)

Compound 121 was prepared in the same manner as in Example 1 using Compound ad.
¹H NMR (DMSO-d₆, δ): 2.89 (d, J = 4.3 Hz, 3H), 3.97 (s, 3H), 7.14-7.23 (m, 2H), 7.30-7.37 (m, 2H), 7.54-7.62 (m, 2H), 8.37 (brs, 1H).

### Example 122:

### 7-Bromo-8-methoxy-2-methylamino-5-(3-trifluoromethylphenyl)quinazoline (Compound 122)

Compound 122 was prepared in the same manner as in Example 1 using Compound ae.
¹H NMR (DMSO-d₆, δ): 2.89 (s, 3H), 3.97 (s, 3H), 7.36 (s, 1H), 7.42-7.46 (m, 1H), 7.55-7.84 (m, 4H), 9.45 (s, 1H).

### Example 123:

### 7-Bromo-8-methoxy-2-methylamino-5-(2-pyridyl)quinazoline (Compound 123)

Compound 123 was prepared in the same manner as in Example 1 using Compound af.
¹H NMR (CDCl₃, δ): 3.12 (d, J = 5.1 Hz, 3H), 4.06 (s, 3H), 5.39 (d, J = 5.1 Hz, 1H), 7.23 (s, 1H), 7.39 (ddd, J = 1.0, 4.8, 7.7 Hz, 1H), 7.48 (dt, J = 1.0, 7.7 Hz, 1H), 7.85 (dt, J = 1.8, 7.7 Hz, 1H), 8.57 (brs. 1H), 8.78 (ddd, J = 1.0, 1.8, 4.8 Hz, 1H).
APCIMS m/z: [M+H]⁺ 345, 347.

### Example 124:

### 7-Bromo-8-methoxy-2-methylamino-5-(3-pyridyl)quinazoline (Compound 124)

Compound 124 was prepared in the same manner as in Example 1 using Compound ag.
¹H NMR (CDCl₃, δ): 3.13 (d, J = 5.0 Hz, 3H), 4.07 (s, 3H), 5.46 (brs, 1H), 7.26 (s, 1H), 7.45 (ddd, J = 0.7, 4.9, 7.9 Hz, 1H), 7.67 (dt, J = 2.0, 7.9 Hz, 1H), 8.53 (s, 1H), 8.58 (dd, J = 0.7, 2.0 Hz, 1H), 8.72 (dd, J = 2.0, 4.9 Hz, 1H).

### Example 125:

### 7-Bromo-8-methoxy-2-methylamino-5-(4-pyridyl)quinazoline (Compound 125)

Compound 125 was prepared in the same manner as in Example 1 using Compound ah.
¹H NMR (CDCl₃, δ): 3.13 (d, J = 5.1 Hz, 3H), 4.06 (s, 3H), 5.44 (brs, 1H), 7.24 (s, 1H), 7.27 (dd, J = 1.5, 4.3 Hz, 2H), 8.51 (brs, 1H), 8.65 (dd, J = 1.5, 4.3 Hz, 2H).
APCIMS m/z: [M+H]⁺ 345, 347.

### Example 126:

### 7-Bromo-5-(5-cyano-3-pyridyl)-8-methoxy-2-(methylamino)quinazoline (Compound 126)

Compound 126 was prepared in the same manner as in Example 1 using Compound ai. ¹H NMR (CDCl₃, δ): 3.14 (d, J = 5.0 Hz, 3H), 4.08 (s, 3H), 5.46 (brs, 1H), 7.25 (s, 1H), 7.97 (t, J = 2.0 Hz, 1H), 8.45 (brs, 1H), 8.78 (d, J = 2.0 Hz, 1H), 8.99 (d, J = 2.0 Hz, 1H).

### Example 127:

### 7-Bromo-5-(5-chloro-2-thienyl)-8-methoxy-2-(methylamino)quinazoline (Compound 127)

Compound 127 was prepared in the same manner as in Example 1 using Compound aj.
¹H NMR (CDCl₃, δ): 3. 13 (d, J = 5.0 Hz, 3H), 4.04 (s, 3H), 5.60 (brs, 1H), 6.82 (d, J = 3.8 Hz, 1H), 7.00 (d, J = 3.8 Hz, 1H), 7.22 (s, 1H), 8.78 (s, 1H).

### Example 128:

### 5-(5-Benzofrazanyl)-7-bromo-8-methoxy-2-(methylamino)quinazoline (Compound 128)

Compound 128 was prepared in the same manner as in Example 1 using Compound ak.
¹H NMR (CDCl₃, δ): 3.14 (d, J = 5.1 Hz, 3H), 4.08 (s, 3H), 5.45 (brs, 1H), 7.36 (dd, J = 1.2, 9.2 Hz, 1H), 7.81 (t, J = 1.2 Hz, 1H), 7.96 (dd, J = 1.2, 9.2 Hz, 1H), 8.62 (brs, 1H).

### Example 129:

### 7-Bromo-8-methoxy-2-methylamino-5-(4-tetrahydropyranyl)quinazoline (Compound 129)

Compound 129 was prepared in the same manner as in Example 1 using Compound am.
¹H NMR (CDCl₃, δ): 1.70-1.75 (m, 2H), 2.46 (brs, 2H), 3.14 (d, J = 5.1 Hz, 3H), 3.55-3.63 (m, 2H), 3.77-3.86 (m, 1H), 3.97 (s, 3H), 4.13-4.18 (m, 2H), 5.37 (d, J = 5.1 Hz, 1H), 7.15 (s, 1H), 9.61 (brs, 1H).
APCIMS m/z: [M+H]⁺ 352, 354.

### Example 130:

### 7-Bromo-5-cyclohexyl-8-methoxy-2-(methylamino)quinazoline (Compound 130)

Compound 130 was prepared in the same manner as in Example 1 using Compound ao.
¹H NMR (CDCl₃, δ): 1.33-1.53 (m, 4H), 1.63 (brs, 2H), 1.81-2.05 (m, 4H), 3.13 (d, J = 5.1 Hz, 3H), 3.55 (brs, 1H), 3.98 (s, 3H), 5.35 (brs, 1H), 7.15 (s, 1H), 9.64 (brs, 1H). APCIMS m/z: [M+H]⁺ 350, 352.

### Example 131:

### 7-Bromo-8-methoxy-2-methylamino-5-piperidinoquinazoline (Compound 131)

Compound 131 was prepared in the same manner as in Example 1 using Compound ap.
¹H NMR (CDCl₃, δ): 1.40-1.52 (m, 1H), 1.71-1.74 (m, 4H), 1.79-1.84 (m, 1H), 2.90-2.94 (m, 2H), 3.14 (d, J = 5.1 Hz, 1H), 3.47-3.59 (m, 2H), 3.97 (s, 3H), 5.37 (d, J = 5.1 Hz, 1H), 7.05 (s, 1H), 9.52 (brs, 1H).

### Example 132:

### 7-Bromo-8-methoxy-2-methylamino-5-morpholinoquinazoline (Compound 132)

Compound 132 was prepared in the same manner as in Example 1 using Compound aq.
¹H NMR (CDCl₃, δ): 2.78-2.81 (m, 2H), 3.15 (d, J = 5.1 Hz, 3H), 3.80-3.84 (m, 4H), 3.93-3.96 (m, 2H), 3.99 (s, 3H), 5.40 (brs, 1H), 7.07 (s, 1H), 9.57 (brs, 1H).
APCIMS m/z: [M+H]⁺ 353, 355.

### Example 133:

### 7-(4-Carboxyphenyl)-8-methoxy-2-methylamino-5-phenylquinazoline (Compound 133)

Compound 133 was prepared in the same manner as in Example 11 using Compound 97 and 4-carboxyphenylboronic acid.
¹H NMR (DMSO-d₆, δ): 2.92 (d, J = 4.8 Hz, 3H), 3.98 (s, 3H), 7.15-7.18 (m, 2H), 7.20 (s, 1H), 7.26 (d, J = 8.3 Hz, 2H), 7.32-7.37 (m, 3H), 7.47 (brs, 1H), 7.74 (d, J = 8.3 Hz, 2H), 8.55 (brs, 1H).
APCIMS m/z: [M+H]⁺ 386.

### Example 134:

### 7-(4-Carboxyphenyl)-5-(2-chlorophenyl)-8-methoxy-2-(methylamino)quinazoline (Compound 134)

Compound 134 was prepared in the same manner as in Example 11 using Compound 114 and 4-carboxyphenylboronic acid.
¹H NMR (DMSO-d₆, δ): 2.92 (d, J = 4.8 Hz, 3H), 4.00 (s, 3H), 7.20-7.23 (m, 1H), 7.22 (s, 1H), 7.29 (d, J = 8.3 Hz, 2H), 7.35-7.41 (m, 1H), 7.50-7.53 (m, 2H), 7.77 (d, J = 8.3 Hz, 2H), 8.32 (brs, 1H).

APCIMS m/z: [M-H]⁻ 418.

### Example 135:

### 7-(4-Carboxyphenyl)-5-(4-chlorophenyl)-8-methoxy-2-(methylamino)quinazoline (Compound 135)

Compound 135 was prepared in the same manner as in Example 11 using Compound 115 and 4-carboxyphenylboronic acid.
¹H NMR (DMSO-d₆, δ): 2.92 (d, J = 4.8 Hz, 3H), 3.98 (s, 3H), 7.19 (s, 1H), 7.19 (d, J = 8.3 Hz, 2H), 7.26 (d, J = 8.3 Hz, 2H), 7.39 (d, J = 8.3 Hz, 2H), 7.50 (brs, 1H), 7.78 (d, J = 8.3 Hz, 2H), 8.56 (brs, 1H).
APCIMS m/z: [M+H]⁺ 420.

### Example 136:

### 7-(4-Carboxyphenyl)-5-(2,3-dichlorophenyl)-8-methoxy-2-(methylamino)quinazoline (Compound 136)

Compound 136 was prepared in the same manner as in Example 11 using Compound 116 and 4-carboxyphenylboronic acid.
¹H NMR (DMSO-d₆, δ): 2.92 (d, J = 4.6 Hz, 3H), 4.00 (s, 3H), 7.21 (s, 1H), 7.24 (dd, J = 1.7, 7.8 Hz, 1H), 7.29 (d, J = 8.3 Hz, 2H), 7.32 (t, J = 7.8 Hz, 1H); 7.54 (brs, 1H), 7.63 (dd, J = 1.7, 7.8 Hz, 1H), 7.79 (d, J = 8.3 Hz, 2H), 8.35 (brs, 1H).
APCIMS m/z: [M+H]⁺ 454.

### Example 137:

### 7-(4-Carboxyphenyl)-5-(2,5-dichlorophenyl)-8-methoxy-2-(methylamino)quinazoline (Compound 137)

Compound 137 was prepared in the same manner as in Example 11 using Compound 117 and 4-carboxyphenylboronic acid.
¹H NMR (DMSO-d₆, δ): 2.92 (d, J = 4.8 Hz, 3H), 4.00 (s, 3H), 7.21 (s, 1H), 7.30 (d, J = 8.1 Hz, 2H), 7.37 (d, J = 2.6 Hz, 2H), 7.45 (dd, J = 2.6, 8.6 Hz, 1H), 7.53 (s, 1H), 7.54 (d, J = 8.6 Hz, 1H), 7.81 (d, J = 8.1 Hz, 2H), 8.37 (brs, 1H).
APCIMS m/z: [M+H]⁺ 454.

### Example 138:

### 7-(4-Carboxyphenyl)-5-(3,4-dichlorophenyl)-8-methoxy-2-(methylamino)quinazoline (Compound 138)

Compound 138 was prepared in the same manner as in Example 11 using Compound 118 and 4-carboxyphenylboronic acid.
¹H NMR (DMSO-d₆, δ): 2.92 (d, J = 4.8 Hz, 3H), 3.98 (s, 3H), 7.16 (dd, J = 1.9, 8.3 Hz, 1H), 7.28 (d, J = 8.3 Hz, 2H), 7.47 (d, J = 1.9 Hz, 1H), 7.53 (brs, 1H), 7.58 (d, J = 8.3 Hz, 1H), 7.81 (d, J = 8.3 Hz, 2H), 8.60 (brs, 1H).
APCIMS m/z: [M+H]⁺ 454.

### Example 139:

### 7-(4-Carboxyphenyl)-5-(3,5-dichlorophenyl)-8-methoxy-2-(methylamino)quinazoline (Compound 139)

Compound 139 was prepared in the same manner as in Example 11 using Compound 119 and 4-carboxyphenylboronic acid.
¹H NMR (DMSO-d₆, δ): 2.93 (d, J = 4.6 Hz, 3H), 3.98 (s, 3H), 7.18 (s, 1H), 7.26 (d, J = 2.0 Hz, 2H), 7.29 (d, J = 8.3 Hz, 2H), 7.54 (brs, 1H), 7.56 (t, J = 2.0 Hz, 1H), 7.82 (d, J = 8.3 Hz, 2H), 8.59 (brs, 1H).
APCIMS m/z: [M+H]⁺ 454.

### Example 140:

### 7-(4-Carboxyphenyl)-5-(3-chloro-4-fluorophenyl)-8-methoxy-2-(methylamino)quinazoline (Compound 140)

Compound 140 was prepared in the same manner as in Example 11 using Compound 120 and 4-carboxyphenylboronic acid.
¹H NMR (DMSO-d₆, δ) 2.92 (s, 3H), 3.98 (s, 3H), 7.14-7.20 (m, 1H), 7.18 (s, 1H), 7.28 (d, J = 8.3 Hz, 2H), 7.34-7.44 (m, 2H), 7.52 (brs, 1H), 7.81 (d, J = 8.3 Hz, 2H), 8.59 (s, 1H).
APCIMS m/z: [M+H]⁺ 438.

### Example 141:

### 7-(4-Carboxyphenyl)-5-(3-fluorophenyl)-8-methoxy-2-(methylamino)quinazoline (Compound 141)

Compound 141 was prepared in the same manner as in Example 11 using Compound 121 and 4-carboxyphenylboronic acid. ¹H NMR(DMSO-d₆, δ): 2.93 (d, J = 6.1 Hz, 3H), 3.98 (s, 3H), 6.99-7.06 (m, 2H), 7.12-7.19 (m, 2H), 7.28 (d, J = 8.1 Hz, 2H), 7.33-7.41 (m, 1H), 7.45 (d, J = 6.1 Hz, 1H), 7.78 (d, J = 8.1 Hz, 2H), 8.57 (brs, 1H), 12.91 (brs, 1H).
APCIMS m/z: [M+H]⁺ 404.

### Example 142:

### 7-(4-Carboxyphenyl)-8-methoxy-2-methylamino-5-(3-trifluoromethylphenyl)quinazoline (Compound 142)

Compound 142 was prepared in the same manner as in Example 11 using Compound 122 and 4-carboxyphenylboronic acid.
¹H NMR (DMSO-d₆, δ): 2.93 (s, 3H), 3.99 (s, 3H), 7.21 (s, 1H), 7.24 (d, J = 8.3 Hz, 2H), 7.45 (s, 1H), 7.48-7.61 (m, 3H), 7.67 (d, J = 7.4 Hz, 1H), 7.76 (d, J = 8.3 Hz, 2H), 8.55 (s, 1H).
APCIMS m/z: [M+H]⁺ 454.

### Example 143:

### 7-(4-Carboxyphenyl)-8-methoxy-2-methylamino-5-(2-pyridyl)quinazoline (Compound 143)

Compound 143 was prepared in the same manner as in Example 11 using Compound 123 and 4-carboxyphenylboronic acid.
¹H NMR (DMSO-d₆, δ): 2.93 (d, J = 4.8 Hz, 3H), 4.01 (s, 1H), 7.03-7.06 (m, 1H), 7.22 (s, 1H), 7.23 (d, J = 8.3 Hz, 2H), 7.31-7.36 (m, 1H), 7.17 (d, J = 4.8 Hz, 1H), 7.63-7.68 (m, 1H), 7.76 (d, J = 8.3 Hz, 2H), 8.60 (brs, 1H), 8.67-8.69 (m, 1H).
APCIMS m/z: [M+H]⁺ 387.

### Example 144:

### 7-(4-Carboxyphenyl)-8-methoxy-2-methylamino-5-(3-pyridyl)quinazoline (Compound 144)

Compound 144 was prepared in the same manner as in Example 11 using Compound 124 and 4-carboxyphenylboronic acid.
¹H NMR (DMSO-d₆, δ): 2.93 (d, J = 4.8 Hz, 3H), 3.99 (s, 3H), 7.21 (s, 1H), 7.25 (d, J = 8.3 Hz, 2H), 7.38 (dd, J = 4.7, 7.7 Hz, 1H), 7.51 (brs, 1H), 7.68 (dt, J = 1.7, 7.7 Hz, 1H), 7.77 (d, J = 8.3 Hz, 2H), 8.29 (d, J = 1.7 Hz, 1H), 8.50 (dd, J = 1.7, 4.7 Hz, 1H), 8.55 (brs, 1H), 12.91 (brs, 1H).
APCIMS m/z: [M+H]⁺ 387.

### Example 145:

### 7-(4-Carboxyphenyl)-8-methoxy-2-methylamino-5-(4-pyridyl)quinazoline (Compound 145)

Compound 145 was prepared in the same manner as in Example 11 using Compound 125 and 4-carboxyphenylboronic acid.
¹H NMR (DMSO-d₆, δ): 2.93 (d, J = 4.8 Hz, 3H), 3.99 (s, 3H), 7.20 (s, 1H), 7.21 (d, J = 5.6 Hz, 2H), 7.27 (d, J = 8.3 Hz, 2H), 7.52 (brs, 1H), 7.78 (d, J = 8.3 Hz, 2H), 8.52 (d, J = 5.6 Hz, 2H), 8.55 (brs, 1H).
APCIMS m/z: [M+H]⁺ 387.

### Example 146:

### 7-(4-Carboxyphenyl)-5-(5-cyano-3-pyridyl)-8-methoxy-2-(methylamino)quinazoline (Compound 146)

Compound 146 was prepared in the same manner as in Example 11 using Compound 126 and 4-carboxyphenylboronic acid.
¹H NMR (DMSO-d₆, δ): 2.93 (d, J = 4.6 Hz, 3H), 4.00 (s, 3H), 7.23 (s, 1H), 7.26 (d, J = 8.3 Hz, 2H), 7.56 (brs, 1H), 7.80 (d, J = 8.3 Hz, 2H), 8.34 (t, J = 2.0 Hz, 1H), 8.49 (d, J = 2.0 Hz, 1H), 8.62 (brs, 1H), 8.94 (d, J = 2.0 Hz, 1H), 12.97 (brs, 1H).
APCIMS m/z: [M+H]⁺ 412.

### Example 147:

### 7-(4-Carboxyphenyl)-5-(5-chloro-2-thienyl)-8-methoxy-2-(methylamino)quinazoline (Compound 147)

Compound 147 was prepared in the same manner as in Example 11 using Compound 127 and 4-carboxyphenylboronic, acid.
¹H NMR (DMSO-d₆, δ): 2.92 (d, J = 4.8 Hz, 3H), 3.98 (s, 3H), 6.99 (d, J = 3.8 Hz, 1H), 7.11 (d, J = 3.8 Hz, 1H), 7.17 (s, 1H), 7.41 (d, J = 8.3 Hz, 2H), 7.58 (brs, 1H), 7.87 (d, J = 8.3 Hz, 2H), 8.82 (brs, 1H), 12.99 (brs, 1H).
APCIMS m/z: [M+H]⁺ 426.

### Example 148:

### 5-(5-Benzofrazanyl)-7-(4-carboxyphenyl)-8-methoxy-2-(methylamino)quinazoline (Compound 148)

Compound 148 was prepared in the same manner as in Example 11 using Compound 128 and 4-carboxyphenylboronic acid.
¹H NMR (DMSO-d₆, δ): 2.93 (d, J = 4.1 Hz, 3H), 4.01 (s, 3H), 7.23 (s, 1H), 7.31 (d, J = 9.3 Hz, 1H), 7.37 (d, J = 7.5 Hz, 2H), 7.54 (brs, 1H), 7.78 (d, J = 7.5 Hz, 2H), 7.94 (d, J = 9.3 Hz, 1H), 7.97 (s, 1H), 8.77 (brs, 1H), 12.95 (brs, 1H).
APCIMS m/z: [M+H]⁺ 428.

### Example 149:

### 7-(4-Carboxyphenyl)-8-methoxy-2-methylamino-5-(4-tetrahydropyranyl)quinazoline (Compound 149)

Compound 149 was prepared in the same manner as in Example 11 using Compound 129 and 4-carboxyphenylboronic acid.
¹H NMR (DMSO-d₆, δ): 1.58-1.63 (m, 2H), 2.10-2.22 (m, 2H), 2.92 (d, J = 4.6 Hz, 3H), 3.13-3.21 (m, 4H), 3.86 (s, 3H), 3.89-3.91 (m, 1H), 6.87 (s, 1H), 7.40 (d, J = 4.6 Hz, 1H), 7.47 (d, J = 8.1 Hz, 2H), 8.03 (d, J = 8.1 Hz, 2H), 9.54 (brs, 1H).
APCIMS m/z: [M+H]⁺ 394.

### Example 150:

### 7-(4-Carboxyphenyl)-5-cyclohexyl-8-methoxy-2-(methylamino)quinazoline (Compound 150)

Compound 150 was prepared in the same manner as in Example 11 using Compound 130 and 4-carboxyphenylboronic acid.
¹H NMR (DMSO-d₆, δ): 1.03-1.08 (m, 2H), 1.31-1.35 (m, 1H), 1.57-1.61 (m, 1H), 1.71-1.74 (m, 4H), 1.96-1.99 (m, 2H), 2.88-2.90 (m, 1H), 2.92 (d, J = 4.6 Hz, 3H), 3.85 (s, 3H), 6.87 (s, 1H), 7.34 (brs, 1H), 7.44 (d, J = 8.2 Hz, 2H), 8.02.(d, J = 8.2 Hz, 2H), 9.59 (brs, 1H).
APCIMS m/z: [M+H]⁺ 392.

### Example 151:

### 7-(4-Carboxyphenyl)-8-methoxy-2-methylamino-5-(1-piperadinyl)quinazoline (Compound 151)

Compound 151 was prepared in the same manner as in Example 11 using Compound 131 and 4-carboxyphenylboronic acid.
¹H NMR (DMSO-d₆, δ) : 0.82-0.87 (m, 1H), 1.22 (brs, 2H), 1.29-1.34 (m, 1H), 1.48 (brs, 4H), 1.69-1.74 (m, 1H), 2.90 (d, J = 4.6 Hz, 3H), 2.97-3.03 (m, 1H), 3.84 (s, 3H), 6.84 (s, 1H), 7.34 (d, J = 4.6 Hz, 1H), 7.43 (d, J = 8.1 Hz, 2H), 8.00 (d, J = 8.1 Hz, 2H), 9.24 (brs, 1H).
APCIMS m/z: [M+H]⁺ 393.

### Example 152:

### 7-(4-Carboxyphenyl)-8-methoxy-2-methylamino-5-morpholinoquinazoline (Compound 152)

Compound 152 was prepared in the same manner as in Example 11 using Compound 132 and 4-carboxyphenylboronic acid.
¹H NMR (DMSO-d₆, δ) : 2.77 (brs, 4H), 2.92 (d, J = 4.8 Hz, 3H), 3.59 (brs, 4H), 3.86 (s, 3H), 7.36 (d, J = 4.8 Hz, 1H), 7.49 (d, J = 8.1 Hz, 2H), 8.03 (d, J = 8.1 Hz, 2H), 9.34 (brs, 1H).
APCIMS m/z: [M+H]⁺ 395.

### Example 153:

### 7-[-4-(Carboxymethyl)phenyl]-8-methoxy-2-methylamino-5-phenylquinazoline (Compound 153)

Compound 153 was prepared in the same manner as in Example 11 using Compound 97 and 4-(carboxymethyl)phenylboronic acid.
¹H NMR (DMSO-d₆, δ): 2.91 (d, J = 3.9 Hz, 3H), 3.49 (s, 2H), 3.97 (s, 3H), 7.09 (s, 4H), 7.16 (m, 3H), 7.32-7.34 (m, 3H), 7.42 (brs, 1H), 8.51 (brs, 1H).
APCIMS m/z: [M+H]⁺ 400.

### Example 154:

### 7-(4-Carboxymethylphenyl)-5-(2,3-dichlorophenyl)-8-methoxy-2-(methylamino)quinazoline (Compound 154)

Compound 154 was prepared in the same manner as in Example 11 using Compound 116 and 4-carboxymethylphenylboronic acid.
¹H NMR (DMSO-d₆, δ): 2.91 (d, J = 4.8 Hz, 3H), 3.51 (s, 2H), 3.98 (s, 3H), 7.13 (s, 4H), 7.17 (s, 1H), 7.23 (dd, J = 1.7, 7.8 Hz, 1H), 7.32 (t, J = 7.8 Hz, 1H), 7.49 (brs, 1H), 7.62 (dd, J = 1.7, 7.8 Hz, 1H), 8.30 (brs, 1H), 12.29 (brs, 1H).
APCIMS m/z: [M+H]⁺ 468.

### Example 155:

### 7-[4-(Carboxymethyl)phenyl]-5-(5-chloro-2-thienyl)-8-methoxy-2-(methylamino)quinazoline (Compound 155)

Compound 155 was prepared in the same manner as in Example 11 using Compound 127 and 4-carboxymethylphenylboronic acid.
¹H NMR (DMSO-d₆, δ): 2.92 (d, J = 4.9 Hz, 3H), 3.57 (s, 2H), 3.97 (s, 3H), 6.98 (d, J = 3.8 Hz, 1H), 7.10 (d, J = 3.8 Hz, 1H), 7.14 (s, 1H), 7.20 (d, J = 8.5 Hz, 2H), 7.25 (d, J = 8.5 Hz, 2H), 7.45 (brs, 1H), 8.78 (brs, 1H).
APCIMS m/z: [M+H]⁺ 440.

### Example 156:

### 7-(4-Carboxyphenyl)-5-cyclohexyl-8-methoxy-2-(methylamino)quinazoline (Compound 156)

Compound 156 was prepared in the same manner as in Example 11 using Compound 130 and 4-carboxymethylphenylboronic acid.
¹H NMR (DMSO-d₆, δ) : 0.95-1.15 (m, 2H), 1.24-1.43 (m, 1H), 1.54-1.80 (m, 5H), 1.88-2.10 (m, 2H), 2.91 (d, J = 4.8 Hz, 3H), 2.98 (m, 1H), 3.65 (s, 2H), 3.84 (s, 3H), 6.85 (s, 1H), 7.26 (d, J = 8.1 Hz, 2H), 7.28 (brs, 1H), 7.35 (d, J = 8.1 Hz, 2H), 9.58 (brs, 1H).
APCIMS m/z: [M+H]⁺ 406.

### Example 157:

### 7-[4-(2-Carboxyethyl)phenyl]-8-methoxy-2-methylamino-5-phenylquinazoline (Compound 157)

Compound 157 was prepared in the same manner as in Example 11 using Compound 97 and 4-(2-carboxyethyl)phenylboronic acid.
¹H NMR (DMSO-d₆, δ): 2.48 (t, J = 7.4 Hz, 2H), 2.75 (t, J = 7.4 Hz, 2H), 2.91 (d, J = 4.4 Hz, 3H), 3.97 (s, 3H), 7.06 (s, 4H), 7.14-7.17 (m, 3H), 7.31-7.34 (m, 3H), 7.40 (brs, 1H), 8.51 (brs, 1H), 12.12 (brs, 1H).
APCIMS m/z: [M+H]⁺ 414.

### Example 158:

### 7-[4-(2-Carboxyethyl)phenyl-]-5-(5-chloro-2-thienyl)-8-methoxy-2-(methylamino)quinazoline (Compound 158)

Compound 158 was prepared in the same manner as in Example 11 using Compound 127 and 4-(2-carboxyethyl)phenylboronic acid.
¹H NMR (DMSO-d₆, δ): 2.53 (t, J = 7.7 Hz, 2H), 2.82 (t, J = 7.7 Hz, 2H), 2.92 (d, J = 4.6 Hz, 3H), 3.96 (s, 3H), 6.97 (d, J = 3.6 Hz, 1H), 7.10 (d, J = 3.6 Hz, 1H), 7.12 (s, 1H) , 7.17 (d, J = 8.6 Hz, 2H), 7.21 (d, J = 8.6 Hz, 2H), 7.49 (brs, 1H), 8.78 (brs, 1H).
APCIMS m/z: [M+H]⁺ 454.

### Example 159:

### 7-Bromo-5-(3-chlorophenyl)-2-cyclopropylamino-8-methoxyquinazoline (Compound 159)

### Step 1:

A mixture of Compound 3 (790 mg, 2.17 mmol), copper(I) iodide (200 mg, 1.05 mmol), diiodomethane (1.70. mL, 21.1 mmol), isoamyl nitrite (0.870 mL, 6.48 mmol) and THF (20 mL) was stirred at 60°C for 1 hour under an argon atmosphere. After cooling to room temperature, the reaction mixture was filtered through Celite. The residue was washed with ethyl acetate, then the filtrates were combined, and the solvent was evaporated under reduced pressure. The residue was purified by silica gel column chromatography (ethyl acetate/hexane = 1/1) to give 7-bromo-5-(3-chlorophenyl)-2-iodo-8-methoxyquinazoline (Compound bp) (666 mg, 65 %) as a colorless solid.
¹H NMR (CDCl₃, δ): 4.11 (s, 3H), 7.18 (dt, J = 1.8, 6.7 Hz, 1H), 7.30 (d, J = 1.8 Hz, 1H), 7.45-7.55 (m, 3H), 8.62 (s, 1H).

### Step 2:

A mixture of Compound bp (300 mg, 0.631 mmol) obtained in the step 1, cyclopropylamine (0.666 mL, 9.61 mmol), triethylamine (0.260 mL, 1. 87 mmol) and THF (6 mL) was heated under reflux at 70°C for 3 hours under an argon atmosphere. After cooling to room temperature, to the reaction mixture were added water and ethyl acetate, and the mixture was extracted. The organic layer was washed with brine and dried over anhydrous magnesium sulfate. After the mixture was filtered, the solvent was evaporated under reduced pressure, and the residue was purified by trituration with ethanol to give Compound 159 (149 mg, 58 %) as a pale yellow solid.
¹H NMR (CDCl₃, δ): 0:54-0.60 (m, 2H), 0.83-0.89 (m, 2H), 2.89-2.93 (m, 1H), 4.05 (s, 3H), 5.65 (brs, 1H), 7.18-7.21 (m, 1H), 7.23 (s, 1H), 7.31-7.32 (m, 1H), 7.40-7.47 (m, 2H), 8.66 (s, 1H).

### Example 160:

### 7-(4-Carboxyphenyl)-5-(3-chlorophenyl)-2-cyclopropylamino-8-methoxyquinazoline (Compound 160)

Compound 160 was prepared in the same manner as in Example 11 using Compound 159 and 4-carboxyphenylboronic acid.
¹H NMR (DMSO-d₆, δ): 0.50-0.55 (m, 2H), 0.68-0.75 (m, 2H), 2.92 (brs, 1H), 3.98 (s, 3H), 7.14-7.17 (m, 1H), 7.19 (s, 1H), 7.25 (m, 1H), 7.28 (d, J = 8.3 Hz, 2H), 7.33-7.41 (m, 1H), 7.79 (d, J = 8.3 Hz, 2H), 8.58 (s, 1H), 12.93 (brs, 1H).
APCIMS m/z: [M+H]⁺ 446.

### Example 161:

### 7-Bromo-5-(3-chlorophenyl)-2-isopropylamino-8-methoxyquinazoline (Compound 161)

Compound 161 was prepared in the same manner as in Step 2 of Example 159 using Compound bp and isopropylamine. ¹H NMR (CDCl₃, δ): 1.26 (d, J = 6.6 Hz, 6H), 4.04 (s, 3H), 4.25-4.32 (m, 1H), 5.30 (brs, 1H), 7.17-7.20 (m, 1H), 7.21 (s, 1H), 7.26-7.31 (m, 1H), 7.31-7.47 (m, 2H), 8.57 (s, 1H).

### Example 162:

### 7-(4-Carboxyphenyl)-5-(3-chlorophenyl)-2-isopropylamino-8-methoxyquinazoline (Compound 162)

Compound 162 was prepared in the same manner as in Example 11 using Compound 161 and 4-carboxyphenylboronic acid.
¹H NMR (DMSO-d₆, δ): 1.20 (d, J = 6.6 Hz, 6H), 3.97 (s, 3H), 4.24 (m, 1H), 7.13-7.16 (m, 1H), 7.17 (s, 1H), 7.25 (s, 1H), 7.27 (d, J = 8.3 Hz, 2H), 7.33-7.40 (m, 2H), 7.46 (brs, 1H), 7.78 (d, J = 8.3 Hz, 2H), 8.55 (s, 1H), 12.92 (brs, 1H).
APCIMS m/z: [M+H]⁺ 448.

### Example 163:

### 7-Bromo-5-(3-chlorophenyl)-2-ethylamino-8-methoxyquinazoline (Compound 163)

Compound 163 was prepared in the same manner as in Step 2 of Example 159 using Compound bp and ethylamine.
¹H NMR (300 MHz, CDCl₃) d(ppm) 1.26 (t, J = 7.3 Hz, 3H), 3.56 (q, J = 7.3 Hz, 2H), 4.05 (s, 3H), 5.36 (brs, 1H), 7.18-7.21 (m, 1H), 7.22 (s, 1H), 7.31 (s, 1H), 7.39-7.45 (m, 2H), 8.56 (s, 1H).

### Example 164:

### 7-(4-Carboxyphenyl)-5-(3-chlorophenyl)-2-ethylamino-8-methoxyquinazoline (Compound 164)

Compound 164 was prepared in the same manner as in Example 11 using Compound 163 and 4-carboxyphenylboronic acid.
¹H NMR (DMSO-d₆, δ): 1.17 (t, J = 7.3 Hz, 3H), 3.42 (q, J = 7.3 Hz, 2H), 3.97 (s, 3H), 7.13-7.16 (m, 1H), 7.17 (s, 1H), 7.23 (s, 1H), 7.26 (d, J = 8.4 Hz, 2H), 7.36-7.37 (m, 2H), 7.54 (brs, 1H), 7.77 (d, J = 8.4 Hz, 2H), 8.54 (s, 1H), 12.8 (brs, 1H).

### Example 165:

### 7-Bromo-5-(3-chlorophenyl)-2-cyclopropylmethylamino-8-methoxyquinazoline (Compound 165)

Compound 165 was prepared in the same manner as in Step 2 of Example 159 using Compound bp and cyclopropylmethylamine.
¹H NMR (CDCl₃, δ): 0.25-0.29 (m, 2H), 0.51-0.56 (m, 2H), 1.04-1.14 (m, 1H), 3.38-3.42 (m, 2H), 4.05 (s, 1H), 5.56 (brs, 1H), 7.17-7.21 (m, 1H), 7.22 (s, 1H), 7.30-7.32 (m, 1H), 7.43-7.45 (m, 2H), 8.56 (s, 1H).

### Example 166:

### 7-(4-Carboxyphenyl)-5-(3-chlorophenyl)-2-cyclopropylmethylamino-8-methoxyquinazoline (Compound 166)

Compound 166 was prepared in the same manner as in Example 11 using Compound 165 and 4-carboxyphenylboronic acid.
¹H NMR (DMSO-d₆, δ): 0.26-0.29 (m, 2H), 0.41-0.44 (m, 2H), 1.08-1.12 (m, 1H), 3.17-3.21 (m, 2H), 3.98 (s, 1H), 7.14-7.18 (m, 2H), 7.24-7.27 (m, 1H), 7.27 (d, J = 8.1 Hz, 2H), 7.35-7.38 (m, 2H), 7.65 (brs, 1H), 7.78 (d, J = 8.1 Hz, 2H), 8.55 (s, 1H), 12.9 (brs, 1H).

### Example 167:

### 2-Benzylamino-7-bromo-5-(3-chlorophenyl)-8-methoxyquinazoline (Compound 167)

Compound 167 was prepared in the same manner as in Step 2 of Example 159 using Compound bp and benzylamine.
¹H NMR (CDCl₃, δ): 4.05 (s, 3H), 4.76 (d, J = 5.7 Hz, 2H), 5.76 (brs, 1H), 7.17-7.47 (m, 10H), 8.58 (s, 1H).

### Example 168:

### 2-Benzylamino-7-(4-carboxyphenyl)-5-(3-chlorophenyl)-8-methoxyquinazoline (Compound 168)

Compound 168 was prepared in the same manner as in Example 11 using Compound 167 and 4-carboxyphenylboronic acid.
¹H NMR (DMSO-d₆, δ): 3.96 (s, 3H), 4.65 (s, 2H), 7.15-7.36 (m, 10H), 7.25 (d, J = 8.4 Hz, 2H), 7.77 (d, J = 8.4 Hz, 2H), 8.12 (brs, 1H), 8.57 (s, 1H), 12.9 (brs, 1H).

### Example 169:

### 5-(3-Chlorophenyl)-6-formyl-8-methoxy-2-(methylamino)quinazoline (Compound 169)

A mixture of Compound aw (1.00 g, 2.92 mmol), 3-chlorophenylboronic acid (0.68 g, 4.4 mmol), tetrakis(triphenylphosphine)palladium (0.34 g, 0.29 mmol), sodium carbonate (0.93 g, 8.8 mmol), dioxane (10 mL) and water (10 mL) was heated under reflux for 3 hours under an argon atmosphere. After cooling to room temperature, to the mixture was added water, and the insoluble matter filtrated off. To the filtrate were added ethyl acetate and water, and the organic layer was separated and washed with water. The organic layer was dried over anhydrous magnesium sulfate, and concentrated under reduced pressure. The residue was dissolved in THF (10 mL), and 6 mol/L hydrochloric acid (5 mL) was added thereto, and then the mixture was stirred overnight. The reaction mixture was neutralized with 1 mol/L aqueous sodium hydroxide, and ethyl acetate and water were added thereto, then the organic layer was separated. The organic layer was dried over anhydrous magnesium sulfate, and concentrated under reduced pressure to give Compound 169 (0.81 g, 86 %).
¹H NMR (CDCl₃, δ): 3.17 (brs, 3H), 4.10 (s, 3H), 5.66 (brs, 1H), 7.26-7.31 (m, 1H), 7.41-7.55 (m, 3H), 7.59 (s, 1H), 8.74 (s, 1H), 9.76 (s, 1H).

### Example 170:

### 6-[E-(2-carboxyethenyl)]-5-(3-chlorophenyl)-8-methoxy-2-(methylamino)quinazoline (Compound 170)

Compound 169 (350 mg, 1.07 mmol) was dissolved in pyridine (7 mL), and malonic acid (222 mg, 2.14 mmol) and piperidine (0.10 mL, 1.07 mmol) were added thereto. The mixture was stirred at 90°C for 1 hour, and water and 2 mol/L hydrochloric acid were added thereto. The resulting crystal was collected by filtration to give Compound 170 (369 mg, 94 %).
¹H NMR (DMSO-d₆, δ): 2.91 (d, J = 4.0 Hz, 3H), 4.02 (s, 3H), 6.61 (d, J = 15.7 Hz, 1H), 7.26 (d, J = 15.7 Hz, 1H), 7.26-7.30 (m, 1H), 7.37-7.43 (m, 1H), 7.50-7.62 (m, 4H), 8.38 (s, 1H), 12.80 (brs, 1H).

### Example 171:

### 5-(3-Chlorophenyl)-8-methoxy-6-{E-[2-(methoxycarbonyl)ethenyl]}-2-(methylamino)quinazoline (Compound 171)

Compound 170 (360 mg, 0.973 mmol) was dissolved in DMF (4 mL), and iodomethane (0.14 mL, 2.0 mmol) and potassium carbonate (400 mg, 2.89 mmol) were added thereto. The mixture was stirred at room temperature for 2 hours, and water was added thereto. The resulting crystal was collected by filtration to give Compound 171 (333 mg, 89 %).
¹H NMR (CDCl₃, δ): 3.15 (d, J = 5.0 Hz, 3H), 3.74 (s, 3H), 4.09 (s, 3H), 5.51 (q, J = 5.0 Hz, 1H), 6.36 (d, J = 15.8 Hz, 1H), 7.14-7.18 (m, 1H), 7.26-7.28 (m, 2H), 7.41-7.53 (m, 3H), 8.60 (s, 1H).

### Example 172:

### 6-(2-Carboxyethyl)-5-(3-chlorophenyl)-8-methoxy-2-(methylamino)quinazoline (Compound 172)

Compound 172 was prepared in the same manner as in the step 1 of Example 32 using Compound 171.
¹H NMR (DMSO-d₆, δ): 2.43 (t, J = 7.6 Hz, 2H), 2.64 (t, J = 7.6 Hz, 2H), 2.88 (d, J = 4.8 Hz, 3H), 3.94 (s, 3H), 7.18 (s, 1H), 7.24-7.30 (m, 2H), 7.36-7.40 (m, 1H), 7.52-7.55 (m, 2H), 8.29 (s, 1H), 12.08 (brs, 1H).

### Example 173:

### 6-Carboxy-5-(3-chlorophenyl)-8-methoxy-2-(methylamino)quinazoline (Compound 173)

Compound 169 (200 mg, 0.611 mmol), was suspended in THF (1.5 mL), water (1.5 mL) and t-butanol (1.5 mL), and sodium chlorite (166 mg, 1.84 mmol), sodium dihydrogenphosphate di-hydrate (191 mg, 1.22 mmol) and sulfamic acid (178 mg, 1.83 mmol) were added thereto, and the mixture was stirred overnight at room temperature. To the reaction mixture were added water and 1 mol/L hydrochloric acid to allow the mixture to acidic condition. The resulting crystal was collected by filtration, and purified by thin-layer chromatography to give Compound 173 (100 mg, 48 %).
¹H NMR (DMSO-d₆, δ): 2.91 (d, J = 4.1 Hz, 3H), 3.97 (s, 3H), 7.21-7.27 (m, 1H), 7.32-7.36 (m, 1H), 7.44-7.53 (m, 3H), 7.67 (q, J = 4.1 Hz, 1H), 8.38 (s, 1H), 12.67 (brs, 1H).

### Example 174:

### 6-(4-Carboxyphenyl)-5-(3-chlorophenyl)-8-methoxy-2-(methylamino)quinazoline (Compound 174)

Compound 174 was prepared in the same manner as in Reference Example 6 using Compound bd and 3-chlorophenylboronic acid.
¹H NMR (DMSO-d₆, δ): 2.93 (d, J = 4.1 Hz, 3H), 3.98 (s, 3H), 7.12-7.18 (m, 1H), 7.19 (s, 1H), 7.22-7.24 (m, 1H), 7.27 (d, J = 8.1 Hz, 2H), 7.35-7.40 (m, 2H), 7.50 (q, J = 4.1 Hz, 1H), 7.99 (d, J = 8.1 Hz, 2H), 8.55 (s, 1H).

### Example 175:

### 6-(3-Carboxyphenyl)-5-(3-chlorophenyl)-8-methoxy-2-(methylamino)quinazoline (Compound 175)

Compound 175 was prepared in the same manner as in Reference Example 6 using Compound be and 3-chlorophenylboronic acid.
¹H NMR (DMSO-d₆, δ): 2.92 (d, J = 4.6 Hz, 3H), 3.98 (s, 3H), 7.12-7.17 (m, 1H), 7.19 (s, 1H), 7.24 (s, 1H), 7.31-7.42 (m, 4H), 7.49 (q, J = 4.6 Hz, 1H), 7.73 (s, 1H), 7.75-7.80 (m, 1H), 8.55 (s, 1H).

### Example 176:

### 6-(4-Carboxyphenyl)-5-(5-chloropyridine-3-yl)-8-methoxy-2-(methylamino)quinazoline (Compound 176)

### Step 1

Using 3-chloro-5-trifluoromethanesulfoxypyridine, 3-chloro-5-(4,4,5,5-tetramethyl-1,3,2-dioxaborolan-2-yl)pyridine (Compound bq) was prepared in the same manner as in Reference Example 12.
¹H NMR (CDCl₃, δ): 1.36 (s, 12H), 8.04 (dd, J = 2.3, 1.3 Hz, 1H), 8.63 (d, J = 2.3 Hz, 1H), 8.79 (d, J = 1.3 Hz, 1H).

### Step 2

Compound 176 was prepared in the same manner as in Reference Example 6 using Compound bd and Compound bq. ¹H NMR (DMSO-d₆, δ): 2.93 (d, J = 4.3 Hz, 3H), 3.99 (s, 3H), 7.21 (s, 1H), 7.28 (d, J = 8.0 Hz, 2H), 7.55 (q, J = 4.3 Hz, 1H), 7.81 (d, J = 8.0 Hz, 2H), 7.89 (s, 1H), 8.24 (s, 1H), 8.55 (s, 1H), 8.59 (s, 1H), 12.96 (brs, 1H).

### Example 177:

### Preparation Example 1: Tablet

A tablet having the following formulation is prepared in a conventional manner.

| Formulation: | | |
|---|---|---|
| Compound 1 | 20 | mg |
| Lactose | 143.4 | mg |
| Potato starch | 30 | mg |
| Hydroxypropyl cellulose | 6 | mg |
| Magnesium stearate | 0.6 | mg |
| | 200 | mg |

### Example 178:

### Preparation Example 2: Injection Preparation

An injection preparation having the following formulation is prepared in a conventional manner.

| Formulation: | |
|---|---|
| Compound 7 | 2 mg |
| D-mannitol | 10 mg |
| Aqueous hydrochloric acid | ad lib. |
| Aqueous sodium hydroxide | ad lib. |
| Distilled water for injection | ad lib. |
| | 2.00 mL |

### INDUSTRIAL APPLICABILITY

The present invention provides 2-amino quinazoline derivatives and the like having PDE-IV inhibitory activity.

## Claims

1. A 2-amino quinazoline derivative represented by formula (I) 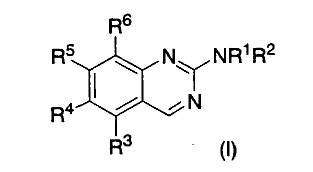 (wherein R¹ and R² are the same or different, and each represents a hydrogen atom, substituted or unsubstituted lower alkyl, substituted or unsubstituted cycloalkyl, substituted or unsubstituted aryl, a substituted or unsubstituted aromatic heterocyclic group, or a substituted or unsubstituted alicyclic heterocyclic group, or R¹ and R² are combined together with the adjacent nitrogen atom to form a substituted or unsubstituted aromatic heterocyclic group, or a substituted or unsubstituted alicyclic heterocyclic group,
R³ represents substituted or unsubstituted aryl, a substituted or unsubstituted aromatic heterocyclic group, substituted or unsubstituted cycloalkyl, substituted or unsubstituted cycloalkenyl, or a substituted or unsubstituted alicyclic heterocyclic group,
R⁴ and R⁵ are the same or different, and each represents a hydrogen atom, halogen, hydroxy, carboxy, substituted or unsubstituted lower alkyl, substituted or unsubstituted cycloalkyl, substituted or unsubstituted lower alkenyl,' substituted or unsubstituted cycloalkenyl, substituted or unsubstituted lower alkoxy, substituted or unsubstituted cycloalkoxy, substituted or unsubstituted lower alkanoyl, substituted or unsubstituted aryl, a substituted or unsubstituted aromatic heterocyclic group, or a substituted or unsubstituted alicyclic heterocyclic group, proviso that they are not simultaneously hydrogen atoms, and
R⁶ represents hydroxy or substituted or unsubstituted lower alkoxy), or a pharmaceutically acceptable salt thereof.

2. The 2-amino quinazoline derivative or a pharmaceutically acceptable salt thereof according to claim 1, wherein R¹ and R² are the same or different, and each represents a hydrogen atom, substituted or unsubstituted lower alkyl, or substituted or unsubstituted cycloalkyl.

3. The 2-amino quinazoline derivative or a pharmaceutically acceptable salt thereof according to claim 1, wherein R¹ and R² are the same or different, and each represents a hydrogen atom, methyl, ethyl, or isopropyl.

4. The 2-amino quinazoline derivative or a pharmaceutically acceptable salt thereof according to claim 1, wherein R¹ represents a hydrogen atom, and R² represents substituted or unsubstituted lower alkyl, or substituted or unsubstituted cycloalkyl.

5. The 2-amino quinazoline derivative or a pharmaceutically acceptable salt thereof according to claim 1, wherein R¹ represents a hydrogen atom, and R² represents methyl, ethyl, or isopropyl.

6. The 2-amino quinazoline derivative or a pharmaceutically acceptable salt thereof according to claim 1, wherein R¹ represents a hydrogen atom, and R² represents cyclopropyl or cyclopropylmethyl.

7. The 2-amino quinazoline derivative or a pharmaceutically acceptable salt thereof according to claim 1, wherein R¹ represents a hydrogen atom, and R² represents aralkyl.

8. The 2-amino quinazoline derivative or a pharmaceutically acceptable salt thereof according to any one of claims 1 to 7, wherein R³ represents a substituted or unsubstituted aromatic heterocyclic group.

9. The 2-amino quinazoline derivative or a pharmaceutically acceptable salt thereof according to any one of claims 1 to 7, wherein R³ represents substituted or unsubstituted pyridyl.

10. The 2-amino quinazoline derivative or a pharmaceutically acceptable salt thereof according to any one of claims 1 to 7, wherein R³ represents substituted or unsubstituted aryl.

11. The 2-amino quinazoline derivative or a pharmaceutically acceptable salt thereof according to any one of claims' 1 to 7, wherein R³ represents substituted or unsubstituted phenyl.

12. The 2-amino quinazoline derivative or a pharmaceutically acceptable salt thereof according to any one of claims 1 to 7, wherein R³ represents substituted or unsubstituted cycloalkyl, substituted or unsubstituted cycloalkenyl, or a substituted or unsubstituted alicyclic heterocyclic group.

13. The 2-amino quinazoline derivative or a pharmaceutically acceptable salt thereof according to any one of claims 1 to 12, wherein R⁴ or R⁵ represents formyl or carboxy.

14. The 2-amino quinazoline derivative or a pharmaceutically acceptable salt thereof according to any one of claims 1 to 12, wherein R⁴ represents substituted or unsubstituted lower alkyl, or substituted or unsubstituted lower alkenyl.

15. The 2-amino quinazoline derivative or a pharmaceutically acceptable salt thereof according to any one of claims 1 to 12, wherein R⁴ represents lower alkyl which substituted with carboxy, or lower alkenyl which substituted with carboxy.

16. The 2-amino quinazoline derivative or a pharmaceutically acceptable salt thereof according to any one of claims 1 to 12, wherein R⁴ represents substituted or unsubstituted aryl.

17. The 2-amino quinazoline derivative or a pharmaceutically acceptable salt thereof according to any one of claims 1 to 12, wherein R⁴ represents aryl which substituted with carboxy.

18. The 2-amino quinazoline derivative or a pharmaceutically acceptable salt thereof according to any one of claims 1 to 17, wherein R⁵ represents substituted or unsubstituted lower alkyl, substituted or unsubstituted cycloalkyl, substituted or unsubstituted lower alkenyl, or substituted or unsubstituted cycloalkenyl.

19. The 2-amino quinazoline derivative or a pharmaceutically acceptable salt thereof according to any one of claims 1 to 17, wherein R⁵ represents substituted or unsubstituted aryl, or a substituted or unsubstituted aromatic heterocyclic group.

20. The 2-amino quinazoline derivative or a pharmaceutically acceptable salt thereof according to any one of claims 1 to 17, wherein R⁵ represents lower alkyl which substituted with carboxy, lower alkenyl which substituted with carboxy, aryl which substituted with carboxy, an aromatic heterocyclic group which substituted with carboxy, or an alicyclic heterocyclic group which substituted with carboxy.

21. The 2-amino quinazoline derivative or a pharmaceutically acceptable salt thereof according to any one of claims 1 to 17, wherein R⁵ represents lower alkyl which substituted with carboxy.

22. The 2-amino quinazoline derivative or a pharmaceutically acceptable salt thereof according to any one of claims 1 to 17, wherein R⁵ represents aryl which substituted with carboxy.

23. The 2-amino quinazoline derivative or a pharmaceutically acceptable salt thereof according to any one of claims 1 to 17, wherein R⁵ represents phenyl which substituted with carboxy.

24. The 2-amino quinazoline derivative or a pharmaceutically acceptable salt thereof according to any one of claims 1 to 17, wherein R⁵ represents an aromatic heterocyclic group which substituted with carboxy.

25. The 2-amino quinazoline derivative or a pharmaceutically acceptable salt thereof according to any one of claims 1 to 17, wherein R⁵ represents an alicyclic heterocyclic group which substituted with carboxy.

26. The 2-amino quinazoline derivative or a pharmaceutically acceptable salt thereof according to any one of claims 1 to 17, wherein R⁵ represents an alicyclic heterocyclic group which substituted with carboxymethyl.

27. The 2-amino quinazoline derivative or a pharmaceutically acceptable salt thereof according to any one of claims 1 to 17, wherein R⁵ represents halogen.

28. The 2-amino quinazoline derivative or a pharmaceutically, acceptable salt thereof according to any one of claims 1 to 27, wherein R⁶ represents substituted or unsubstituted lower alkoxy.

29. The 2-amino quinazoline derivative or a pharmaceutically acceptable salt thereof according to any one of claims 1 to 27, wherein R⁶ represents methoxy.

30. The 2-amino quinazoline derivative or a pharmaceutically acceptable salt thereof according to any one of claims 1 to 27, wherein R⁶ represents hydroxy.

31. A pharmaceutical composition which comprises the 2-amino quinazoline derivative or a pharmaceutically acceptable salt thereof according to any one of claims 1 to 30 as an active ingredient.

32. A phosphodiesterase (PDE)-IV inhibitor which comprises the 2-amino quinazoline derivative or a pharmaceutically acceptable salt thereof according to any one of claims 1 to 30 as an active ingredient.

33. A preventive and/or therapeutic agent for diseases related to the function of phosphodiesterase (PDE)-IV which comprises the 2-amino quinazoline derivative or a pharmaceutically acceptable salt thereof according to any one of claims 1 to 30 as an active ingredient.

34. A method for preventing and/or treating diseases related to the function of phosphodiesterase (PDE)-IV which comprises a step of administering an effective amount of the 2-amino quinazoline derivative or a pharmaceutically acceptable salt thereof according to any one of claims 1 to 30.

35. Use of the 2-amino quinazoline derivative or a pharmaceutically acceptable salt thereof according to any one of claims 1 to 30 for the manufacture of phosphodiesterase (PDE)-IV inhibitor.

36. Use of the 2-amino quinazoline derivative or a pharmaceutically acceptable salt thereof according to any one of claims 1 to 30 for the manufacture of a preventive and/or therapeutic agent for diseases related to the function of phosphodiesterase (PDE)-IV.
